(19) Europäisches Patentamt
European Patent Office
Office européen des brevets

(11) EP 2 206 498 B1

(12) **EUROPÄISCHE PATENTSCHRIFT**

(45) Veröffentlichungstag und Bekanntmachung des Hinweises auf die Patenterteilung:
**25.01.2012 Patentblatt 2012/04**

(51) Int Cl.:
***A61K 31/407*** *(2006.01)* ***A61K 31/438*** *(2006.01)*
***A61P 25/04*** *(2006.01)*

(21) Anmeldenummer: **10003202.8**

(22) Anmeldetag: **26.09.2007**

(54) **Gemischte ORL1/μ-Agonisten zur Behandlung von Schmerz**

Mixed ORL1/[mu] agonists for treating pain

Agonistes mixtes des récepteurs ORL1/[mu] pour le traitement de la douleur

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **29.09.2006 DE 102006046745**

(43) Veröffentlichungstag der Anmeldung:
**14.07.2010 Patentblatt 2010/28**

(62) Dokumentnummer(n) der früheren Anmeldung(en) nach Art. 76 EPÜ:
**07818462.9 / 2 081 571**

(73) Patentinhaber: **Grünenthal GmbH**
**52078 Aachen (DE)**

(72) Erfinder:
- **Linz, Klaus, Dr.**
  **53343 Wachtberg (DE)**
- **Kögel, Babette-Yvonne, Dr.**
  **52379 Langerwehe-Hamich (DE)**
- **Schröder, Wolfgang, Dr.**
  **52074 Aachen (DE)**
- **Christoph, Thomas, Dr.**
  **52080 Aachen (DE)**
- **De Vry, Jean, Dr.**
  **52223 Stolberg (DE)**
- **Friderichs, Elmar**
  **52223 Stolberg (DE)**

(74) Vertreter: **Bülle, Jan et al**
**Patentanwälte Kutzenberger & Wolff**
**Theodor-Heuss-Ring 23**
**50668 Köln (DE)**

(56) Entgegenhaltungen:
**WO-A-2004/043967 WO-A-2005/066183**
**US-A1- 2005 153 998**

- **OBARA I ET AL: "Spinal and local peripheral antiallodynic activity of Ro64-6198 in neuropathic pain in the rat" PAIN, ELSEVIER SCIENCE PUBLISHERS, AMSTERDAM, NL, Bd. 116, Nr. 1-2, Juli 2005 (2005-07), Seiten 17-25, XP004944204 ISSN: 0304-3959**
- **TAMAI H ET AL: "Anti-allodynic and anti-hyperalgesic effects of nociceptin receptor antagonist, JTC-801, in rats after spinal nerve injury and inflammation" EUROPEAN JOURNAL OF PHARMACOLOGY, AMSTERDAM, NL, Bd. 510, Nr. 3, 14. März 2005 (2005-03-14) , Seiten 223-228, XP004781072 ISSN: 0014-2999**
- **CEPEDA M SOLEDAD ET AL: "Side effects of opioids during short-term administration: effect of age, gender, and race" CLINICAL PHARMACOLOGY AND THERAPEUTICS, NATURE PUBLISHING GROUP, US LNKD- DOI: 10.1016/S0009-9236(03)00152-8, Bd. 74, Nr. 2, 1. August 2003 (2003-08-01) , Seiten 102-112, XP009128546 ISSN: 0009-9236**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 206 498 B1

## Beschreibung

Neben akuten Schmerzen, die von begrenzter Dauer sind und nach Beseitigung der auslösenden Reize in der Regel rasch wieder abklingen, stellen insbesondere chronische Schmerzen eine Herausforderung an die Medizin dar. Akute Schmerzereignisse durch Stimulation intakter Nociceptoren haben eine Wamfunktion zur Bewahrung der Körperintegrität. Die darauf folgenden Reaktionen zur Schmerzvermeidung schützen vor Schäden. Der chronische Schmerz hat diese Schutzfunktion verloren. Es liegt eine Schmerzerkrankung vor. Chronische Schmerzen können dabei in zwei große Gruppen unterteilt werden. Der pathophysiologische Nociceptorschmerz wird nach Gewebetraumen durch die Erregung von intakten Nociceptoren hervorgerufen. Hierzu gehören insbesondere chronische Entzündungsschmerzen. Schmerzen hingegen, die durch eine Schädigung von Nerven selbst entstehen, werden als neuropathische Schmerzen bezeichnet.

Der Übergang von akutem Schmerz zu chronischem Schmerz kann innerhalb von Stunden erfolgen. Betroffen ist hiervon beispielsweise die Schmerzbehandlung während und im Anschluss an eine Operation. Obwohl der Behandlung des Akutschmerzes heute im Bewusstsein der Ärzte eine große Bedeutung zukommt, unterliegt die Behandlung des postoperativen Schmerzes starken Limitierungen (Power, Brit. J. Anaesth., 2005, 95, 43-51). Akutschmerz kann sich im Anschluß an eine Gewebeschädigung, beispielsweise eine Operation, über pathophysiologische Prozesse peripher und im ZNS chronifizieren. Die Assoziation zwischen Gewebeschädigung, akutem postoperativen Schmerz und sich entwickelndem chronischem Schmerz ist gut untersucht, wobei die Stärke des Akutschmerzes als prädiktiver Faktor für die Dauer des chronischen Schmerzes betrachtet werden kann (Power, Brit. J. Anaesth., 2005, 95, 43-51). Allein schon aus diesem Grund ist eine zufrieden stellende Behandlung von Akutschmerz unabdingbar.

Ein Problem bei der Bekämpfung von Akutschmerz sind die Nebenwirkungen der im Akutschmerz hochwirksamen $\mu$-Opioide wie Morphin oder Fentanyl, insbesondere die Atemdepression. Da diese Nebenwirkung gelegentlich zu Todesfällen bei frisch operierten Patienten führt, werden die Arzneimittel in vielen Fällen nicht In ausreichender Menge zur zufrieden stellenden Bekämpfung des Schmerzes gegeben. Auf der anderen Seite ist heute eine Behandlung von postoperativem Schmerz ohne Opioide nicht vorstellbar. Die Furcht vor Atemdepression und weiteren $\mu$-opioid-typischen Nebenwirkungen führt jedoch In vielen Fällen dazu, dass Opioide bei starken Schmerzen, beispielsweise bei Krebspatienten, in zu geringem Maße eingesetzt werden (Davis et al, Respiratory Care Journal 1999, 44 (1)). Weiterhin ist das Risiko, dass Atemdepression nach Gabe von Opioiden auftritt, bei älteren Menschen im Vergleich zu jüngeren Menschen erhöht. Tatsächlich steigt das Risiko, Atemdepression zu entwickeln, bei Menschen ab 60 Jahren deutlich an (Cepeda et al, Clinical Pharmacology and Therapeutics 2003, 74, 102-112). Es besteht daher ein dringender Bedarf an neuen Arzneimitteln zur Behandlung von Schmerz, bei denen die Atemdepression reduziert ist.

Wie bereits erwähnt stellt jedoch die Behandlung des chronischen Schmerzes eine größere Herausforderung dar, da die am Markt befindlichen Arzneimittel zwar zum Teil im Akutschmerz hochwirksam sind, im chronischen Schmerz jedoch in vielen Fällen zu keiner zufrieden stellenden Schmerzbehandlung führen.

## Entzündungsschmerzen

Schmerz tritt neben Rötung, Schwellung, Überwärmung und gestörter Funktion als eines der fünf Kardinalsymptome einer Entzündung auf. Entzündungsvorgänge gehören zu den wichtigsten Mechanismen der Schmerzentstehung. Die typischen Entzündungsschmerzen werden durch Freisetzung von Bradykinin, Histamin und Prostaglandinen mit einer Ansäuerung des Gewebes und dem Druck des Exsudats auf die Nociceptoren ausgelöst. Nociception unterliegt, anders als andere Sinnesempfindungen keiner Habituation. Vielmehr können vorangegangene Schmerzimpulse die Verarbeitung nachfolgender Reize im Sinne einer Sensibilisierung verstärken. Kommt es z.B. durch lang anhaltende Aktivierung von Nociceptoren im entzündeten Gewebe zu einem vermehrten Einstrom von Schmerzimpulsen ins zentrale Nervensystem, treten dauerhafte Sensibilisierungsphänomene an den zentralen Synapsen auf. Diese zentralen Sensibilisierungsphänomene äußern sich in der Zunahme der Spontanaktivität und in stärkeren Reizantworten zentraler Neurone, deren rezeptive Felder sich ebenfalls vergrößern (Coderre et al., Pain 1993, 52, 259-285). Diese Veränderungen im Antwortverhalten zentraler Neurone können zum Spontanschmerz und zur Hyperalgesie (gesteigerte Schmerzempfindung auf einen noxischen Reiz) beitragen, die für entzündetes Gewebe typisch sind (Yaksh et al., PNAS 1999, 96, 7680-7686).

Einer der wichtigsten Prozesse bei Entzündungen ist das Auftreten von Arachidonsäuremetaboliten. Diese Verbindungen aktivieren Nociceptoren nicht direkt, sondern reduzieren die Reizweiterleitungs-Schwelle der C-Fasern und sensitisieren sie so für andere Stimuli. Zur Behandlung von Entzündungsschmerzen haben sich insbesondere nichtsteroidale Antiphlogistika (NSAIDs) bewährt, da sie den Arachidonsäure-Abbau blockieren (Dickensen, A., International Congress and Symposium Series - Royal Society of Medicine (2000), 246, 47-54). Ihre Anwendung bei der Langzeittherapie chronischer Schmerzen ist jedoch durch zum Teil erhebliche unerwünschte Wirkungen, wie gastroenterale Ulzera oder toxische Nierenschädigungen, limitiert.

Auch bei der Behandlung von Entzündungsschmerz ist jedoch die inhibitorische Kontrolle der Reizweiterleitung

von Bedeutung. μ-Opioide stellen die wichtigsten Vertreter dieser Klasse dar. Chronische Pankreatitis beispielsweise geht mit Schmerzen einher, die zu den klinisch am schwierigsten zu behandelnden Schmerzzuständen gehören. Die Gabe von NSAIDs reduziert den Schmerz möglicherweise nur leicht, führt jedoch aufgrund der erhöhten Blutungsgefahr zu einem zu hohen Risiko. Der nächste Schritt ist im Allgemeinen die Behandlung mit μ-Opioiden. Unter den betroffenen Personen ist die Abhängigkeit von Narkotika weit verbreitet (Vercauteren et al., Acta Anaesthesiologica Belglca 1994, 45, 99-105). Es besteht daher dringender Bedarf an Verbindungen, die im Entzündungsschmerz gut wirksam sind und ein vermindertes Abhängigkeitspotential besitzen.

**Neuropathische Schmerzen**

Neuropathische Schmerzen entstehen, wenn periphere Nerven auf mechanische, metabolische oder entzündliche Weise geschädigt werden. Die dabei auftretenden Schmerzbilder sind vorwiegend durch das Auftreten von Spontanschmerz, Hyperalgesie und Allodynie (Schmerz wird bereits durch nicht-noxische Reize ausgelöst) gekennzeichnet. Als Folge der Läsionen kommt es zu einer vermehrten Expression von Na+-Kanäten und damit zur Spontanaktivität in den geschädigten Axonen und ihren Nachbaraxonen (England et al., Neurology 1996, 47, 272-276). Die Erregbarkeit der Neurone wird gesteigert und sie reagieren auf ankommende Reize mit einer erhöhten Entladungsfrequenz. Es resultiert eine verstärkte Schmerzempfindlichkeit, die zur Entwicklung von Hyperalgesie und Spontanschmerz beiträgt (Baron, Clin. J. Pain 2000;16 (2 Suppl), 12-20).

Die Ursachen und Ausprägungen und daher auch die Behandlungserfordernisse von neuropathischerm Schmerz sind vielfältig. Sie entstehen als Folge von Verletzungen oder Erkrankungen von Gehirn, Rückenmark oder peripheren Nerven. Ursachen können Operationen sein, z. B. Phantomschmerz nach Amputation, Schlaganfälle, Multiple Sklerose, Rückenmarksverletzungen, Alkohol- oder Medikamentenmissbrauch bzw. weitere Giftstoffe, Krebserkrankungen aber auch Stoffwechselkrankheiten wie Diabetes, Gicht, Niereninsuffizienz oder Leberzirrhose, oder Infektionserkrankungen wie Mononukleose, Ehrlichiose, Typhus, Diphtherie, HIV, Lues oder Borrelien. Das Schmerzerleben hat sehr unterschiedliche Zeichen und Symptome, die sich in Anzahl und Intensität über die Zeit verändern können. Paradoxerweise schildern die Patienten mit neuropathischen Schmerzen eine Abnahme oder Störung der akuten Schmerzwahrnehmung bei gleichzeitiger Zunahme des neuropathischen Schmerzes. Die typischen Symptome neuropathischer Schmerzen werden als Kribbeln, Brennen, einschießend, elektrisierend oder ausstrahlend beschrieben.

Zur pharmakologischen Basistherapie neuropathischer Schmerzen gehören trizyklische Antidepressiva und Antikonvulsiva, die als Monotherapie oder auch in Kombination mit Opioiden eingesetzt werden. Diese Medikamente erbringen meistens nur eine gewisse Schmerzerleichterung, während eine Schmerzfreiheit aber oftmals nicht erreicht wird. Die sich häufig einstellenden Nebenwirkungen stehen dabei Dosiserhöhungen der Arzneimittel zur Erzielung einer ausreichenden Schmerzlinderung häufig im Wege. Tatsächlich wird zur zufrieden stellenden Behandlung von neuropathischem Schmerz häufig eine höhere Dosierung eines μ-Opioids benötigt als zur Behandlung von Akutschmerz, wodurch die Nebenwirkungen eine noch größere Bedeutung erlangen. Durch den Eintritt der μ-Opioid-typischen Toleranzentwicklung und die damit einhergehende Notwendigkeit der Dosissteigerung wird dieses Problem noch verstärkt. Zusammenfassend kann festgehalten werden, dass neuropathischer Schmerz heute schwer zu behandeln ist und durch hohe Dosen von μ-Opioiden nur teilweise gelindert wird (Saudi Pharm. J. 2002, 10 (3), 73-85). Es besteht daher der dringende Bedarf nach Arzneimitteln zur Behandlung von chronischen Schmerzen, deren Dosis nicht bis zum Auftreten von nicht tolerierbaren Nebenwirkungen gesteigert werden muss, um eine zufrieden stellende Schmerztherapie zu gewährleisten.

In den vergangenen Jahrzehnten wurden verschiedene andere Wirkprinzipien zur Behandlung von chronischem Schmerz vorgeschlagen und umgesetzt, die die opioidtypischen Nebenwirkungen nicht aufweisen. So werden in der Therapie von mittelstarken bis starken chronischen Schmerzen beispielsweise auch Antidepressiva verwendet, die neben der stimmungsaufhellenden Wirkung auch eine analgetische Wirkung aufweisen. Bislang konnte jedoch kein Wirkprinzip die μ-Opioide aus der zentralen Bedeutung in der Schmerztherapie verdrängen. Einer der Hauptgründe ist die bislang unerreichte Wirkstärke der μ-Opioide. Neben der Atemdepression weisen μ-Opioide jedoch auch weitere Nachteile auf:

**a) Opioid-Induzierte Hyperalgesie**

Seit über 100 Jahren ist bekannt, dass eine erhöhte Schmerzwahmehmung zu den Symptomen von Opioid-Entzug gehört. Heute gehört das Auftreten von Schmerz-Symptomen zu den Kriterien für die Diagnose von Opioid-Entzug (Angst et al, Anesthesiology 2006, 104, 570-587). Eine wachsende Zahl von Studien am Tier und am Menschen zeigen, dass unter bestimmten Umständen μ-Opioide Veränderungen bei der Schmerzempfindung verursachen können, die zu Hyperalgesie (Empfindung von erhöhter Schmerzintensität nach schmerzhaftem Stimulus) führen. Diese Studien zeigen, dass sowohl bei kurzer als auch bei chronischer Opioid-Gabe das Phänomen der opioid-induzierten Hyperalgesie auftritt (Pud et al, Drug and Alcohol Dependence 2006, 218-223). Es ist beispielsweise bekannt, dass Patienten, die

eine Narkose mit hohem Opioid-Anteil erhalten, postoperativ etwa das Dreifache an Opioiden benötigen, verglichen mit Patienten mit Hypnotika-Narkose. Dieser deutliche Effekt schränkt den sicheren Gebrauch der μ-Opioide ebenfalls ein, da bei der dadurch notwendigen Dosis-Steigerung die Nebenwirkungen wie etwa Atemdepression eine größere Bedeutung erhalten. Da jedoch die Behandlung von starken Schmerzen ohne Opioide heute nicht vorstellbar ist, besteht ein dringender Bedarf an Arzneimitteln, die nicht bereits von sich aus bereits zu erhöhter Schmerzintensität beim Patienten führen.

**b) Abhängigkeitspotential**

Die zur Schmerztherapie eingesetzten μ-Opioide wie Morphin und Fentanyl weisen ein Abhängigkeitspotential auf. In vielen Fällen kommt es beim Absetzen dieser Arzneimittel zu Entzugserscheinungen. Diese Nebenwirkung von μ-Opioiden führt zu einer erheblichen Einschränkung des Nutzens dieser hochwirksamen Schmerztherapeutika, da aus Furcht vor Abhängigkeit μ-Opioide bei starken Schmerzen oft nicht verschrieben oder eingenommen werden. Es besteht daher dringender Bedarf an Schmerztherapeutika, die hochwirksam sind und gleichzeitig ein im Vergleich zu μ-Opioiden vermindertes Abhängigkeitspotential aufweisen.

Die typischen Nebenwirkungen von μ-Opioiden sind nicht an allen Patienten gleich stark ausgebildet. So gibt es Patientengruppen, für die die Nebenwirkungen tolerierbar sind und andere, für die sie ein großes Problem darstellen. Im Durchschnitt sind die Nebenwirkungen jedoch ein Problem, dass bislang nicht gelöst werden konnte, obwohl μ-Opioide, ursprünglich als Naturstoffextrakt Opium verwendet, seit langer Zeit zur Behandlung von Schmerz eingesetzt werden. Die ersten Versuche, ein Morphin-Derivat ohne Abhängigkeitspotential zu synthetisieren, wurden bereits 1874 unternommen. Es zeigte sich jedoch, dass die resultierende Substanz - Heroin - kein verbessertes Nebenwirkungsprofil im Vergleich zu Morphin aufwies. Bis heute wurden zahlreiche weitere Versuche unternommen, stark wirksame Analgetika mit verbessertem Nebenwirkungsprofil herzustellen. So wurde 1925 Oxycodon synthetisiert, 1946 Methadon, 1961 Fentanyl und 1965 Tilidin. Es stellte sich jedoch heraus, dass eine deutliche Reduktion der Nebenwirkungen mit einer deutlichen Reduktion der Wirksamkeit einhergeht. Die μ-typischen Nebenwirkungen sind gut untersucht; sie sind mit dem μ-Antagonisten Naloxon antagonisierbar und gehören somit zum Wirkprofil von μ-Opioiden. Bis heute gibt es keine Arzneimittel, die die gleiche Wirkstärke wie die klinisch eingesetzten μ-Opioide der Stufe 3 (WHO-Stufenschema) wie Fentanyl, Sufentanil, Morphin, Oxycodon, Buprenorphin und Hydromorphon, und gleichzeitig ein signifikant reduziertes Nebenwirkungsprofil besitzen.

Zusammenfassend kann festgehalten werden, dass die Therapie von mittelstarkem bis starkem Schmerz, sowohl im akutem als auch im chronischen Schmerz, trotz all ihrer Nachteile zu einem großen Teil auf der Verwendung von μ-Opioiden beruht. Dies resultiert vor allem aus der hohen Wirkstärke dieser Verbindungen. Die Nachtelle sind jedoch so beträchtlich, dass viele Patienten aus Furcht vor Nebenwirkungen - sowohl aus eigenen Überlegungen wie auch aus den Bedenken des Arztes - die notwendige Therapie nicht erhalten. Es besteht daher ein dringender Bedarf an neuartigen Schmerztherapeutika, die auf einem Wirkprinzip beruhen, das einerseits die hohe Wirksamkeit der μ-Opioide mitbringt, die Nachteile wie Abhängigkeit, erhöhte Schmerzempfindung, Atemdepression und verminderte Wirksamkeit im chronischen Schmerz jedoch reduziert.

Aufgabe der vorliegenden Erfindung war daher, ein Wirkprinzip für Arzneimittel zu finden, wobei Arzneimittel, die nach diesem Prinzip wirken, einerseits die hohe Wirksamkeit der μ-Opioide mitbringen, die Nachteile wie Abhängigkeit, Atemdepression und verminderte Wirksamkeit im chronischen Schmerz jedoch Im Vergleich zu μ-Opioiden in verringertem Maße aufweisen.

Die Aufgabe wird durch die vorliegende Erfindung gelöst.

Gegenstand der Erfindung ist die Verwendung von gemischten ORL1/μ-Agonisten gemäß Formel (I), die eine Affinität zum μ-Opioid-Rezeptor von mindestens 100 nM ($K_i$-Wert human) und eine Affinität zum humanen ORL-1-Rezeptor aufweisen, wobei das Verhältnis zwischen den Affinitäten ORL1/μ definiert als $1/[K_{t(ORL1)}/K_{t(\mu)}]$ zwischen 0,1 und 30 liegt,

- zur Anwendung bei einer Narkose;
- zur Anwendung bei der narkosebegleitenden Behandlung von Schmerz;
- zur Anwendung bei der Behandlung von neuropathischem Schmerz ausgewählt aus der Gruppe bestehend aus chronischem neuropathischem Schmerz, polyneuropathischem Schmerz und Cytostatika-induziertem Polyneuropathleschmerz.

Erfindungsgemäße gemischte ORL1/μ-Agonisten sind spirocyclische Cyclohexan-Derivate der allgemeinen Formel (I):

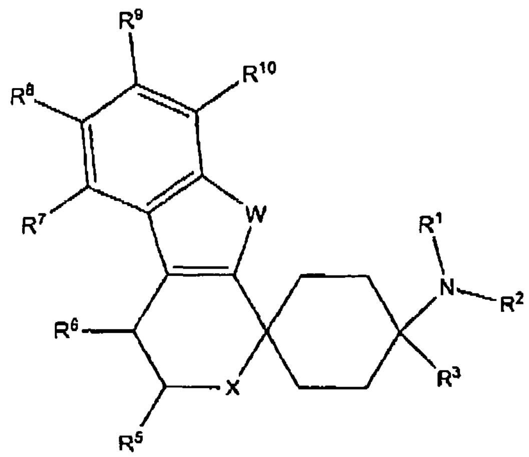

(I)

worin

$R^1$ und $R^2$ unabhängig voneinander für H oder $CH_3$ stehen, wobei $R^1$ und $R^2$ nicht gleichzeitig H bedeuten;
$R^3$ für Phenyl, Benzyl oder Heteroaryl jeweils unsubstituiert oder einfach oder mehrfach mit F, Cl, OH, CN oder $OCH_3$ substituiert, steht;
W für $NR^4$. O oder S steht; wobei
$R^4$ für H; $C_{1-5}$-Alkyl Phenyl: über eine $C_{1-3}$-Alkyl-Gruppe gebundenes Phenyl, $COR^{12}$; $SO_2R^{12}$ steht,
$R^{12}$ H; $C_{1-7}$-Alkyl, verzweigt oder unverzweigt, gesättigt oder ungesättigt, unsubstituiert oder einfach oder mehrfach mit OH, F oder $COOC_{1-4}$-Alkyl substituiert; $C_{4-6}$-Cycloalkyl; Aryl-, oder Heteroaryl, unsubstituiert oder einfach oder mehrfach mit F, Cl, Br, $CF_3$, $OCH_3$, $C_{1-4}$-Alkyl, verzweigt oder unverzweigt, unsubstituiert oder substituiert mit F, Cl, CN, $CF_3$, $OCH_3$ oder OH: oder über gesättigtes oder ungesättigtes $C_{1-3}$-Alkyl gebundenes Phenyl oder Heteroaryl, unsubstituiert oder einfach oder mehrfach mit F, Cl, Br, $CF_3$, $OCH_3$, $C_{1-4}$-Alkyl, verzweigt oder unverzweigt, unsubstituiert oder substituiert mit F, Cl, CN, $CF_3$, $OCH_3$ oder OH substituiert; oder über gesättigtes oder ungesättigtes $C_{1-3}$-Alkyl gebundenes $C_{5-6}$-Cycloalkyl; $OR^{13}$; $NR^{14}R^{15}$ bedeutet;
$R^6$ für H; $COOR^{13}$, $CONR^{13}$. $OR^{13}$; $C_{1-5}$-Alkyl, gesättigt oder ungesättigt, verzweigt oder unverzweigt, unsubstituiert oder einfach oder mehrfach mit OH, F, $CF_3$ oder CN substituiert, steht;
$R^6$ für H steht;
oder $R^5$ und $R^6$ gemeinsam $(CH_2)_n$ mit n = 2, 3, 4, 5 oder 6 bedeuten, wobei einzelne Wasserstoffatome auch durch F, Cl, $NO_2$, $CF_3$, $OR^{13}$, CN oder $C_{1-5}$-Alkyl ersetzt sein können;
$R^7$, $R^8$, $R^9$ und $R^{10}$ unabhängig voneinander für H, F, Cl, Br, $NO_2$, $CF_3$, OH, $OCH_3$, CN, $COOR^{13}$, $NR^{14}R^{15}$; $C_{1-5}$-Alkyl, Heteroaryl, unsubstituiert oder einfach oder mehrfach mit Benzyl, $CH_3$, Cl, F, $OCH_3$ oder OH substituiert, stehen; wobei $R^{13}$ H oder $C_{1-5}$-Alkyl bedeutet;
$R^{14}$ und $R^{15}$ unabhängig voneinander H oder $C_{1-5}$-Alkyl bedeuten;
X für O, S, SO, $SO_2$ oder $NR^{17}$ steht;
$R^{17}$ für H; $C_{1-5}$-Alkyl, gesättigt oder ungesättigt, verzweigt oder unverzweigt; $COR^{12}$ oder $SO_2R^{12}$ steht;
in Form ihrer reinen Diastereomeren, ihrer Racemate, ihrer reinen Enantiomeren, oder in Form von Mischungen der Stereoisomeren in einem bellebigen Mischungsverhältnis; als Basen oder in Form ihrer Salze, insbesondere der physiologisch verträglichen Salze oder Salze physiologisch verträglicher Säuren oder Kationen.

Die $K_i$-Werte werden an rekombinanten CHO-Zellen bestimmt, die den jeweiligen Rezeptor exprimieren.

Der Begriff "ORL1/μ definiert als $1/[K_{(ORL\ 1)}/K_{(\mu)}]$" wird in verkürzier Form als "ORL1/μ" bezeichnet. Der Begriff "mindestens 100 nM" bedeutet, dass die Affinität bei 100 nM oder besser (= niedrigerer $K_t$-Wert, beispielsweise 99,9 nM) liegt.

Überraschenderweise hat sich herausgestellt, dass Verbindungen, die ein Verhältnis von ORL1/μ von 0,1 bis 30 aufweisen, ein Fenster bilden, in dem die ORL-1-Komponente zwar eine deutliche Reduktion einiger μ-typischer Nebenwirkungen wie Atemdepression und Abhängigkeit bewirkt, die antiopioide Wirkung dieser Komponente jedoch noch nicht die analgetische Wirkung im Akutschmerz verhindert. Im Gegensatz zum Akutschmerz kommt es bei chronischen Schmerzzuständen sogar zu einem analgetischen Synergismus von ORL1- und μ-Komponente, d. h., von den jeweiligen Beiträgen, die die Wirkung der Verbindungen an dem einzelnen Rezeptor zur Gesamtwirksamkeit liefert. Dadurch wird bei Verbindungen, die ein Verhältnis von ORL1 zu μ definiert als $1/[K_{i(ORL1)}/K_{t(\mu)}]$ von 0,1 bis 30 aufweisen eine deutlich erhöhte Wirksamkeit erreicht, die es erlaubt, die Dosis im Vergleich zum Akutschmerz zu reduzieren, um zu einer zufrieden stellenden Wirkung zu kommen. Vorzugsweise beträgt das Verhältnis von ORL1 zu μ definiert als $1/[K_{t(ORL1)}/K_{t(\mu)}]$ 0,1 bis 20. Bei den erfindungsgemäßen Verbindungen kann es sich auch um Metabolite einer Muttersubstanz handeln, wobei die Metabolite einzeln oder als Metabolitengemisch in Kombination mit der verbleibenden Menge der Muttersubstanz die erfindungsgemäßen Eigenschaften besitzen können.

Für die Wirksamkeit der Verbindungen ist es wichtig, dass die Affinität der Verbindungen oder die Affinität der Metaboliten zum μ-Opioid-Rezeptor mindestens 100 nM ($K_I$-Wert human) beträgt. Dieser Wert liegt im Bereich gut wirksamer μ-Opioide in klinischer Anwendung wie Hydrocodon (human μ-OR Ki 76 nM), Ketobemidon (human μ-OR Ki 22 nM) und Meptazinol (Ki 150 nM human μ-OR). Bevorzugt beträgt die Affinität der Verbindungen zum μ-Opioid-Rezeptor mindestens 50 nM.

Die genannten überraschenden Eigenschaften von Verbindungen mit den erfindungsgemäßen Eigenschaften wurden durch zahlreiche Tierexperimente belegt. Die Verbindungen weisen eine Bandbreite an ORL1/μ-Anteilen auf und belegen die Ausnahmestellung der gemischten ORL1/μ-Agonisten im erfindungsgemäßen Bereich. Für die Vergleichsuntersuchungen wurden Arzneimittel ausgewählt, die heute für die Behandlung von starken Schmerzen angewendet werden. Bei den Referenzsubstanzen B1-B6 handelt es sich um die μ-Opioide Fentanyl, Sufentanil, Morphin, Oxycodon, Buprenorphin und Hydromorphon, die alle zu den Stufe 3-Opioiden gemäß WHO Stufenschema zur Behandlung von Schmerz gehören. Diese Arzneimittel repräsentierten den Goldstandard für die Therapie von starken Schmerzen heutzutage.

Der ORL1-Rezeptor ist homolog zu den μ, κ und δ Opioid-Rezeptoren und die Aminosäuresequenz des endogenen Liganden, des Nociceptin-Peptids, weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase, zu einer Inhibierung von spannungsabhängigen Calciumkanälen und einer Aktivierung von Kaliumkanälen (Meunier et al., Nature 377, 1995, S. 532-535; Ronzoni et al., Exp Opin Ther Patents 2001, 11, 525-546).

Das Nociceptin-Peptid zeigt nach intra cerebro venticulärer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337).

Auf der anderen Seite konnte in verschiedenen Tiermodellen nach intrathekaler Applikation, auch ein antinociceptiver Effekt von Nociceptin gezeigt werden. (Abdulla und Smith, J. Neurosci., 18, 1998, S. 9685-9694). Nociceptin besitzt also, je nach Wirkort und physiologischem Zustand des Organismus, antinociceptive als auch pronociceptive Eigenschaften.

Weiterhin ist bekannt, dass der endogene ORL-1-Ligand Nociceptin eine Wirkung im neuropathischen Schmerz aufweist. Darüber hinaus konnte nachgewiesen werden, dass Nociceptin und Morphin eine synergistische Wirkung im neuropathischen Schmerz zeigen (Courteix et al., Pain 2004, 110, 236-245). Nociceptin allein ist nach systemischer Gabe im Akutschmerz (gemessen im Tail-flick test) jedoch nicht wirksam. Reine ORL1-Agonisten sind daher möglicherweise zur Behandlung von neuropathischem Schmerz geeignet. Tritt der zu behandelnde Schmerz in einer Mischform auf oder tritt der bei neuropathischen Schmerzen typische Spontanschmerz auf, sind reine ORL-1-Agonisten nach den Befunden aus Tierexperimenten jedoch nicht ausreichend wirksam.

Aus der Literatur sind gemischte ORL1/μ-Agonisten bereits bekannt, beispielsweise aus EP 0997464 oder WO 1999059997. Diese Dokumente offenbaren jedoch nur Strukturen, die ohne Angabe von konkreten biologischen Daten als gemischte ORL1/μ-Agonisten beschrieben werden, offenbaren jedoch nicht, dass Verbindungen im erfindungsgemäßen Affinitätsbereich Vorteile aufweisen. WO 2001039775 offenbart gemischte ORL1/μ-Agonisten sowie einen allgemeinen, nicht näher spezifizierten Bereich, in dem Verbindungen eine ORL1- und μ-Affinität besitzen können, jedoch ohne Nachweis eines Vorteils solcher Verbindungen.

**Wirkverstärkung bei chronischen Schmerzen gegenüber reinen μ-Opioiden**

**a) Neuropathischer Schmerz**

In Gegensatz zu klassischen μ-Agonisten kann überraschenderweise bei gemischten ORL1/μ-Agonisten im Bereich von 0,1 bis 30, vorzugsweise bis 20, eine deutliche Erhöhung der analgetischen Wirksamkeit in Neuropathieschmerzmodellen beobachtet werden. In Antagonisierungs-Experimenten wurde gezeigt, dass die ORL1-Komponente bei gemischten ORL1/μ-Agonisten einen direkten Beitrag zur analgetischen Wirkung liefert (Abb. 3). Der direkte Vergleich einer Substanz mit einem ORL1/μ-Verhältnis von 0,5 (Verbindung A4) und Morphin in naiven und neuropathischen Tieren zeigt, dass nach Ausbildung der Neuropathie die Wirksamkeit von Morphin abnimmt (was der klinischen Situation entspricht), während sie für die gemischten Agonisten tendenziell zunimmt (Abb. 5, 5a, 6, 6a).

Im Vergleich der analgetischen Wirksamkeit im Akutschmerzmodell (Tail-flick, Ratte/Maus) und in den Neuropathieschmerzmodellen Chung-Modell an der Ratte sowie dem Bennett-Modell an Ratte/Maus zeigt sich die Ausnahmestellung der Verbindungen mit den erfindungsgemäßen Eigenschaften (s. Abbildungen 1 und 2). Im Gegensatz zu reinen μ-Opioiden, bei denen die analgetische Potenz im Neuropathieschmerzmodell geringer ist als im Akutschmerzmodell (bis zu Faktor 5), ist die analgetische Potenz von gemischten ORL1/μ-Agonisten im Neuropathieschmermodell

um einen Faktor 2 bis 10 höher als im Akutschmerzmodell. So ist beispielsweise das klinisch eingesetzte μ-Opioid Oxycodon im Neuropathieschmerz drei- bis fünffach weniger potent im Vergleich zum Akutschmerz (abhängig vom Tiermodell); ein gemischter Agonist mit einem ORL1/μ-Verhältnis von 0,5 (Verbindung A4) ist dagegen im Neuropathieschmerz ca zehnfach potenter als im Akutschmerz.

Die obere Grenze des Bereichs, in dem der Effekt auftritt, wird durch die Verbindungen B8 belegt, die ein ORL1/μ-Verhältnis von 0.03 aufweist und im Neuropathie-Modell nicht mehr besser wirksam ist als im Akutschmerzmodell. Das Beispiel A1 (ORL1/μ-Verhältnis 0,1) ist dagegen noch um den Faktor 10 besser wirksam.

Die Verbindung A11 mit einem ORL1/μ-Verhältnis von 20 weist intrathekal gegeben, eine noch höhere Wirkverstärkung im Neuropathieschmerz auf. Die Verbindung ist im Akutschmerz nach systemischer Gabe nach wie vor gut wirksam (Tail-flick Maus i. v. $ED_{50}$ = 0,42 mg/kg). Verbindung B9 mit einem ORL1/μ-Verhältnis von 140:1 weist, intrathekal gegeben, ebenfalls eine hohe Wirkverstärkung im Neumpathieschmerz auf. Die Verbindung ist nach systemischer Gabe im Akutschmerz aufgrund der zu geringen μ-Komponente jedoch nicht mehr wirksam. Der endogene ORL-1-Ligand Nociceptin weist keine Wirkung im Akutschmerzmodell (Tail-flick i. v.) mehr auf. Aufgrund der antiopioiden Eigenschaften der ORL1-Komponente ist bei Verbindungen, die eine ORL1-Komponente besitzen, die deutlich besser ist als 30:1 im Vergleich zur μ-Komponente, die Wirkung im Akutschmerz zu schlecht um in der Wirkstärke mit einem Stufe-3-Opioid vergleichbar zu sein. Dieser Zusammenhang kann durch die Antagonisierung der ORL1-Komponente dargestellt werden. Die Befunde zeigen, dass die Verbindungen mit den erfindungsgemäßen Eigenschaften eine definierte Untergruppe von gemischten μ/ORL1-Agonisten bilden, die die offenbarten außerordentlichen Eigenschaften besitzen. Die untere Grenze des erfindungsgemäßen Bereichs liegt daher bei 30, vorzugsweise bei 20.

In Antagonisierungsexperimenten im Chung-Modell wurde bewiesen, dass die analgetische Wirksamkeit der gemischten ORL1/μ-Agonisten auf beiden Komponenten beruht. Nach Verabreichung von gemischten ORL1/μ-Agonisten kann mit einem μ-Antagonisten wie auch mit einem ORL1-Antagonisten jeweils eine partielle Aufhebung der analgetischen Wirkung gezeigt werden (Abbildungen 3 und 4). Dies bestätigt, dass sowohl die μ-Opioid-Komponente als auch die ORL1-Komponente im chronischen neuropathischen Schmerz zur Wirkung beitragen.

Die Antagonisierungsexperimente zeigen deutlich, dass die erfindungsgemäßen Eigenschaften direkt auf die ORL1-agonistische und die μ-agonistische Wirkung der Verbindungen zurückzuführen sind.

Um einen möglichen Einfluß der Schmerzqualität" (Tail-flick, nociceptiver Reiz vs. Chung, taktile Allodynie) beim Vergleich der unterschiedlichen Wirksamkeit bei Akutschmerz und Neuropathieschmerz auszuschließen, wurden A4 und Morphin vergleichend in Chung-Tieren und scheinoperierten Tieren getestet. Als Schmerzmodell wurde in allen Fällen der Tail-flick verwendet. Der direkte Vergleich zeigt, dass Morphin zwar eine sehr gute Wirkung am scheinoperierten Tier aufweistwas der Situation im Akutschmerz entspricht -, dass aber nach Ausbildung der Neuropathie im operierten Tier die Wirksamkeit von Morphin vergleichsweise deutlich geringer ist (Abbildung 7). Dies entspricht der klinischen Situation und bildet eines der Probleme von μ-Opioiden in der Klinik ab, A4 dagegen zeigt eine deutliche Wirkung am scheinoperierten Tier, die nach Ausbildung der Neuropathie sogar noch zunimmt (Abbildung 8). Dies zeigt den deutlichen Vorteil von gemischten ORL1/μ-Agonisten Im Vergleich zu reinen μ-Opioiden bei der Behandlung von Neuropathieschmerz.

Die erfindungsgemäßen Verbindungen mit einem ORL1/μ-Verhältnis definiert als $1/[K_{(ORL1)}/K_{(\mu)}]$ von 0,1 bis 30, vorzugsweise von 0,1 bis 20, bei einem $K_i$-Wert am μ-Opioid-Rezeptor von unter 100 nM können daher zur Behandlung von neuropathischern Schmerz verwendet werden.

**Trennung von antinociceptivem und antlailodynischem Effekt bei neuropathischen Tieren**

Einen weiteren Vorteil zeigen die gemischten ORL1/μ-Agonisten im erfindungsgemäßen Bereich durch die Trennung von antinociceptivem und antiallodynischem Effekt. Bei der sogenannten Allodynie wird durch einen an einer nicht betroffenen Körperregion sicher nichtschmerzhaften Reiz (z. B. Berührung, Warm-, Kaltrelz) Schmerz evoziert Die mechanische Allodynie ist typisch bei der postzosterischen Neuralgie, die Källeallodynle ist häufig bei posttraumatischen Nervenläsionen und einigen Polyneuropathien. Insbesondere bei diabetischer Neuropathie ist das Auftreten von mechanischer Allodynie typisch (Calcutt and Chaplan, Br. J. Pharmacol. 1997, 122, 1478-1482).

Bei bestimmten Patientengruppen von chronischen Schmerzpatienten ist es von Vorteil, wenn Allodynie und Hyperalgesie bekämpft werden, das normale Schmerzempfinden jedoch weitgehend bestehen bleibt. Diese Patienten, für die im täglichen Leben der Schutzmechanismus des Schmerzes sinnvoll ist, benötigen daher eine Medikation, die speziell nur Allodynie und Hyperalgesie bekämpft, das allgemeine Schmerzempfinden aber möglichst unberührt lässt Dies gilt beispielsweise für die postzosterischer Neuralgie, bei der typischerweise Schmerzen durch Reize ausgelöst werden, die üblicherweise überhaupt nicht schmerzhaft sind, wie z.B. durch leichte Berührungen oder Kleidung.

Im Chung-Modell kann durch vergleichende Testung der Schmerzreaktion an der ipsi- und an der contralateralen Pfote (bezogen auf die Seite, auf der die Spinalnervenligatur gesetzt wurde) zwischen antinociceptiver (contralateral) und antiallodynischer (ipsilateral) Wirkung unterschieden werden.

Für den μ-Agonisten Morphin konnte eine rein antiallodynische Wirkung nur nach Applikation von 1 mg/kg iv.

beobachtet werden. Die maximale Wirksamkeit beträgt dabei 29% MPE (maximal möglicher Effekt), was zwar einer erkennbaren, jedoch schwachen Wirkung entspricht. Bei der nächst höheren Testdosierung (2,15 mg/kg iv.) tritt bereits eine deutliche antinociceptive Wirkung ein (Abbildungen 5, 5a). Somit ist hier eine klare Trennung zwischen deutlichem antiallodynischen Effekt und antinociceptivem Effekt bei Morphin nicht erreichbar.

Im Gegensatz dazu beträgt die maximale, rein antiallodynische Wirkung von A4 56% MPE. Diese wird bei einer Testdosierung von 1 $\mu$g/kg iv. erreicht und entspricht einer guten Wirksamkeit (Abbildungen 6, 6a). Dies zeigt einen weiteren Vorteil der gemischten ORL1/$\mu$-Agonisten im Vergleich zu reinen $\mu$-Opioiden.

Es ist daher auch bevorzugt, die Verbindungen mit einem ORL 1/$\mu$-Verhältnis von 0,1 bis 30, vorzugsweise von 1:10 bis 20:1, bei einem $K_i$-Wert am $\mu$-Opioid-Rezeptor von unter 100 nM zur Behandlung von Allodynie, Hyperalgesie und Spontanschmerzen zu verwenden, vorzugsweise in einer Dosierung, in der das allgemeine Schmerzempfinden weitgehend erhalten bleibt. Die Erhaltung des allgemeinen Schmerzempfindens am Menschen kann im cold pressor Modell überprüft werden (Enggaard et al., Pain 2001, 92, 277-282).

Welterhin ist es bevorzugt, die Verbindungen mit einem ORL1/$\mu$-Verhältnis von 0,1 bis 30, vorzugsweise von 0,1 bis 20, bei einem $K_i$-Wert am $\mu$-Opioid-Rezeptor von unter 100 nM zur Behandlung von Schmerzen bei postzosterischer Neuralgie, einzusetzen.

Zur genaueren Untersuchung der vielfältigen Neuropathieschmerzformen wurde A4 in einem Modell zur Untersuchung des Cytostatika-induzierten Polyneuropathieschmerzes untersucht. Der Cytostatika-induzierte Polyneuropathieschmerz bildet eine klinisch sehr relevante Untergruppe des Neuropathleschmerzes. Die Polyneuropathie wurde durch Verabreichung des Cytostatikums Vincristin ausgelöst. Somit entstand an der Ratte ein Krankheitsbild, das die klinische Situation nach einer Chemotherapie mit Vincristin abbildet. Als Vergleichssubstanz wurde hier Morphin untersucht.

A4 zeigte eine signifikante Wirksamkeit ab einer Dosierung von 1 $\mu$g/kg, d. h., ab einer Dosierung, die im Bereich des $ED_{50}$ im chronischen Schmerz liegt Bei der niedrigeren Dosierung von 0,464 $\mu$g/kg war jedoch noch keine signifikante Wirksamkeit zu sehen (Abb. 24). Für Morphin wird eine gute Wirksamkeit ab einer Dosierung von 2,15 mg/kg beobachtet ($ED_{50}$ Chung Ratte 3,7 mg/kg).

Weiterhin wurde die Wirksamkeit gegen diabetisch induzierten Polyneuropathieschmerz untersucht Diese Schmerzform wurde in einem Modell an der Ratte untersucht, wobei durch Verabreichung von Streptozotocin diabetische Polyneuropathie ausgelöst wurde. A4 zeigte bereits bei der niedrigsten getesteten Dosierung von 0,316 $\mu$g/kg i. v. eine signifikante Hemmung der diabetisch induzierten mechanischen Hyperalgesie an der Ratte und damit in einem niedrigeren Dosisbereich als im Cytostatika-induzierten Polyneuropathleschmerz, worin bei einer Dosierung von 0,464 $\mu$g/kg noch keine signifikante Wirksamkeit zu beobachten war. In diesem niedrigen Dosisbereich hatte A4 keinen Effekt auf die Kontrollgruppe. Das bedeutet, dass im diabetisch induzierten Polyneuropathieschmerz

1.) überraschenderweise die Wirksamkeit von A4 noch einmal besser ist als in anderen Neuropathieschmerzformen und

2.) die anti-hyperalgetische Wirkung von A4 bereits in einem Dosisbereich vorhanden ist, in dem noch keine antinociceptive Wirkung auftaucht (Abbildung 26) und damit die Linderung des Polyneuropathieschmerzes möglich ist, ohne das Akutschmerzempfinden zu beeinträchtigen.

Bei Morphin dagegen ist eine anti-hyperalgetische Wirkung erst in einem Dosisbereich zu beobachten, in dem auch eine antinociceptive Wirkung in der Kontrollgruppe auftaucht (Abbildung 27). Da im diabetisch induzierten Polyneuropathieschmerz die Standardtherapie heute nicht die Verabreichung eines $\mu$-Agonisten wie Morphin, sondern unter anderem die Gabe von Pregabalin ist, wurde Pregabalin als weiterer Vergleich im gleichen Modell untersucht. Auch hier zeigte sich, dass eine anti-hyperalgetische Wirkung erst in einem Dosisbereich zu beobachten ist, in dem auch eine antinociceptive Wirkung in der Kontrollgruppe auftaucht (Abbildung 28). Dies unterstreicht die außergewöhnliche Wirksamkeit der Verbindungen mit den erfindungsgemäßen Eigenschaften im diabetisch induzierten Polyneuropathieschmerz.

Daher werden Verbindungen mit einem ORL1/$\mu$-Verhältnis von 0,1 bis 30, vorzugsweise von 0,1 bis 20, bei einem $K_i$-Wert am $\mu$-Opioid-Rezeptor von unter 100 nM besonders bevorzugt zur Behandlung von diabetischem Polyneuropathieschmerz eingesetzt.

**b) Entzündungsschmerz**

In zwei in-vivo-Modellen (Single-Motor-Unit Ableitung an spinalisterten Ratten und CFA**-induzierte Hyperalgesie) konnte gezeigt werden, dass nach chronischer Entzündung die Wirksamkeit von gemischten ORL1/$\mu$-Agonisten erhöht ist.

**Single-Motor-Unit Ableitungen in spinalisierten Ratten. Vergleich von naiven Tieren und Tieren nach Carrageenan-induzierter Entzündung.**

In der Ratte wird beobachtet, dass 24 h nach Induktion einer Entzündung die antinociceptive Wirkung von A4 (Verhältnis ORL1/μ 1:2, Abbildungen 9 und 10) und A11 (Verhältnis ORL1:μ 20:1) im Vergleich zum Wert vor der Entzündung deutlich erhöht ist. Die antinociceptive Wirkung des μ-Agonisten Morphin ist dagegen nach Entzündung tendenziell schwächer. (Abildungen 11 und 11a). Dies zeigt, dass nach chronischer Entzündung die Wirksamkeit von gemischten ORL1/μ-Agonisten erhöhtist, die reiner μ-Agonisten dagegen nicht

**CFA-Induzierte Hyperalgesie**

In einem Modell für chronischen Entzündungsschmerz wurde durch injektion von CFA eine Entzündung der Hinterpfote induziert. 1h, 3h, 24h und 4 Tage nach Entzündungsinduktion wurden die taktile Hyperalgesie und die Nociception bestimmt. Während Morphin über den gesamten Untersuchungszeitraum eine leicht abfallende antihyperalgetische bzw. eine gleich bleibende antinociceptive Wirkung zeigte, nahmen antihyperalgetische und antinociceptive Wirkung von A4 über 24 h zu. Der Effekt ist für mindestens 4 Tage stabil (Abbildungen 12 und 12a). Dies zeigt, dass, analog zur Situation beim neuropathischen Schmerz, sich gemischte ORL1/μ-Agonisten im Entzündungsschmerz durch eine deutliche Wirkverstärkung gegenüber der Analgesie bei Akutschmerz auszeichnen.

**Visceraler Entzündungsschmerz**

Die vergleichende Testung von A4 und Fentanyl In einem Modell zur übertragenen Allodynie und zur übertragenen Hyperalgesie in Mäusen nach Senföl induzierter, nichtneurogener visceraler Entzündung zeigte für beide Schmerzparameter eine signifikant höhere Wirksamkeit des gemischten ORL1/μ-Agonisten im Vergleich zu dem reinen μ-Opioid. Die analgetische Wirksamkeit von A4 in bezug auf beide getesten Schmerzparameter ist um ca. einen Faktor 6 bis 7 höher als im Akutschmerz. Im Gegensatz dazu ist die analgetische Wirksamkeit von Fentanyl im visceralen Entzündungsschmerz geringer als im Akutschmerz. Dies zeigt gleichfalls, dass, analog zur Situation beim neuropathischen Schmerz, sich gemischte ORL1/μ-Agonisten durch eine deutliche Wirkverstärkung der Analgesie bei viszeralem Entzündungsschmerz gegenüber dem Akutschmerz auszeichnen. Die Verbindungen zeigen damit neben den verminderten Nebenwirkungen im Vergleich zu reinen μ-Opioiden auch eine bessere Wirksamkeit im Entzündungsschmerz.

Gemischte ORL1/μ-Agonisten mit einem ORL1:μ-Verhältnis von 0,1 bis 30, vorzugsweise von 0,1 bis 20, bei einem $K_r$Wert am μ-Opioid-Rezeptor von unter 100 nM, zeichnen sich demnach durch eine hohe Wirksamkeit im Entzündungsschmerz aus. Gegenstand der Erfindung ist daher auch die Verwendung von Verbindungen einem ORL1:μ-Verhältnis von 0,1 bis 30, vorzugsweise von 0,1 bis 20, bei einem $K_r$-Wert am μ-Opioid-Rezeptor von unter 100 nM zur Behandlung von Patienten, die an Entzündungsschmerz leiden. Der Entzündungsschmerz kann beispielsweise durch rheumathoide Arthritis oder Pankreatitis, ausgelöst werden.

Es wurde gezeigt, dass gemischte ORL1/μ-Agonisten mit einem ORL1:μ-Verhältnis von 0,1 bis 30, vorzugsweise von 0,1 bis 20, bei einem $K_i$-Wert am μ-Opioid-Rezeptor von unter 100 nM im chronischen Schmerz eine Wirkverstärkung im Vergleich zu Akutschmerz zeigen. Es ist daher bevorzugt, die Verbindungen im chronischen Schmerz in einer Dosierung anzuwenden, die unterhalb der im Akutschmerz notwendigen Dosierung liegt. Vorzugsweise werden die Verbindungen im chronischen Schmerz in einer Dosierung angewendet, die um mindestens einen Faktor 2 niedriger liegt als die im Akutschmerz angewandte Dosierung, besonders bevorzugt um mindestens einen Faktor 5 niedriger. Am Tier kann die Dosierung als $ED_{50}$ im Tail-flick bestimmt werden, am Menschen im cold pressor Modell (Enggaard et al., Pain 2001, 92, 277-282).

**c) Akutschmerz**

Die gemischten ORL1/μ-Agonisten mit einem ORL1 : μ-Verhältnis von 0,1 bis 30, vorzugsweise von 0,1 bis 20, zeigen volle Wirksamkeit in verschiedenen Akutschmerzmodelien und Species nach i. v.-Gabe. Sowohl an der Ratte als auch an der Maus (Tail-flick, Abbildung 13) konnte dieser Effekt gezeigt werden.

Im Vergleich der gemischten ORL1/μ-Agonisten mit reinen μ-Agonisten zeigen die gemischten ORL1/μ-Agonisten eine vergleichbare Wirksamkeit bei besserer Verträglichkeit. Diese Ergebnisse zeigen, dass die gemischten ORL1/μ-Agonisten auch im Akutschmerz eine hervorragende Wirksamkeit aufweisen. Die Verbindungen sind in ihrer Wirksamkeit im Akutschmerz vergleichbar mit den Stufe-3 Opioiden. Das bedeutet, dass hier Verbindungen vorliegen, die ihre analgetische Wirkung über einen anderen Mechanismus ausüben, als die reinen μ-Antagonisten, die seit Jahrhunderten das Segment der Behandlung starker Schmerzen beherrschen, jedoch die gleiche Wirkstärke besitzen. Neben ihrer überraschenden Wirkverstärkung im chronischen Schmerz im Vergleich zum Akutschmerz zeigen Verbindungen mit dem erfindungsgemäßen Bindungsprofil auch ein deutlich verbessertes Nebenwirkungsprofil im Vergleich zu den

reinen Agonisten.

**d) Nebenwirkungen**

**Opioid-Induzierte Hyperalgesie**

Die chronische Gabe von Opioiden führt zu einer Hyperalgesie bei Schmerzpatienten (vgl. Chu et al. 2006, J. Pain 7:43-48). Ein ähnliches Phänomen tritt auch nach akuter Gabe in der Entzugssituation auf (Angst et al. 2003, Pain 106:49-57). Im Tiermodell induzieren reine μ-Oploide nach akuter Gabe eine transiente Hyperalgesie, die z.B. im Soft Tall-flick-Modelt als transiente ‚pronociceptive" Phase detektierbar ist.
Diese oploidinduzierte Hyperalgesie kann mit Hilfe eines modifizierten Tail-flick-Modells mit verminderter Reizstärke (25% Brennstrahlintensität) für reine μ-Oploide (Fentanyl und Morphin) gezeigt werden. Im Gegensatz dazu wurde nach akuter Gabe von gemischten ORL1/μ-Agonisten (A4 und A7) keine transiente Hyperalgesie beobachtet (Abbildungen 14-14c).

Dies zeigt, dass bei chronischer Gabe eines gemischten ORL1/μ-Agonisten keine bzw. eine gegenüber reinen μ-Opioiden verringerte Hyperalgesie induziert wird. Eine der typischen μ-Opioid-Nebenwirkungen ist in gemischten ORL1/μ-Agonisten demzufolge reduziert.

Bevorzugt werden daher die Verbindungen mit einem ORL1/μ-Verhältnis von 0,1 bis 30, vorzugsweise von 0,1 bis 20, bei einem $K_i$-Wert am μ-Opioid-Rezeptor von unter 100 nM zur Reduktion der Opioid-induzierten Hyperalgesie bei der Behandlung von Schmerzen verwendet.

Besonders vorteilhaft ist der Einsatz der Verbindungen mit einem ORL1/μ-Verhältnis von 0,1 bis 30, vorzugsweise von 0,1 bis 20, bei einem $K_i$-Wert am μ-Opioid-Rezeptor von unter 100 nM bei der Behandlung von Patienten, die ein erhöhtes Risiko haben, Hyperalgesie zu entwickeln. Dazu gehören beispielsweise Patienten, die bereits an einer Hyperalgesie leiden und sich einer Operation unterziehen müssen wie etwa Reizdarmpatienten (viszerale Hyperalgesie), Tumorschmerzpatienten und Patienten mit muscoloskeletalen Schmerzen oder Patienten, die intraoperativ intrathekal ein stark wirksames Opioid wie Fentanyl erhalten haben (z. B. Kalserschnittpatientinnen). Gegenstand der Erfindung ist daher auch die Verwendung von Verbindungen mit einem ORL1/μ-Verhältnis von 0,1 bis 30, vorzugsweise von 0,1 bis 20, bei einem $K_r$-Wert am μ-Opioid-Rezeptor von unter 100 nM zur Schmerzlinderung bei Patienten, die ein erhöhtes Risiko haben, eine Hyperalgesie zu entwickeln.

**Entzug**

Im Naloxon-induzierten Entzugsspringen an der Maus konnte gezeigt werden, dass Verbindungen mit einer ORL-1-Komponente, die weniger als einen Faktor 10 schwächer ist als die μ-Komponente, das Entzugsspringen unterdrücken. Verbindungen mit einer schwächeren ORL1-Komponente lösen dagegen Entzugsspringen aus. Im Test "Entzugsspringen" werden Mäuse über einen definierten Zeitraum hinweg mehrfach mit der Testsubstanz behandeit. Bei einem μ-Opioid wird innerhalb dieses Zeitraumes eine körperliche Abhängigkeit erreicht. Am Ende der Behandlung wird durch Gabe von Naloxon, einem μ-Antagonisten, die Wirkung des Opioids mit einem Mal aufgehoben. Bei Ausprägung einer körperlichen Abhängigkeit zeigen die Mäuse charakteristische Entzugssymptome, die sich in Form von Sprungbewegungen äußern (Saelens JK, Arch Int Pharmacodyn 190: 213-218, 1971).

Die Verbindungen mit den erfindungsgemäßen Eigenschaften besitzen auf Grund der ORL1-Wirkkomponente zusätzliche Eigenschaften, die die reinen μ-Opioide nicht besitzen und zur Therapieverbesserung führen. Im Entzugsspringen an der Maus wurde gezeigt, dass bei Tieren, die mit kombinierten ORL1/μ-Agonisten wie A9, A6 A4 oder A7 behandelt wurden, durch Naloxon kein oder ein nur minimales Entzugsvefialten ausgelöst wird (s. Abbildungen 15c-e). A1 hingegen zeigt im Entzugsspringen deutliche Entzugssymptomatik (Abbildung 15b). Im Spontanentzug an der Ratte, wobei das Gewicht der Ratte nach Absetzen der Testsubstanz Ober mehrere Tage hinweg dokumentiert wird, ist jedoch noch ein deutlicher Unterschied zwischen Morphin und A1 (ORL1:μ 0,1) zu erkennen (Abbildung 16). Während das Gewicht der Ratten nach Absetzen von Morphin um beinahe 10% abnimmt, nimmt es nach Absetzen von A9 nur um 3% ab. Das Verhältnis ORL1/μ von 0,1 ist auch hier eine Grenze bis zu der die vorteilhafte Wirkung der Verbindungen mit den erfindungsgemäßen Eigenschaften zu beobachten ist Durch diese Eigenschaften sind die Verbindungen mit einem ORL1/μ-Verhältnis von 0,1 bis 30, vorzugsweise von 0,1 bis 20, bei einem $K_i$-Wert am μ-Opioid-Rezeptor von unter 100 nM besonders geeignet für Patientengruppen, die ein erhöhtes Risiko für körpertiche Abhängigkeit besitzen. Zu dieser Gruppe können beispielweise Patienten gehören, die bereits μ-Opioid-Erfahrung haben.

Zur Unterdrückung der körperlichen Abhängigkeit ist es jedoch bevorzugt, dass die ORL1-Komponente etwas erhöht ist, wobei die körperliche Abhängigkeit jedoch bereits bei einem ORL1:μ-Verhältnis von 0,1 reduziert ist. Vorzugsweise beträgt das Verhältnis ORL1/μ einer Verbindung zur Behandlung von Schmerz bei gleichzeitiger Unterdrükkung von Entzugssymtornen mindestens 0,25, besonders bevorzugt mindestens 0,5. Verbindungen mit dieser erhöhten ORL1-Komponente werden bevorzugt bei Patientengruppen angewendet, die ein besonderes Risiko für körperliche

Abhängigkeit besitzen.

Verbindungen, die eine Affinität von mindestens 100 nM zum μ-Opioid-Rezeptor und zum ORL1-Rezeptor aufweisen und aufgrund der ORL1-Komponente eine im Vergleich zu einem μ-Opioid im gleichen Affinitäsbereich geringere Entzugssymptomatik induzieren, sind zur Behandlung von Schmerz geeignet. Der Effekt kann durch die in den Beispielen beschriebene Modelle zum Entzugsspringen und zum Spontanentzug gezeigt werden.

**Reduktion der psychischen Abhängigkeit/Sucht**

Gemischte ORL1/μ-Agonisten induzieren - ähnlich wie reine Agonisten - eine Platzkonditionierung bei Ratten. Während die Schwellendosis zur induktion einer Platzpräferenz bei reinen N-Opioiden (am Beispiel von B1, B3-B6) deutlich unterhalb der analgetisch halbmaximal wirksamen Dosis liegt, liegt sie bei gemischten ORL11/μ-Agonisten (am Beispiel von A4, A7 und A6) Im Bereich bzw. oberhalb der analgetisch halbmaximal wirksamen Dosis (Abbildung 21). Dies bedeutet, dass gemischte ORL1/μ-Agonisten ein gegenüber reinen μ-Oploiden reduziertes Suchtpotential aufweisen.

Trotz des körperlichen und psychischen Abhängigkeitspotentials von μ-Opioiden werden sie erfolgreich seit langer Zeit in der Klinik eingesetzt, wobei die meisten Patienten nach erfolgter Therapie das Arzneimittel wieder absetzen. Bestimmte Patientengruppen sind jedoch anfällig für Suchtverhalten. Es ist daher bevorzugt, die Verbindungen mit den efindungsgemäßen Eigenschaften zur Schmerztherapie bei Patienten mit erhöhtem Suchtpotential zu verwenden.

Zu diesen Patientengruppen gehören beispielsweise Menschen mit psychischen Erkrankungen, insbesondere depressive Menschen oder Menschen, die unter Angststörungen leiden (Paton et al., Journal of Genetic Psychology 1977, 131, 267-289). Vorzugsweise werden die Verbindungen mit den erfindungsgemäßen Eigenschaften daher bei Patienten eingesetzt, die eine psychische Erkrankung aufweisen, zur Verminderung der Gefahr von psychischer Abhängigkeit im Verlauf der Schmerztherapie. Besonders bevorzugt werden die Verbindungen mit den erfindungsgemäßen Eigenschaften zur Schmerztherapie bei Patienten angewendet, die unter Depressionen oder Angststörungen leiden.

Verbindungen, die eine Affinität von mindestens 100 nM zum μ-Opioid-Rezeptor und zum ORL1-Rezeptor aufweisen und aufgrund der ORL1-Komponente eine im Vergleich zu einem μ-Opioid im gleichen Affinitätsbereich geringere psychische Abhängigkeit bewirken, können zur Behandlung von Schmerz verwendet werden. Dieser Effekt kann beispielsweise durch Antagonisierungsexperimente gezeigt werden, aber auch durch Untersuchungen zur Platzpräferenz wie in den Beispielen beschrieben.

**Atemdepression**

Die μ-vermittete Atemdepression ist bei den gemischten ORL1/μ-Agonisten deutlich verringert Gemessen wurde die akute atemdepressorische Wirkung als Anstieg des $pCO_2$ des arteriellen Blutes an der Ratte In einer analgetisch voll wirksamen Dosierung als auch in einer analgetischen Schwellendoslerung.

Bei reinen μ-Opioploden gezeigt an B1 (Fentanyl, Abbildung 17) und B4 (Oxycodon, Abbildung 17a) kommt es zum Zeitpunkt des analgetischen Wirkmaximums aufgrund der μ-induzlerten Atemdepression zu einem deutlichen Anstieg des arteriellen $pCO_2$. Bei einer 90 - 100% wirksamen Dosis steigt der $pCO_2$-Wert um mehr als 50% an.

Im Gegensatz dazu steigt der $pCO_2$-Wert bei gemischten ORL1/μ-Agonisten wie A4, A5, A6 und A9 nur geringfügig an (Abbildungen 17b-e), Selbst bei einer sehr hohen Dosierung, die ober mehrene Stunden analgetisch maximal Wirksam ist, steigt der arterielle $pCO_2$ nur um ca. 20 - 30% an.

Durch Antagoniserungsversuche wurde gezeigt, dass

(1) die Atemdepression nach Antagonisierung der ORL1-Komponente am Beispiel von A4 mit B11 deutlich verstärkt ist (ca 70%), und
(2) die Atemdepression durch anschließende μ-Antagonisierung mit Naloxon komplett unterdrückt wird (Abbildung 18).

Dies zeigt, dass die verringerte Atemdepression bei gemischten ORL1/μ-Agonisten mit den erfindungsgemäßen Eigenschaften auf die ORL1-Komponente zunrückzuführen Ist. Die Atemdepression wird vollständig durch die μ-Komponente ausgelöst. Die Antagonisieerungsexperimente beweisen, dass die Reduktion der Atemdepression durch die ORL1-Komponente bewirkt wild.

Da die Atemdepression ausgelöst von μ-Oploiden insbesondere bei der Narkose zu folgenschweren Komplikationen führen kann, können, die Verbindungen mit den erfindungsgemäßen Eigenschaften zur Narkose oder narkosebegleltend eingesetzt werden. Dabei ist es bevorzugt, wenn dle Halbwertszeit der Verbindung weniger als eine Stunde beträgt, besonders bevorzugt weniger als 30 Minuten.

Dabei versteht man als Halbwertszeit die zeit, in der die Hälfte der aufgenommenen Verbindung mit den erfindungsgemäßen Eigenschaften verstoftwechselt und/oder ausgeschieden ist.

Auch im Anschluss an eine Operation ist das Risiko von Atemdepression erhöht. Durch den Einsatz der Verbindungen mit einem ORL1/μ-verhältnis von 0.1 bis 30, vorzugsweise von 0,1 bis 20, bei einem $K_r$Wert am μ-Opioid-Rezeptor van unter 100 nM können höhere Dosierungen postoperativ eingesetzt werden und dadurch, falls erforderlich, eine stärkere Analgesie erreicht werden als mit reinen μ-Agonisten. Es Ist daher bevorzugt, die Verbindungen mit den erfindungsgemäßen Eigenschaften zur Behandlung von postoperativem Schmerz einzusetzen.

Da das Risiko von Atemdepression bei Menschen ab 60 Jahren im Vergleich zu jüngeren Menschen deutlich erhöht ist, wie durch Studien belegt ist (Cepeda et al., Clinical Pharmacology & Therapeutics 2003, 74, 102-112), werden die Verbindungen mit einem ORL1/μ-Verhältnis von 0,1 bis 30, vorzugsweise von 0,1 bis 20, bei einem $K_i$-Wert am μ-Opioid-Rezeptor von unter 100 nM bevorzugt zur Behandlung von Schmerz bei Patienten über 60 Jahren verwendet. Daher ist es besonders bevorzugt, die Verbindungen mit den erfindungsgemäβen Eigenschaften zur Narkose, narkosebegleitend oder postoperativ bei Patienten über 60 Jahre einzusetzen. Besonders bevorzugt werden die Verbindungen auch an Patienten über 60 Jahre zur Behandlung von neuropathischem Schmerz eingesetzt.

Die Reduktion der Atemdepression durch die ORL1-Komponente lässt sich, wie In den Beispielen gezeigt durch Antagonislerungsexperimente belegen. Gegenstand der Erfindung ist daher auch die Verwendung von Verbindungen, die eine Affinität von mindestens 100 nM zum p-Opioid-Rezeptor und zum ORL1-Rezeptor aufweisen und aufgrund der ORL1-Komponente eine im Vergleich zu einem μ-Opioid im gleichen Affinitätsbereich geringere Atemdepresion aufweisen, zur narkosebegleitenden oder postoperativen Behandlung von Schmerzen.

**Höhere SIcherheltssbstände bei gemischten ORL1/μ-Agonisten** Aufgrund der reduzierten μ-OR-vermittelten Atemdepression einerseits und der erhöhten Wirksamkeit bei neuropathischem Schmerz andererseits zeichnen sich die gemischten ORL1/μ-Agonisten durch deutlich vergrößerte Sicherheitsabstände gegenüber reinen μ-opioiden aus. Die Schwellendosis ($ED_{10}$) für einen Anstieg des arteriellen $pCO_2$ liegt liegt für gemischte ORL1/μ-Agonisten mit den erfindungsgemäßen Eigenschaften gezeigt an den Beispielen A1, A5, A7, A6 und A4 um ca einen Faktor 3 bis 20 oberhalb der im neuropathischen Schmerz halbmaximal wirksamen Dosis ($ED_{50}$) (Abbildung 20). Das bedeutet, dass insbesondere bei chronischen Schmerzzuständen der Abstand zu den möglichen opioiden Nebenwirkungen aufgrund der erhöhten Wirksamkeit der Verbindungen mit den erfindungsgemäßen Eigenschaften einerseits und der antioploiden Komponente andererseits so groß ist, dass die μ-typischen Nebenwirkungen bei gleich bleibender Wirksamkeit im therapeutischen Bereich vergleichsweise reduziert auftreten.

Aufgrund des höheren Abstandes zwischen Wirkung und Nebenwirkung bei den Verbindungen mit den erfindungsgemäßen Eigenschaften sind die Verbindungen besonders geeignet zur Behandlung von Schmerz bei Pelliativpatienten. Palliativpatienten sind aufgrund ihres multi-morbiden Zustandes besonders stark von opioidtypischen Nebenwirkungen betroffen. Gegenstand der Erfindung ist daher auch die Verwendung von Verbindungen mit einem ORL1/μ-Verhältnis von 0,1 bis 30, vorzugsweise von 0,1 bis 20, bei einem $K_i$-Wert am μ-Opioid-Rezeptor von unter 100 nM bei der Schmerzbehandlung von Palliativpatienten.

Verbindungen, die eine Affinität zum μ-Opioid-Rezeptor von mindestens 100 nM (Ki-Wert human) und eine Affinität zum ORL-1-Rezeptor aufweisen, wobei das Verhältnis zwischen den Affinitäten ORL1:μ (Ki-Werte) zwischen 0,1 und 30, vorzugsweise von 1:10 bis 20:1, liegt, weisen daher zusammenfassend insbesondere folgende Vorteile gegenüber der Standardtherapie mit μ-Opioiden auf:

- Wirkverstärkung im chronischen Schmerz, insbesondere Im neuropathischen Schmerz und im Entzündungsschmerz
- Deutlich reduzierte Nebenwirkungen gezeigt am Beispiel der Atemdepression, Entzug/Sucht und opioid-induzierter Hyperalgesie bei vergleichbarer Wirksamkeit im Akutschmerz

Die Verbindungen, die eine Affinität zum μ-Opioid-Rezeptor von mindestens 100 nM ($K_i$-Wert human) und eine Affinität zum ORL-1-Rezeptor aufweisen, wobei das Verhältnis zwischen den Affinitäten ORL1:μ ($K_i$-Werte) zwischen 0,1 und 30, vorzugsweise von 0,1 bis 20, liegt, besitzen die vorgenannten Eigenschaften. Die beobachteten Vorteile beruhen nicht auf Eigenschaften, die speziell die untersuchten Verbindungen besitzen, sondern es handelt sich um Effekte, die aus dem Wirkmechanismus resultieren. Dies konnte durch Antagonisierungsexperimente belegt werden. Dabei wurde gezeigt, dass die ORL1-Komponente einen Beitrag zur Analgesie leistet, die μ-typischen Nebenwirkungen jedoch unterdrückt, Im Bereich der Analgesie verhält sich die ORL1-Komponente synergistisch, Im Bereich der untersuchten Nebenwirkungen jedoch entgegengesetzt. Entscheidend dafür ist das Verhältnis der beiden Komponenten.

Die Werte, die den erfindungsgemäßen Bereich definieren, beziehen sich auf in vitro-Daten; in Fällen, bei denen in vivo ein oder mehrere aktive Metaboliten gebildet werden, können die Metabolite die Aktivität beeinflussen. Wenn Metabolite gebildet werden, können folgende Fälle unterschieden werden:

a) Einsatz von Prodrugs

Verbindungen, die nicht das erfindungsgemäße Bindungsprofil aufweisen, können Metaboliten bilden. die eine Affinität zum μ-Opioid-Rezeptor von mindestens 100 nM (Ki-Wert human) und eine Affinität zum ORL-1-Rezeptor aufweisen, wobei das Verhältnis zwischen den Affinitäten ORL1/μ definiert als $1/[K_{(ORL1)}/K_{(\mu)}]$ zwischen 0,1 und 30, vorzugsweise von 0,1 bis 20 liegt, und daher doch die erfindungsgemäßen Eigenschaften aufweisen. Dies kann durch die Bestimmung der $K_i$-Werte der Metaboliten festgestellt werden. Daher können auch Verbindungen verwendet werden, die Metaboliten bilden, die eine Affinität zum μ-Opioid-Rezeptor von mindestens 100 nM ($K_i$-Wert human) und eine Affinität zum ORL-1-Rezeptor aufweisen, wobei das Verhältnis zwischen den Affinitäten ORL1/μ definiert als $1/[K_{i(ORL1)}/K_{i(\mu)}]$ zwischen 0,1 und 30, vorzugsweise von 0,1 bis 20 liegt, wobei der Beitrag zur Wirksamkeit und/oder zur Reduktion der μ-typischen Nebenwirkungen durch Antagonisierungsexperimente nachweisbar ist.

b) Bildung von Metaboliten, die gemeinsam oder zusammen mit der Muttersubstanz das erfindungsgemäße Profil bilden

Wird beispielsweise ein selektiver μ-Agonist teilweise zu einem selektiven ORL1-Agonisten metabolisiert und weist das entstehende Gemisch die erfindungsgemäßen Eigenschaften auf, d. h., das Verhältnis von ORL1/μ definiert als $1/[K_{i(ORL1)}/K_{i(\mu)}]$ liegt zwischen 0,1 und 30 und der Ki-Wert am humanen μ-Opioid-Rezeptor beträgt mindestens 100 nM, kann das Gemisch ebenfalls verwendet werden. Diese Gemische können auch aus Verbindungen entstehen, die keine Selektivität aufweisen, aber trotzdem außerhalb des erfindungsgemäßen Bereiches liegen. Die erfindungsgemäßen Eigenschaften können einerseits dadurch nachgewiesen werden, dass die Bindungskonstanten des Gemisches bestimmt werden, das in-vivo entsteht, wobei die Konzentrationen durch HPLC-MS-Untersuchungen bestimmt werden können, und andererseits dadurch, dass der Beitrag der ORL1-Komponente zur Wirkverstärkung im chronischen Schmerz und/oder zur Verringerung der μ-typischen Nebenwirkungen durch Antagonisierungsexperimente mit einem ORL1-Antagonisten gezeigt wird. Weiterhin besitzen die Verbindungen die Eigenschaft, im Akutschmerz wirksam zu sein. Durch Metabolismus gebildete Substanzgemische, die die erfindungsgemäßen Eigenschaften aufweisen, wobei die Bindungskonstanten des Gemisches dem erfindungsgemäßen Bereichs entsprechen und der Beitrag zur Wirksamkeit und/oder zur Reduktion der μ-typischen Nebenwirkungen durch Antagonisierungsexperimente nachweisbar ist, können auch verwendet werden.

Die Wirkungen, die die erfindungsgemäßen Verbindungen ausüben, können auch durch Verabreichung von zwei oder mehr verschiedenen Substanzen erreicht werden. Dies kann einerseits dadurch nachgewiesen werden, dass die Bindungskonstanten des Gemisches bestimmt werden, und andererseits dadurch, dass der Beitrag der ORL1-Komponente zur Wirkverstärkung im chronischen Schmerz und/oder zur Verringerung der μ-typischen Nebenwirkungen durch Antagonisierungsexperimente mit einem ORL1-Antagonisten gezeigt wird. Weiterhin besitzen die Verbindungen die Eigenschaft, im Akutschmerz wirksam zu sein.

Die erfindungsgemäßen Arzneimittel enthalten neben mindestens einer Verbindung mit den erfindungsgemäßen Eigenschaften gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosalen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder In einem Pflaster, gegebenenfalls unter Zusatz von die Hautpenetration fördernden Mitteln, sind geeignete perkutane Applikationszubereitungen. Oral oder perkutan anwendbare Zubereigtungsformen können die Verbindungen mit den erfindungsgemäßen Eigenschaften oder eine erfindungsgemäße Kombination verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 20 mg/kg, bevorzugt 0,05 bis 5 mg/kg wenigstens einer Verbindung oder Kombination mit den erfindungsgemäßen Eigenschaften appliziert.

Die Verbindungen A1 bis A10, die alle die erfindungsgemäßen Eigenschaften aufweisen, fallen unter die Gruppe der spirocyclischen Cyclohexan-Derivate. Diese Verbindungen besitzen eine Affinität zum μ-Opioid-Rezeptor und/oder zum ORL-1-Rezeptor, eine Untergruppe dieser Verbindungen weist jedoch die erfindungsgemäßen Eigenschaften auf.

Verbindungen aus der Gruppe der spirocyclischen Cyclohexan-Derivate der allgemeinen Formel I

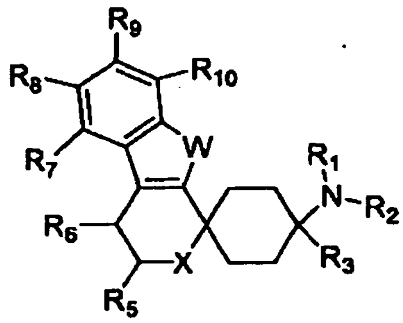

I

, worin
$R^1$ und $R^2$ unabhängig voneinander für H oder $CH_3$ stehen, wobei $R^1$ und $R^2$ nicht gleichzeitig H bedeuten;
$R^3$ für Phenyl, Benzyl oder Heteroaryl jeweils unsubstituiert oder einfach oder mehrfach mit F, Cl, OH, CN oder $OCH_9$ substituiert, steht;
W für $NR^4$, O oder S steht und
$R^4$ für H; $C_{1-5}$-Alkyl, Phenyl; Ober eine $C_{1-3}$-Alkyl-Gruppe gebundenes Phenyl, $COR^{12}$; $SO_2R^{12}$ steht, wobei $R^{12}$ H; $C_{1-7}$-Alkyl, verzweigt oder unverzweigt, gesättigt oder ungesättigt, unsubstituiert oder einfach oder mehrfach mit OH, F oder $COOC_{1-4}$-Alkyl substituiert; $C_{4-6}$-Cycloalkyl; Aryl-, oder Heteroaryl, unsubstituiert oder einfach oder mehrfach mit F, Cl, Br, $CF_3$, $OCH_3$, $C_{1-4}$-Alkyl, verzweigt oder unverzweigt, unsubstituiert oder substituiert mit F, Cl, CN, $CF_3$, $OCH_3$ oder OH; oder über gesättigtes oder ungesättigtes $C_{1-3}$-Alkyl gebundenes Phenyl oder Heteroaryl, unsubstituiert oder einfach oder mehrfach mit F, Cl, Br, $CF_3$, $OCH_3$, $C_{1-4}$-Alkyl, verzweigt oder unverzweigt, unsubstituiert oder substituiert mit F, Cl, CN, $CF_3$, $OCH_3$ oder OH substituiert; oder Ober gesättigtes oder ungesättigtes $C_{1-3}$-Alkyl gebundenes $C_{5-6}$-Cycloalkyl; $OR^{13}$; $NR^{14}R^{15}$ bedeutet;
$R^5$ für H; $COOR^{13}$, $CONR^{13}$, $OR^{13}$; $C_{1-5}$-Alkyl, gesättigt oder ungesättigt, verzweigt oder unverzweigt, unsubstituiert oder einfach oder mehrfach mit OH, F, $CF_3$ oder CN substituiert, steht;
$R^6$ für H steht;
oder $R^5$ und $R^6$ gemeinsam $(CH_2)_n$ mit n = 2, 3, 4, 5 oder 6 bedeuten, wobei einzelne Wasserstoffatome auch durch F, Cl, $NO_2$, $CF_3$, $OR^{13}$, CN oder $C_{1-5}$-Alkyl ersetzt sein können;
$R^7$, $R^8$, $R^9$ und $R^{10}$ unabhängig voneinander für H, F, Cl, Br, $NO_{2-}$ $CF_3$, OH, OCH3, CN, $COOR^{13}$, $NR^{14}R^{15}$; $C_{1-5}$-Alkyl, Heteroaryl, unsubstituiert oder einfach oder mehrfach mit Benzyl, $CH_3$, Cl, F, $OCH_3$ oder OH substituiert, stehen;
wobei $R^{13}$ H oder $C_{1-5}$-Alkyl bedeutet;
$R^{14}$ und $R^{15}$ unabhängig voneinander H oder $C_{1-5}$-Alkyl bedeuten;
X für O, S, SO, $SO_2$ oder $NR^{17}$ steht;
$R^{17}$ für H; $C_{1-5}$-Alkyl, gesättigt oder ungesättigt, verzweigt oder unverzweigt; $COR^{12}$ oder $SO_2R^{12}$ steht,
in Form ihrer reinen Diastereomeren, ihrer Racemate, ihrer reinen Enantiomeren, oder in Form von Mischungen der Stereoisomeren in einem beliebigen Mischungsverhältnis;
als Basen oder in Form ihrer Salze, Insbesondere der physiologisch verträglichen Salze oder Salze physiologisch verträglicher Säuren oder Kationen;
die eine Affinität zum $\mu$-Opioid-Rezeptor von mindestens 100 nM ($K_i$-Wert human) und eine Affinität zum ORL-1-Rezeptor aufweisen, wobei das Verhältnis zwischen den Affinitäten ORL1/$\mu$ definiert als $1/[K_{i(ORL1)}/K_{i(\mu)}]$ von 0,1 bis 30 liegt, sind auch zur Herstellung eines Arzneimittels zur Behandlung von diabetischem Polyneuropathieschmerz, postoperativem Schmerz oder Schmerzen bei postzosterischer Neuralgie geeignet.

**Beispiele**

**Verwendete Abkürzungen**

| | |
|---|---|
| AUC | Fläche unter der Kurve |
| CFA | Complete Freund's Adjuvant |
| DBTC | Dibutylzinndichlorid |
| MPE | Maximal möglicher Effekt |

Die Erfindung wird anhand der folgenden Beispiele verdeutlicht. Es wurden typische Vertreter stellvertretend für μ-Agonisten, gemischte μ/ORL1-Agonisten, ORL1-Agonisten sowie ein ORL1 Antagonist verwendet. Als μ-Antagonist wurde die klinisch verwendete Verbindung Naloxon eingesetzt. Diese beispielhaften Verbindungen wurden einer Vielzahl von Untersuchungen unterzogen, die die Ausnahmestellung der Verbindungen mit den erfindungsgemäßen Eigenschaften belegen.

| **Bezeichnung** | **Struktur** | **Quelle oder Herstellverfahren** |
|---|---|---|
| B1 (Fentanyl) | 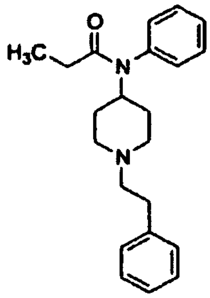 | Kommerziell erhältlich |
| B2 (Sufentanil) | 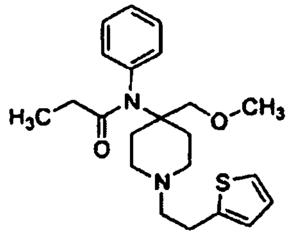 | Kommerziell erhältlich |
| B3 (Morphin) | 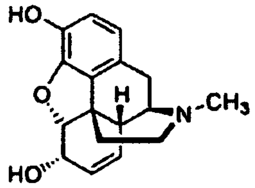 | Kommerziell erhältlich |
| B4 (Oxycodon) | 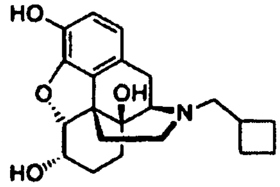 | Kommerziell erhältlich |
| B5 (Buprenorphin) | 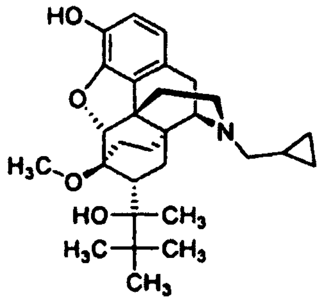 | Kommerziell erhältlich |
| B6 (Hydromorphon) | 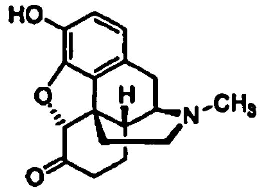 | Kommerziell erhältlich |

(fortgesetzt)

| Bezeichnung | Struktur | Quelle oder Herstellverfahren |
|---|---|---|
| B7 (L-Methadon) | 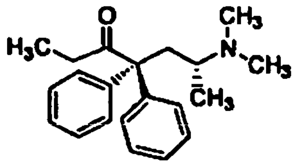 | Kommerziell erhältlich |
| B8 | 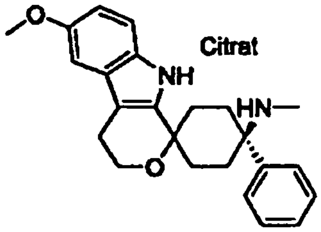 | Synthese analog zu A1 |
| A1 | 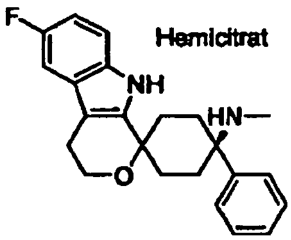 | Beispiel 49, EP1560835 1,1-(3-Methylamino-3-phenylpentamethylen)-6-fluor- 1,3,4,9-tetrahydropyrano[3,4- b]indol Hemicitrat |
| A2 | 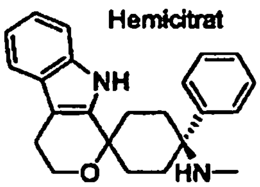 | Beispiel 28, EP1560835 1,1-(3-Methylamino-3-phenylpentamethylen)- 1,3.4,9-tettahydropyrano [3,4- b]indol Hemicitrat |
| A3 | 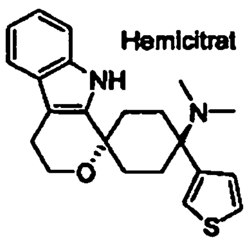 | Beispiel 8, WO200566183 1,1-[3-Dimethylamino-3-(3- thlenyl)pentamethylen]- 1,3,4,9-tetrahydro-pyrano[3,4-b]indol Hemicitrat |
| A4 | 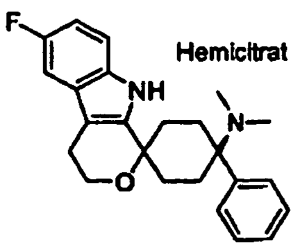 | Beispiel 24, EP1560835 1,1-(3-Dimethylamino-3-phenylpentamethylen)-6-fluor- 1,3,4,9-tetrahydropyrano[3,4- b]indol Hemicitrat, |
| A5 | 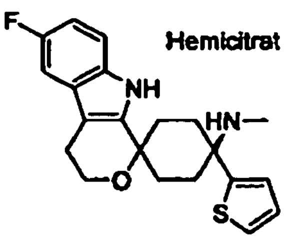 | Beispiel 15, WO200566183 1,1-[3-Methylamino-3-(2-thienyl)pentamethylen]- 1,3,4,8-tetrahydro-pyrano [3,4- b]-6-fluorindol Citrat |

(fortgesetzt)

| Bezeichnung | Struktur | Quelle oder Herstellverfahren |
|---|---|---|
| A6 | 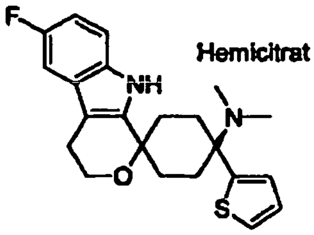 Hemicitrat | Beispiel 10, WO200566183 1,1-[3-Dimethytamino-3-(2- thienyl)pentamethylen]- 1,3,4,9-tetra-hydro-pyrano[3,4-b]-6-fluorindol Hemicitral |
| A7 | 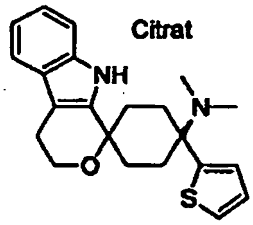 Citrat | Beispiel 7, WO200566183 1,1-[3-Dimethylamino-3-(2- thienyl)pentamethylen]- 1,3,4,9-tetra-hydro-pyrano[3,4-b]indol Citrat |
| A8 | 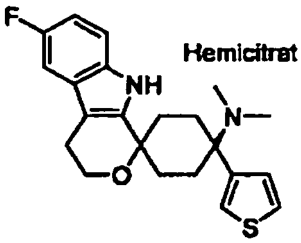 Hemicitrat | Beispiel 13, WO200566183 1,1-[3-Dimethylamino-3-(3- thienyl)pentamethylen]- 1.3,4,9-tetra-hydro-pyrano[3,4-b]-6-fluorindol Hemicitrat |
| A9 | 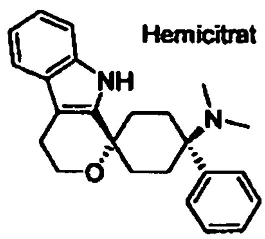 Hemicitrat | Beispiel 3, EP1560835 1,1-(3-Dimethylamino-3-phenylpentamethylen)- 1,3,4,9-tetrahydropyrano [3,4- b]indol Hemicitrat |
| A10 | 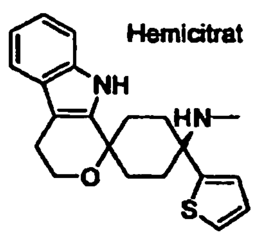 Hemicitrat | Beispiel 14, WO200566183 1,1-[3-Methylamino-3-(2-thienyl)pentamethylen]- 1,3,4,9-tetrahydro-pyrano [3.4- b]indol Citrat |
| A11 | 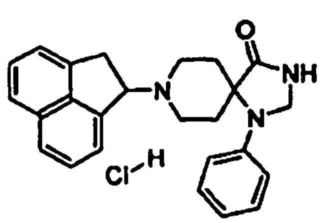 | EP 08856514 |

(fortgesetzt)

| Bezeichnung | Struktur | Quelle oder Herstellverfahren |
|---|---|---|
| 89 | 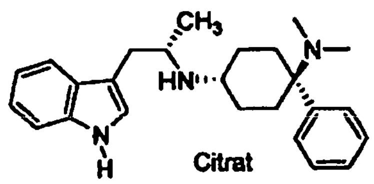 | Beispiel 59, EP1392641 + Enantiomerentrennung |
| B10 (Nociceptin) | Peptid, endogener Ligand | Kommerziell erhältlich |
| B11 | 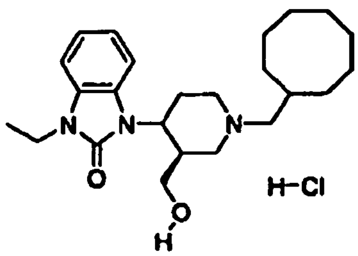 | ORL1-Antagonist WO 9854168 |

**Messung der ORL1-Bindung**

Die Cyclohexan-Derivate der allgemeinen Formel wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H.Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM $MgCl_2$ und 1 mM EDTA durchgeführt Die Bindung an den ORL1-Rezeptor wurde unter Verwendung von je 1 mg WGA-SPA Beads (AmershamPharmacia, Freiburg), durch einstündige Inkubation des Ansatzes bei RT und anschliessende Messung im Szintillationscounter Trilux (Wallac, Finnland), bestimmt Die Affinität wird in Tabelle 1 als nanomolarer $K_i$-Wert in oder % Inhibition bei c=1 µM angegeben.

**Messung der µ-Bindung**

Die Rezeptoraffinität zum humanen µ-Opiatrezeptor wurde in einem homogenen Ansatz in Mikrotiterplatten bestimmt Hierzu wurden Verdünnungsreihen der jeweils zu prüfenden Substanzen mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

Die $K_i$-Werte der Beispielverbindungen sind in der folgenden Tabelle zusammengefasst.

| Substanz | $K_i$ (ORL1) | $K_i$ (µ-OR) | Ratio ORL1: µ[1] | Klassifizierung |
|---|---|---|---|---|
| B1 (Fentanyl) | 1600 nM | 7,9 nM | - | µ-Agonist |
| B2 (Sufentanil) | 145 nM | 0,8 nM | - | µ-Agonist |

(fortgesetzt)

| Substanz | $K_i$ (ORL1) | $K_i$ (μ-OR) | Ratio ORL1: μ[1] | Klassifizierung |
|---|---|---|---|---|
| B3 (Morphin) | > 1 μM | 9 nM | - | μ-Agonist |
| B4 (Oxycodon) | > 10 μM | 130 nM | - | μ-Agonist |
| B5 (Buprenorphin) | 36 nM | 0.3 nM | - | Opiold-Agonist (schwache ORL1-Komponente) |
| B6 (Hydromorphon) | > 10 μM | 4 nM | - | μ-Agonist |
| B7 (L-Methadon) | > 1 μM | 7 nM | - | μ-Agonist |
| B8 | 70 nM | 2,4 nM | 0,03 (1 : 30) | μ-Agonist, schwacher ORL1-Agonist |
| A1 | 14 nM | 1,8 nM | 0,1 (1 : 10) | gem. ORL1/μ-Agonist |
| A2 | 1,7 nM | 0,4 nM | 0.25 (1:4) | gem. ORL1/μ-Agonist |
| A3 | 0.3 nM | 0,1 nM | 0,3 (1:3) | gem. ORL1/μ-Agonist |
| A4 | 2 nM | 1 nM | 0,5 (1 : 2) | gem. ORL1/μ-Agonist |
| A5 | 2 nM | 1 nM | 0,5 (1 : 2) | gem. ORL1/μ-Agonist |
| A6 | 1 nM | 1 nM | 1 (1 : 1) | gem. ORL1/μ-Agonist |
| A7 | 0,4 nM | 0,3 nM | 1 (1 : 1) | gem. ORL1/μ-Agonist |
| A8 | 0,5 | 1,3 nM | 2 (2 : 1) | gem. ORL1/μ-Agonist |
| A9 | 0.5 nM | 1 nM | 2 (2 : 1) | gem. ORL1/μ-Agonist |
| A10 | 0,2 nM | 0,5 nM | 2 (2 : 1) | gem. ORL1/μ-Agonist |
| A11 | 1 nM | 23 nM | 20(20:1) | ORL1/μ-Agonist vergleichsweise schwache μ-Komponente |
| B9 | 0,4 nM | 55 nM | 140(140:1) | ORL1-Agonist; vergleichsweise schwache μ-Komponente |
| B10 (Nociceptin) | 0,3 nM | ~250 nM | 800 : 1 | ORL1-Agonist; endogener Ligand |
| [1] Definition: $1/[K_{i(ORL1)}/K_{i(\mu)}]$ | | | | |

**Vergleich der analgetischen Wirksamkeit (als ED50, %MPE) Im Akutschmerzmodell (Tail-flick, Ratte/Maus) und in**

**Neuropathleschmerzmodellen (Chung, Ratte; Bennett, Ratte/Maus):**

**<u>Analgesieprüfung im Tail-Flick-Test an der Maus</u>**

Die analgetische Wirksamkeit der Testverbindung wurde im Brennstrahl (Tail-flick)-Test an der Maus nach der Methode von D'Amour and Smith (J. Pharm. Exp. Ther. 72 74 79 (1941) untersucht Dazu wurden NMRI-Mäuse mit einem Gewicht zwischen 20 - 24 g verwendet. Die Tiere wurden einzeln in spezielle Testkäfige gesetzt und die Schwanzbasis einem focussierten Wärmestrahl einer elektrischen Lampe (Tail-flick Typ 55/12/10.fl, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittelwert berechnet Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel:

$$[(T_1 - T_0)/(T_2 - T_0)] \times 100$$

Dabei ist die $T_0$ die Latenzzeit vor und $T_1$ die Latenzzeit nach Substanzapplikatlon, $T_2$ ist die maximale Expo-

sitionszeit (12 sec).

Zur Bestimmung der Dosisabhängigkeit wurde die Testverbindung in 3-5 logarithmisch ansteigenden Dosen, die jeweils die Schwellen- und die maximale Wirkdosis einschlossen, appliziert und die $ED_{50}$-Werte mit Hilfe der Regressionsanalyse bestimmt. Die $ED_{50}$-Berechnung erfolgte im Wirkmaximum 20 Minuten nach intravenöser Substanzgabe.

**Analgesieprüfung im Tail-Flick-Test an der Ratte**

Die analgetische Wirksamkeit der Testverbindungen wurde im Brennstrahl (Tail-flick)-Test an der Ratte nach der Methode von D'Amour and Smith (J. Pharm. Exp. Ther. 72, 74 79 (1941) untersucht. Dazu wurden weibliche Sprague Dawley mit einem Gewicht zwischen 134 und 189 g verwendet. Die Tiere wurden einzeln in spezielle Testkäfige gesetzt und die Schwanzbasis einem focussierten Wärmestrahl einer Lampe (Tail-flick Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel:

$$[(T_1 - T_0)/(T_2 - T_0)] \times 100$$

Dabei ist die $T_0$ die Latenzzeit vor und $T_1$ die Latenzzeit nach Substanzapplikation, $T_2$ ist die maximale Expositionszeit (12 sec).

Zur Bestimmung der Dosisabhängigkeit wurde die jeweilige Testverbindung in 3 - 5 logarithmisch ansteigenden Dosen, die jeweils die Schwellen- und die maximale Wirkdosis einschlossen, appliziert und die $ED_{50}$-Werte mit Hilfe der Regressionsanalyse bestimmt. Die $ED_{50}$-Berechnung erfolgte im Wirkmaximum, 20 Minuten nach intravenöser Substanzgabe.

**Tail-fllck mit verringerter Brennstrahlintensität an der Ratte**

Die modulatorische Wirksamkeit der Testsubstanzen auf akute, noxische thermische Reize wurde im Brennstrahl (Tail-flick)-Test an der Ratte nach der Methode von D'Amour und Smith (J. Pharm. Exp. Ther. 72, 74 79 (1941)) untersucht Dazu wurden männliche Sprague-Dawley Ratten (Züchter Janvier, Le Genest St. Isle, Frankreich) mit einem Gewicht zwischen 200 - 250 g verwendet. Die Tiere wurden einzeln In spezielle Testkompartimente verbracht und die Schwanzbasis einem focussierten Brennstrahl eines Analgesiemeters (Modell 2011, Rhema Labortechnik, Hofheim, Deutschland) ausgesetzt. Die Gruppengröße betrug 10 Tiere. Die Brennstrahlintensität wurde so eingestellt, dass die Zeit vom Einschalten des Brennstrahls bis zum plötzlichen Wegziehen des Schwanzes (Wegziehlatenz) bei unbehandelten Tieren ca. 12-13 Sekunden betrug. Vor Gabe einer erfindungsgemäßen Substanz wurde im Abstand von fünf Minuten zweimal die Wegziehlatenz bestimmt und der Mittelwert als Kontroll-Latenzzeit definiert Die Messung der Wegziehlatenz des Schwanzes wurde erstmal 10 Minuten nach intravenöser Substanzgabe durchgeführt. Nach Abklingen des antinociceptiven Effekts (nach 2-4 Stunden) erfolgten die Messungen im Abstand von 30 Minuten bis maximal 6.5 Stunden nach Substanzapplikation. Die anti- oder pronociceptive Wirkung wurde als Zu- bzw. Abnahme der Wegziehlatenzzeit nach folgender Formel bestimmt:

$$(\% \text{ MPE}) = [(T_1 - T_0)/(T_2 - T_0)] \times 100$$

Dabei sind: $T_0$: Kontroll-Latenzzeit vor Substanzapplikation, $T_1$: Latenzzeit nach Substanzapplikation, $T_2$: maximale Expositionszeit des Brennstrahls (30 Sekunden), MPE: maximal möglicher Effekt.

Mittels Varianzanalyse (repeated measures ANOVA) wurde auf statistisch signifikante Unterschiede zwischen Substanz- und Vehikelgruppe getestet. Das Signifikanzniveau wurde auf $\leq 0.05$ gesetzt.

**Chung-Modell: Mononeuropathieschmerz nach spinaler Nervenligatur**

Tiere: Männliche Sprague Dawley Ratten (140-160g), von einem kommerziellen Züchter (Janvier, Genest St. Isle, Frankreich), wurden unter einem 12:12h Licht-Dunkel Rhythmus gehalten. Die Tiere wurden mit Futter und Leitungswasser ad libitum gehalten. Zwischen der Lieferung der Tiere und der Operation wurde eine Pause von einer Woche eingehalten. Die Tiere wurden nach Operation Ober einen Zeitraum von 4-5 Wochen mehrfach getestet, wobei eine Auswaschzeit von mindestens einer Woche eingehalten wurde.

Modellbeschreibung: Unter Pentobarbital Narkose (Narcoren ®, 60 mg/kg i.p., Merial GmbH, Hallbergmoos, Deutschland), wurden die linken L5, L6 Spinalnerven exponiert, Indem ein Stück des paravertebralen Muskels und ein Teil des linken spinalen Prozesses des L5 lumbalen Wirbelkörpers entfernt wurden. Die Spinalnerven L5 und L6 wurden vorsichtig isoliert und mit einer festen Ligatur abgebunden (NC-silk black,USP 5/0, metric 1, Braun Melsungen AG, Melsungen, Deutschtand) (Kim and Chung 1992). Nach Ligatur wurden Muskel und benachbarte Gewebe zugenäht und die Wunde mittels Metallklammern geschlossen.

Nach einer Woche Erholungszeit wurden die Tiere zur Messung der mechanischen Allodynie in Käfige mit einem Drahtboden gesetzt. An ipsi- und/oder kontralateraler Hinterpfote wurde die Wegziehschwelle mittels eines elektronischen von Frey Filamentes (Somedic AB, Malmö, Schweden) bestimmt. Der Median von fünf Stimulierungen ergab einen Messzeitpunkt. Die Tiere wurden 30 min vor und zu verschiedenen Zeiten nach Applikation von Testsubstanz- oder Vehikellösung getestet. Die Daten wurden als % maximal mögliche Wirkung (%MPE) aus den Vortesten der Einzeltiere (=0%MPE) und den Testwerten einer unabhängigen Sham-Kontrollgruppe (=100%MPE) bestimmt Alternativ wurden die Wegziehschwellen in Gramm dargestellt.

Statistische Auswertung: $ED_{50}$ Werte und 95% Vertrauensberelche wurden über semilogarithmische Regressionsanalyse zum Zeitpunkt der maximalen Wirkung bestimmt. Die Daten wurden über eine Varianzanalyse mit wiederholten Messungen sowie einer post hoc Analyse nach Bonferroni analysiert. Die Gruppengröße betrug üblicherweise n=10.

Referenzen: Kim,S.H. and Chung,J.M., An experimental model for peripheral neuropathy produced by segmental spinal nerve ligation in the rat, Pain, 50 (1992) 355-363.

**Bennett Modell: Neuropathischer Schmerz an der Maus bzw. an der Ratte**

Die Untersuchung auf Wirksamkeit im neuropathischen Schmerz wurde im Bennett-Modell (chronic constriction Injury; Bennett und Xie, 1988, Pain 33: 87-107) untersucht

Sprague-Dawley Ratten mit einem Gewicht von 140-160g werden unter Narcoren-Narkose mit vier losen Ligaturen des rechten nervus ischiaticus versehen. NMRI Mäuse mit einem Gewicht von 16-18g werden unter Ketavet-Rompun-Narkose mit drei losen Ligaturen des rechten nervus ischiaticus versehen. Die Tiere entwickeln an der vom geschädigten Nerv innervierten Pfote eine Überempfindlichkeit, die nach einer Erholungsphase von einer Woche über etwa vier Wochen mittels einer 4°C kalten Metallplatte quantifiziert wird (Kälte-Allodynie). Die Tiere werden für einen Zeitraum von 2 min. auf dieser Platte beobachtet und die Anzahl der Wegziehreaktionen der geschädigten Pfote wird gemessen. Bezogen auf den Vorwert vor Substanzapplikation wird die Substanzwirkung über einen Zeitraum von einer Stunde an vier Zeitpunkten (z.B. 15, 30, 45, 60 min. nach Applikation) bestimmt und die resultierend Fläche unter der Kurve (AUC) sowie die Hemmung der Kälte. Allodynie zu den einzelnen Meßpunkten in Prozent Wirkung zur Vehikelkontrolle (AUC) bzw. zum Ausgangswert (Einzelmeßpunkte) ausgedrückt. Die Gruppengröße beträgt n=10, die Signifikanz einer anti-allodynischen Wirkung (*=p<0.05) wird anhand einer Varianzanalyse mit wiederholter Messung und einer post hoc Analyse nach Bonferroni bestimmt.

**Vincristin-induzierte Polyneuropathie**

Das Modell ist in der Literatur beschrieben (KO Aley, DB Reichling, JD Levine, Neuroscience 1996, 73, 259-265).

**Diabetischer Polyneuropathieschmerz**

Das Modell ist In der Literatur beschrieben (SC Ahlgren, JD Levine, Neuroscience 1993, 52, 1049-1055).

Relative Wirkverstärkung in Neuropathieschmerz-Modellen

| Substanz | Ratio ORL1/μ | $ED_{50}$ akut | $ED_{50}$ chron. | Applikations route | Faktor Wirkverstärkung |
|---|---|---|---|---|---|
| B3 (Morphin) | < 1 : 100 | 1.1 mg/kg[1] | 3.7 mg/kg[4] | i.v. | 0.3 x |
| B3 (Morphin) | < 1 : 100 | 1.1 mg/kg[1] | 1.3 mg/kg[5] | i.v. | 0.8 x |
| B3 (Morphin) | < 1 : 100 | 2 μg/Tier[3] | ~10 μg/Tier[4] | i.th. | 0.5 x |
| B4 (Oxycodon) | < 1 : 100 | 360 μg/kg[1] | 2170 μg/kg[4] | i.v. | 0.2 x |

(fortgesetzt)

| Substanz | Ratio ORL1/μ | $ED_{50}$ akut | $ED_{50}$ chron. | Applikations route | Faktor Wirkverstärkung |
|---|---|---|---|---|---|
| 84 (Oxycodon) | < 1 : 100 | 360 μg/kg[1] | 900 μg/kg[5] | i.v. | 0.4 x |
| B4 (Oxycodon) | < 1 : 100 | 670 μg/kg[1] | 2520 μg/kg[4] | i.p. | 0.3 x |
| 84 (Oxycodon) | < 1 : 100 | 670 μg/kg | 1290 μg/kg[5] | i.p. | 0.5 x |
| B6 (Hydromorphon) | < 1 : 100 | 150 μg/kg[1] | 220 μg/kg[4] | i.v. | 0.7 x |
| B7 (L-Methadon) | < 1 : 100 | 210 μg/kg[1] | 490 μg/kg[4] | i.v. | 0.4 x |
| 85 (Buprenorphin) | < 1 : 100 | 17 μg/kg[1] | 55 μg/kg[4] | i.v. | 0.3 x |
| B1 (Fentanyl) | < 1 : 100 | 10 μg/kg[1] | 11 μg/kg[4] | i.v. | 0.9 x |
| B1 (Fentanyl) | < 1 : 100 | 43 μg/kg[1] | 230 μg/kg[4] | i.p. | 0.2 x |
| | | | | | |
| B8 | 1 : 30 | 330 μg/kg[1] | 363 μg/kg[4] | i.v. | 0.9 x |
| | | | | | |
| A1 | 1 : 10 | 110 μg/kg[1] | 9 μg/kg[4] | i.v. | 12 x |
| A1 | 1 : 10 | 110 μg/kg[1] | 11 μg/kg[5] | i.v. | 10 x |
| A4 | 1 : 2 | 7 μg/kg[1] | 1 μg/kg[4] | i.v. | 7 x |
| A4 | 1 : 2 | 7 μg/kg[1] | 1 μg/kg[5] | i.v. | 7 x |
| A5 | 1 : 2 | 71 μg/kg[1] | 8 μg/kg[4] | i.v. | 8 x |
| A5 | 1 : 2 | 71 μg/kg[1] | 10 μg/kg[5] | i.v. | 7 x |
| A6 | 1 : 1 | 9 μg/kg[1] | 4 μg/kg[4] | i.v. | 2.5 x |
| A6 | 1 : 1 | 9 μg/kg[1] | 1 μg/kg[5] | i.v. | 9 x |
| A7 | 1 : 1 | 2 μg/kg[1] | 1 μg/kg[4] | i.v. | 2 x |
| A9 | 2 : 1 | 2 μg/kg[1] | 0.8 μg/kg[4] | i.v. | 2.5 x |
| | | | | | |
| A11 | 20 : 1 | >10 μg/Tier 2 | 0.2 μg/Tier[8] | i.th. | > 50 x |
| A11 | | 420 μg/kg[2] | keine Daten | i. v. | |

[1]) Tail-flick Modell, Ratte
[2]) Tail-flick Modell, Maus
[3]) Soft Tail-flick Modell, Ratte
[4]) Chung-Modell, Ratte
[5]) Bennett-Modell, Ratte
[8]) Bennett-Modlell, Maus

Zur graphischen Darstellung wurden die $ED_{50}$-Wert aus dem Tail-flick Test und aus den Neuropathieschmerz-Modellen auf die $ED_{50}$-Wert im Tail-flick Test normiert, um die Relation der jeweiligen halbmaximal wirksamen Dosierungen darzustellen (s. Abbildungen 1 und 2).

Antagonisierung der μ- und der ORL1-Komponente im Chung-Modell

In Antagonisierungsexperimenten wurde jeweils ein partieller Antagonismus mit Naloxon (μ-OR) und B11 (ORL1-R) gezeigt werden. Die Daten beweisen, dass beide Komponenten zur Analgesie beitragen (s. Abbildung 3).

Auch bei einer hohen sehr hohen Dosierung von B11, d. h. bei vollständiger Blockierung des ORL1-Wirkmechanismus bleibt eine analgetische Wirksamkeit von A4 bestehen.

Abbildung 4 zeigt, dass durch Antagonisierung der μ- bzw. der ORL1-Komponente von A6, A5 und A1 mit

Naloxon bzw B11 jeweils eine analgetische Wirkung der nicht antagonisierten Komponente bestehen bleibt.

**Trennung von anti-nociceptivem und anti-allodynischem Effekt bei neuropathischen Tieren: Vergleich von A4 und Morphin bei neuropathischen Tieren**

Im Chung-Modell kann durch vergleichende Testung der Schmerzreaktion an der ipsi- und an der contratateralen Pfote zwischen antinociceptiver (contralateral) und antiallodynischer (ipsilateral) Wirkung unterschieden werden.

Für Morphin konnte eine rein antiallodynische Wirkung nur nach Applikation von 1 mg/kg iv. beobachtet werden. Die maximale Wirksamkeit beträgt dabei 29% MPE. Bei der nächst höheren Testdosierung (2,15 mg/kg iv.) tritt bereits eine deutliche antinociceptive Wirkung ein (Abbildungen 5, 5a).

Im Gegensatz dazu beträgt die maximale, rein antiallodynische Wirkung von A4 56% MPE. Diese wird bei einer Testdosierung von 1 μg/kg iv. erreicht (Abbildungen 6, 6a).

<u>Schlussfolgerung:</u> Durch die ORL1-Komponente wird eine signifikant stärkere antiallodynische Wirkung als bei reinen μ-Opioiden erreicht.

**Direkter Vergleich von A4 und Morphin in naiven und neuropathischen Tieren**

Um einen möglichen Einfluß der "Schmerzqualität" (Tail-flick, nociceptiver Reiz vs. Chung, taktile Allodynie) beim Vergleich der unterschiedlichen Wirksamkeit bei Akutschmerz und Neuropathieschmerz auszuschließen, wurden A4 und Morphin vergleichend in Tieren mit spinaler Nervenligatur (Chung-Modell) und scheinoperierten Tieren getestet. Als Schmerzmodell wurde in allen Fällen der Tail-flick verwendet. Der direkte Vergleich zeigt, dass nach Ausbildung der Neuropathie die Wirksamkeit von Morphin abnimmt (was der klinischen Situation entspricht), während sie für A4 zunimmt (s. Abbildungen 7 und 8).

Bei vergleichbarer Wirksamkeit beider Substanzen im Akutschmerz (s.u.) ist die antiallodynische Wirksamkeit von A4 um ca. einen Faktor 10 höher als von Fentanyl.

**Vergleich von Cytostatika-induziertem Polyneuropathieschmerz und diabetisch induziertem Polyneuropathieschmerz**

A4 zeigt im Vincristin-induzierten Polyneuropathieschmerz an der Ratte eine signifikante Wirksamkeit bei einer Dosierung von 1 μg/kg (Abb. 24). Bei einer Dosierung von 0,464 mg/kg ist noch keine signifikante Wirksamkeit zu beobachten (14,7 ± 10,2 % MPE). Im diabetisch induzierten Neuropathieschmerz dagegen wird bereits für die niedrigste untersuchte Dosierung (0,316 μg/kg) eine signifikante Wirksamkeit beobachtet (Abb. 26). In diesem Dosisbereich ist noch kein antinociceptiver Effekt zu beobachten. Die klinisch eingesetzten Vergleichssubstanzen Morphin und Pregabalin zeigen erst in einem Dosisbereich Wirksamkeit im diabetischen Polyneuropathleschmerz, in dem auch ei antinociceptiver Effekt zu beobachten ist (Abb. 27, 28).

**Wirkverstärkung bei Entzündungsschmerz durch gemischte ORL1/μ-Agonisten**

**a) SIngle-Motor-Unit Ableitungen In spinalisierten Ratten. Vergleich von naiven Tieren und Tieren nach Carrageenan-induzierter Entzündung.**

Das Modell ist in der Uteratur beschrieben (Herrero & Headley, 1996, Br J Pharmacol 118, 968-972).
24 h nach Induktion einer Entzündung (100μl Carrageenan, 1%, intraplantar) ist die anti-nociceptive Wirkung von A4 (gemessen als Inhibition der SMU-Aktivitat nach mechanischer (Pinch) bzw. elektrischer (Wind-up) Stimulation) deutlich erhöht (s. Abbildungen 10 und 10a). Die antinociceptive Wirkung von Morphin ändert sich dagegen nach Entzündung nicht (s. Abbildungen 11 und 11a).

Darüber hinaus konnte in diesem Modell auch für A11 eine Wirkverstärkung nach Entzündungsinduktion gezeigt werden.

**CFA-Induzierte Hyperalgesie**

**Complete Freund's adjuvant (CFA) induzierte Hyperalgesie bei der Ratte**

Die CFA-induzierte Hyperalgesie stellt ein Tiermodell des chronischen Entzündungsschmerzes dar. Männlichen Sprague-Dawley Ratten (150 -180g) wird einmalig 100 μl unter Hitze abgetöteten und getrockneten Mycobakterien (Mycobacterium tuberculosis; H37 Ra) in einer Mischung aus Paraffinöl und Mannid monooleat als Emulgator (Complete Freund's Adjuvant, CFA) subplantar injiziert (Dosis 1 mg/ml). Einen Tag nach der CFA-Injektion wird mit Hilfe eines

elektronischen von Frey Haares (Somedic Sales AB, Hörby, Schweden) die taktile Hyperalgesie überprüft. Dazu werden die Tiere in eine Plastikbox mit einem Gitterboden gesetzt, welcher freien Zugang zu beiden Hinterpfoten ermöglicht. Mit dem von Frey Filament wird die Pfote subplantar gereizt. Zur Quantifizierung der Sensitivität sowohl der ipsials auch der (unbehandelten) contralateralen Pfote auf den mechanischen Stimulus wird die Pfotenwegziehschwelle in Gramm des aufgebrachten Drucks angegeben. Pro Pfote wird die Stimulation 4x in einem zeitlichen Intervall von je 30 Sekunden wiederholt. Aus den vier Messwerten wird der Median gebildet. Die Wegziehschwelle der ipsi- und contralateralen Pfote wird zu verschiedenen Zeitpunkten nach CFA injektion (1h, 3h, 1.Trag, 4.Trag), vor (= Vorwert) und zu verschiedenen Zeitpunkten nach Substanzgabe (Messwert) bestimmt. Eine Kontrolltiergruppe der Lösungsmittel appliziert wird, wird mitgeführt. Die Wirksamkeit einer Substanz wird als % Inhibition der Hyperalgesie und weiterhin als MPE% wie folgt berechnet

$$\% \text{ Inhibition der HA} = (1 - \text{HA Messwert} / \text{HA Vorwert}) \times 100$$

HA Vorwert = Wegziehschwelle contralateral - Wegziehschwelle ipsilateral vor Substanzgabe
HA Messwert = Wegziehschwelle contralateral - Wegziehschwelle ipsilateral nach Substanzgabe

$$\% \text{ MPE} = [(\text{WSs ipsi} - \text{WSo ipsi}) / \text{WSo contra} - \text{WSo ipsi}] \times 100$$

WSo contra = Wegziehschwelle der contralateralen unbehandelten Pfote
WSo ipsi = Wegziehschwelle der ipsilateralen unbehandelten Pfote
WSs ipsi = Wegziehschwelle der ipsilateralen behandelten Pfote nach Substanzgabe
MPE%: Prozent des maximal möglichen Effekts; der maximale mögliche Effekt ist als die Wegziehschwelle der contralateralen unbehandelten Pfote definiert

Insgesamt werden pro Versuchstiergruppe (Substanz und Kontrolle) 10 Ratten eingesetzt. Der Mittelwert ± SEM wird aus den Medianen der Einzeltiere berechnet. Die Signifikanzberechnung erfolgt mittels Zwei-Faktor ANOVA für wiederholte Messungen. Die Signifikanz der Interaktion Substanz-Applikation (Behandlung), Zeit, Zeit * Behandlung wird analysiert mit Wilks' Lambda statistics. Im Falle eines signifikanten Behandlungseffektes wird ein Fischer-Test mit anschließendem post hoc Dunnett-Test durchgeführt.

Während Morphin über den Untersuchungszeitraum tendenziell eine leichte Abnahme der antihyperalgetischen bzw. eine gleichbleibende antinociceptive Wirkung zeigt, nehmen antihyperalgetische und antinociceptive Wirkung von A4 Ober 24 h zu. Der Effekt ist für mindestens 4 Tage stabil (s. Abbildungen 12, 12a).

**Senföl induzierter visceraler Entzündungsschmerz bei der Maus**

Männliche NMRI Mäuse (Körpergewicht 20-35g) werden auf einem Gitterrost in Plexiglaskäfigen (14.5 x 14.5 cm, Höhe 10cm) für etwa dreißig Minuten habituiert.

Das Verhalten der Mäuse auf zehnmalige mechanische Stimulierung mittels von Frey Filamenten (1, 4, 8, 16, 32 mN) auf die Bauchdecke wird als Vorwert erhoben. Das Verhalten wird entweder über die Summe der Anzahl der nozifensiven Reaktionen oder Ober die Qualität dieser nozifensiven Reaktionen und deren Gewichtung durch Multiplikation der Anzahl der Reaktionen mit dem zugehörigen Faktor (Faktor 1: leichtes Anheben des Abdomens, Lecken an der Reizstelle, Weggehen; Faktor 2: Wegstrecken der Hinterpfoten, leichtes Weghüpfen, Zucken der Hinterpfoten, ruckartiges, starkes Lecken der Reizstelle; Faktor 3: Wegspringen, Vokalisation) und anschließende Summenbildung analysiert.

Anschließend erfolgt die Applikation von Prüfsubstanz oder Vehikel je nach kinetischer Eigenschaft der Substanz mit einer geeigneten Applikationsart zu einer geeigneten Zeit vor der Gabe von Senföl. Die Gruppengröße beträgt üblicherweise n=7.

Eine akute Kolitis wird durch rektale Gabe von 50µl Senföl (3.5% in PEG200) induziert. Zwei bis zwölf Minuten nach Senfölgabe zeigen die Tiere ein spontanes viszerales Schmerzverhalten, weiches beobachtet wird. Die Anzahl der Reaktionen wird mit dem zugehörigen Faktor multipliziert (Faktor 1: Lecken der Abdominalwand; Faktor 2: Strecken. Drücken des Abdomens gegen den Boden, Brückenhaltung, Kontraktion des Abdomens, Rückwärtsbewegung oder Kontraktion der Flankenmuskulatur) und anschließend die Summe gebildet, welche den spontanen viszeralen Schmerzscore darstellt. Eine Gruppe von Tieren erhält anstelle von Senföl eine rektale Applikation von 50µl PEG200.

Zwanzig bis vierzig Minuten nach Senfölgabe wird erneut das Verhalten der Tiere auf zehnmalige mechanische Stimulierung mittels von Frey Filamenten (1, 4, 8, 16, 32 mN) auf die Bauchdecke beobachtet und wie oben beschrieben

quantifiziert. Die übertragene mechanische Allodynia wird hierbei aus der Summe der Reaktionen auf die Stimulierung mit dem von Frey Filament der Stärke 1mN bestimmt. Die übertragene mechanische Hyperalgesie wird aus der Summe der gewichteten Reaktionen auf die Stimulierung mit dem von Frey Filament der Stärke 16mN bestimmt.

Die Wirkung von der Prüfsubstanz wird im Vergleich zu Vehikel beschrieben durch 1. Hemmung des spontanen viszeralen Schmerzverhaltens, 2. Hemmung der übertragenen mechanischen Allodynie und 3. Hemmung der übertragenen mechanischen Hyperalgesie.

Die Daten werden mittels mehrfaktorieller Varianzanalyse mit wiederholter Messung untersucht und bei Vorliegen einer signifikanten Wirkung der Prüfsubstanz (P<0.05) werden die Einzeldaten über eine post hoc Analyse nach Bonferroni auf Signifikanz geprüft. Bei Dosiswirkungskurven können $ED_{50}$ Werte, welche die Dosis mit der halbmaximalen Wirkung beschreiben, durch lineare Regressionsanalyse bestimmt werden. (nach Christoph et al., 2005, Eur. J. Pharmacol. 507:87-98).

Die vergleichende Testung von A4 und Fentanyl in einem Modell zur übertragenen Allodynie und zur übertragenen Hyperalgesie in Mäusen nach Senföl induzierter, nichtneurogener visceraler Entzündung zeigte für alle drei Schmerzparameter, vor allem aber für die Allodynie und die Hyperalgesie eine signifikant höhere Wirksamkeit des gemischten ORL1/μ-Agonisten im Vergleich zu dem reinen μ-Opioid.

Übertragene Allodynie

| Substanz | Ratio ORL1/μ | $ED_{50}$ akut | $ED_{50}$ visceraler Schmerz | Faktor Wirkverstärkung |
|---|---|---|---|---|
| B1 (Fentanyl) | < 1 : 100 | 30 μg/kg i.v.[1] | 47 μg/kg i.v. | 0.6 x |
| A4 | 1 : 2 | 19 μg/kg i.v.[1] | 2.8 μg/kg i.v. | 7 x |
| [1]) Tail-flick, Maus | | | | |

Übertragene Hyperalgesie

| Substanz | Ratio ORL1/μ | $ED_{50}$ akut | $ED_{50}$ visceraler Schmerz | Faktor Wirkverstärkung |
|---|---|---|---|---|
| B1 (Fentanyl) | < 1 : 100 | 30 μg/kg i.v.[1] | 42 μg/kg i.v. | 0.7 x |
| A4 | 1 : 2 | 19 μg/kg i.v.[1] | 3.0 μg/kg i.v. | 6 x |
| [1]) Tail-flick, Maus | | | | |

Die analgetische Wirksamkeit von A4 in bezug auf die beiden getesten Schmerzparameter ist um ca. einen Faktor 6 bis 7 höher als im Akutschmerz. Im Gegensatz dazu ist die analgetische Wirksamkeit von Fentanyl im visceralen Entzündungsschmerz geringer als im Akutschmerz.

**Wirkung In Akutschmerzmodellen**

Die gemischten ORL1/μ-Agonisten mit einem ORL1 : μ-Verhältnis von 1 : 10 bis 30:1 zeigen volle Wirksamkeit in Akutschmerzmodellen (Tail-flick, Maus und Ratte). Die Ergebnisse im Tail-flick sind in Tabelle 3 dargestellt (s. o.). Gezeigt wird der Effekt anhand von Beispielen zwischen ORL1:μ 1:10 bis 20:1. Entsprechend ihrer Bindungsaffinität zum μ-OR liegt die Wirkstärke im Bereich der Standardopioide (Sufentanil, Fentanyl, Buprenorphin, Oxycodon, Morphin) (s. Abbildung 13).

**Opioid-induzierte Hyperalgesie**

Die chronische Gabe von Opioiden führt zu einer Hyperalgesie bei Schmerzpatienten (vgl. Chu et al. 2006, J. Pain 7:43-48). Ein ähnliches Phänomen tritt auch nach akuter Gabe in der Entzugssituation auf (Angst et al. 2003, Pain 106:49-57). Im Tiermodell induzieren reine μ-Opioide nach akuter Gabe eine transiente Hyperalgesie (Opioid-induced Hyperalgesia. A qualitative systematic review.

Angst and Clark, Anesthesiology 2006; 104:570-87), die z.B. im Soft Tail-flick-Modell als transiente "pronociceptive" Phase detektierbar ist. Entsprechende Befunde sind literaturbeschrieben Diese opioidinduzierte Hyperalgesie wurde mit Hilfe eines modifizierten soft Tail-flick-Modells (25% Brennstrahlintensität) für reine μ-Opioide (Fentanyl und Morphin) gezeigt. Im Gegensatz dazu wurde nach akuter Gabe von gemischten ORL1/μ-Agonisten (A4 und A10) keine transiente Hyperalgesie beobachtet (Abbildungen 14-14c).

**Bestimmung der körperlichen Abhängigkeit**

Eine Testung erfolgte in zwei Modellen: Naloxon-induzierter Entzug bei Mäusen und Spontanentzug bei Ratten. In beiden Modellen waren die Entzugssymptome bei gemischten ORL1/μ-Agonisten gegenüber reinen μ-Agonisten deutlich reduziert.

**Jumping-test an der Maus: Versuch zur Bestimmung der körperlichen Abhängigkeit (Saelens JK, Arch Int Pharmacodyn 190: 213.218, 1971)**

Die Testsubstanzen werden insgesamt 7x über zwei Tage intraperitoneal appliziert 5 Applikationen erfolgen am ersten Tag um 9:00, 10:00, 11:00, 13:00 und 15:00 Uhr und am zweiten Tag um 9:00 und 11:00 Uhr. Die ersten 3 Applikationen werden In aufsteigenden Dosierungen (Dosierungsschema) gegeben und dann weiter in der Dosierung der dritten. Der Entzug wird 2 Stunden nach der letzten Substanzaplikation mit Naloxon 30 mg/kg (i.p.) präzipititert. Unmittelbar danach werden die Tiere einzeln in durchsichtige Beobachtungsboxen (Höhe 40 cm, Durchmesser 15 cm) gesetzt und die Sprungreaktionen über 15 Minuten in jeweils 5-Minuten- Perioden gezählt. Morphin wird in einer Dosierung als Vergleich/Standard mitgeführt.

Die Quantifizierung des Entzugs erfolgt über die Anzahl der Sprünge 0 bis 10 min. nach Naloxonapplikation. Die Anzahl der Tiere pro Gruppe mit mehr als 10 Sprüngen/10 min wird bestimmt und als "% positive Tiere" dokumentiert. Außerdem wird die durchschnittliche Sprung-Frequenz in der Gruppe errechnet. Pro Gruppe werden 12 Tiere eingesetzt.

Die μ-Agonisten B1-B4 induzieren ein deutliches Entzugsspringen. Der μ-Agonist B7 (L-Methadon, Levomethadon, Abbildung 15) induziert ein im Vergleich zu B1-B4 reduziertes, jedoch signifikantes Entzugsspringen aus. Auch B8 und A1 lösen in desem Test ein signifikantes Entzugsspringen aus (Abbildungen 15a und 15b). A9 löst dagegen nur ein geringes Entzugsspringen aus, dass bei höheren Dosierungen vollständig unterdrückt wird (Abbildung 15c). Nach Gabe von A4 bzw A7 tritt kaum bzw. kein signifikantes Entzugsspringen auf (Abbildungen 15d und 15e).

**Spontanentzug an der Ratte:**

Die Studie zum Opiatspontanentzug wurde in 5 Phasen durchgeführt.

> <u>Phase 1</u> (chronische Behandlungsphase): Ratten werden über 3 Wochen mit der Testsubstanz behandelt. Die Applikation wurde 2 oder 3x täglich (je nach Wirkdauer der Testsubstanz) intraperitoneal vorgenommen.
> <u>Phase 2</u> (Spontanentzug): Anschließend erfolgte ein Spontanentzug und eine behandlungsfreie Periode (<u>Phase 3)</u> von einer Woche. Anschließend erhielten die Tiere eine weitere Woche die Testsubstanz (<u>Phase 4</u>).
> <u>Phase 5</u> (Naloxon-induzierter Entzug): Dann wurde mit Naloxon (10 mg/kg i.p.) ein Entzug eingeleitet.

Messparameter im Entzug: Tiergewichte, Verhaltensparameter:

> Beurteilung der (6) Hauptsymptome im Entzug: Tremor, Salivation, Writhing, wet dog shaking, Hopsen und Springen, Zähneknirschen
> 0= nicht vorhanden, 1=leicht, 2= schwer
> maximaler Score = 12
> Als Referenzsubstanz wurde Morphin mitgeführt

Die Studie zum Opiatspontanentzug wurde nach Literaturbeschreibung angelegt Jaffe JH (1990) Drug addiction and drug abuse. In: Goodman Gilman A, Rall TW, Nies AS, Taylor P (eds.) The pharmacological basis of therapeutics. New York, Pergamon Press: 522-573.

Bläsig J, Herz A, Reinhold K, Zieglgänsberger S (1973) Development of physical dependence on morphine in respect to time and dosage and quantification of the precipitated withdrawal syndrome. Psychopharmacology 33: 19-38.

Die Ergebnisse im Spontanentzug sind in Abbildung 16 dargestellt

A4, A7 und A9 zeigen im Jumping test keine oder zumindest im Vergleich zu Morphin deutlich verringerte Entzugssymptomatik A1 (ORL1:μ 1:10) bewirkt im Entzugsspringen Entzugsverhalten, im Spontanentzug ist jedoch

kein signifikanter Gewichtsverlust zu beobachten. Bei vorheriger Gabe von Morphin sinkt das Körpergewicht der Ratte jedoch um ca 10%. A1 zeichnet sich daher im Vergleich zu Morphin durch ein reduziertes Abhängigkeitspotential aus.

**Reduktion der μ-vermittelten Atemdepression durch einen ORL1-abhängigen Mechanismus**

**Akute μ-vermittelte Atemdepression bei der Ratte**

**Methode zur $pCO_2$- und $pO_2$- Messung bei der Ratte (Blutgasanalyse)**

Die atemdepressive Wirkung von Testsubstanzen wird nach i.v.-Gabe an instrumentierten, wachen Ratten untersucht. Testparameter ist die Veränderung von Kohlendioxid-Partialdruck ($pCO_2$) und Sauerstoff-Partialdruck ($pO_2$) im arteriellen Blut nach Substanzgabe.

Versuchstiere: männl.Ratten, Sprague-Dawley; Gewicht: 250-275g Versuchsvorbereitung: Mindestens 6 Tage vor der Prüfsubstanzapplikation wird den Ratten unter Pentobarbitalnarkose jeweils ein PP-Katheter in die Arteria femoralis und in die Vena jugularis implantiert. Die Katheter werden mit Heparinlösung (4000 I.E.) gefüllt und mit einem Drahtstift verschlossen.

Versuchsablauf: Die Substanz- bzw. Vehikelapplikation erfolgt über den Venenkatheter. Vor der Substanz- bzw. Vehikelapplikation und zu definierten Zeitpunkten nach Substanz- bzw. Vehiketapplikation wird jeweils der Arterienkatheter geöffnet und mit ca. 500 μl Heparinlösung gespült. Dann werden ca. 100 μl Blut aus dem Katheter entnommen und mittels einer heparinisierten Glaskapillare aufgenommen. Der Katheter wird nochmals mit Heparinlösung gespült und wieder verschlossen. Das arterielle Blut wird sofort mit Hilfe eines Blutgasanalysegerätes (ABL 5, Radiometer GmbH,Willich, Deutschland) vermessen.

Nach einer Mindestauswaschzeit von einer Woche können die Tiere erneut in den Versuch genommen werden.

Versuchsauswertung: Das Blutgasanalysegerätes liefert automatisch die Werte für $pCO_2$ und $pO_2$ des Blutes in mmHg. Substanzeffekte auf den Partialdruck werden als prozentuale Änderungen gegenüber den Vorwerten ohne Substanz- bzw. Vehikel berechnet. Zur statistischen Auswertung werden die Messwerte nach Substanzgabe und die zeitgleichen Messwerte nach Vehikelapplikation mittels Ein-Faktor Varianzanalyse verglichen. Im Falle eines signifikanten Substanzeffektes wird ein post hoc Dunnett-Test durchgeführt.

Bei reinen μ-Opioiden (hier Fentanyl und Oxycodon, Abbildungen 17 und 17a) kommt es zum Zeitpunkt des analgetischen Wirkmaximums aufgrund der μ-induzierten Atemdepression zu einem deutlichen Anstieg des arteriellen $pCO_2$. Bei einer 90 - 100% wirksamen Dosis steigt der $pCO_2$-Wert um mehr als 50% an.

Im Vergleich dazu wurde der $pCO_2$-Wert bei gemischten ORL1/μ-Agonisten bestimmt. Selbst bei einer Dosierung, die über mehrere Stunden analgetisch maximal wirksam ist, steigt der arterielle pCO2 nach Gabe der gemischten ORL1/μ-Agonisten nur um ca. 20 - 30% an (Abbildungen 17b-17e).

Der beobachtete Effekt wurde am Beispiel von A4 auf seine Ursache hin untersucht. Dabei wurde zum Zeitpunkt 0 mit A4 zusammen B11 (2.15 mg/kg) verabreicht (i. v.), um die ORL1-Komponente zu antagonisieren und nur noch den μ-Effekt zu beobachten. In einem weiteren Experiment wurde 20 Minuten nach Gabe von A4 + B11 Naloxon (1 mg/kg i.v.) gegeben, um zu prüfen, ob der resultierende atemdepressorische Effekt ausschließlich ein μ-vermittelter Effekt ist.

Das Ergebnis zeigt, dass die im Vergleich zu reinen μ-Opioiden reduzierte Atemdepression von A4 ganz klar auf die ORL1-Komponente zurückzuführen ist (Abbildung 18). So steigt der $pCO_2$-Wert nach Antagonisierung mit B11 auf einen Wert an, der typisch für reine μ-Opioide ist. Wird nach Erreichen der maximalen $pCO_2$-Erhöhung Naloxon gegeben, sinkt der Wert wieder. Das belegt, dass die μ-vermittelte Atemdepression durch die ORL1-Komponente reduziert wird.

**Sicherheitsabstand**

Die Sicherheitsabstände für verschiedene gemischte ORL1/μ-Agonisten und reine μ-Agonisten dargestellt als Abstand zwischen Schwellendosis ($ED_{10}$) für einen Anstieg des arteriellen $pCO_2$ und der im Chung-Modell halbmaximal wirksamen Dosierung ($ED_{50}$) sind in Abbildung 20 verdeutlicht.

Die Schwellendosis ($ED_{10}$) für einen Anstieg des arteriellen $pCO_2$ liegt bei A1, A4, A5 und A7 ca. um einen Faktor 3 bis 20 oberhalb der im Chung-Modell halbmaximal wirksamen Dosierung ($ED_{50}$), während die Schwellendosis für die μ-Agonisten B1, B3, und 85 im Bereich der im Chung-Modell halbmaximal wirksamen Dosierung ($ED_{50}$) liegt bzw. für B4 sogar deutlich unterhalb. Die Sicherheitsabstände zwischen Wirkung und Nebenwirkung sind daher für die gemischten ORL1/μ-Agonisten im Vergleich zu den μ-Agonisten deutlich vergrößert.

**Psychische Abhängigkeit/Sucht**

Zur Untersuchung der Platzpräferenz siehe: Tzschentke, T.M., Bruckmann, W. and Friderichs, F. (2002) Lack of sensitization during place conditioning in rats is consistent with the low abuse potential of tramadol. Neuroscience Letters 329, 25-28.

A4, A6 und A7 induzieren eine Platzpräferenz, die jedoch im Vergleich zu den reinen $\mu$-Antagonisten B1 und B3-B5 in einem Dosisbereich, die um bis zu einem Faktor 100 niedriger liegt (Abbildung 21).

Es wurde am Beispiel von A7 gezeigt, dass die hier reduzierte Platzpräferenz auf die ORL1-Komponente zurückzuführen ist. Zunächst wurde die Platzpräferenz bei verschiedenen Dosierungen getestet (Abbildung 22)

Nach Gabe von A7 wurde mit B11 antagonisiert. Es konnte gezeigt werden, dass nach Block der ORL1-Komponente die Schwelle zur Induktion einer Platzpräferenz zu niedrigeren Dosierung hin verschoben wird (Abbildung 23). Dieser Befund belegt, dass die ORL1-Komponente eine $\mu$-OR vermittelte Platzkonditionierung abschwächt.

## Patentansprüche

1. Verbindungen, die eine Affinität zum $\mu$-Opioid-Rezeptor von mindestens 100 nM ($K_i$-Wert human) und eine Affinität zum ORL-1-Rezeptor aufweisen, wobei das Verhältnis zwischen den Affinitäten ORL1/$\mu$ definiert als 1 /[$K_i$(ORL1) /$K_i$($\mu$)] von 0,1 bis 30 liegt, und welche spirocyclische Cyclohexan-Derivate der allgemeinen Formel (I) sind

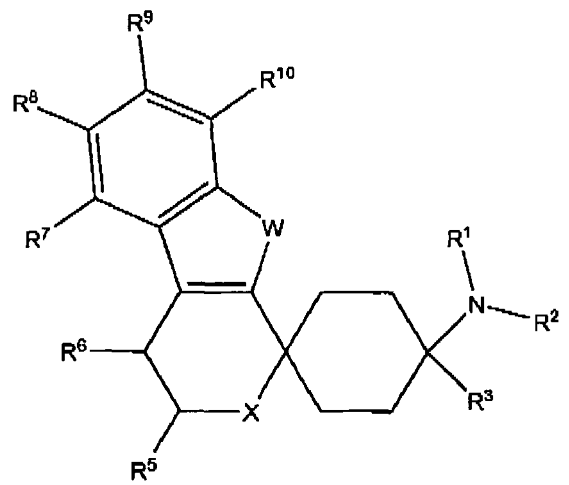

(I)

worin
$R^1$ und $R^2$ unabhängig voneinander für H oder $CH_3$ stehen, wobei $R^1$ und $R^2$ nicht gleichzeitig H bedeuten;
$R^3$ für Phenyl, Benzyl oder Heteroaryl jeweils unsubstituiert oder einfach oder mehrfach mit F, Cl, OH, CN oder $OCH_3$ substituiert, steht;
W für $NR^4$, O oder S steht; wobei
$R^4$ für H; $C_{1-5}$-Alkyl Phenyl; über eine $C_{1-3}$-Alkyl-Gruppe gebundenes Phenyl, $COR^{12}$; $SO_2R^{12}$ steht,
$R^{12}$ H; $C_{1-7}$-Alkyl, verzweigt oder unverzweigt, gesättigt oder ungesättigt, unsubstituiert oder einfach oder mehrfach mit OH, F oder $COOC_{1-4}$-Alkyl substituiert; $C_{4-6}$-Cycloalkyl; Aryl-, oder Heteroaryl, unsubstituiert oder einfach oder mehrfach mit F, Cl, Br, $CF_3$, $OCH_3$, $C_{1-4}$-Alkyl, verzweigt oder unverzweigt, unsubstituiert oder substituiert mit F, Cl, CN, $CF_3$, $OCH_3$ oder OH; oder über gesättigtes oder ungesättigtes $C_{1-3}$-Alkyl gebundenes Phenyl oder Heteroaryl, unsubstituiert oder einfach oder mehrfach mit F, Cl, Br, $CF_3$, $OCH_3$, $C_{1-4}$-Alkyl, verzweigt oder unverzweigt, unsubstituiert oder substituiert mit F, Cl, CN, $CF_3$, $OCH_3$ oder OH substituiert; oder über gesättigtes oder ungesättigtes $C_{1-3}$-Alkyl gebundenes $C_{5-6}$-Cycloalkyl; $OR^{13}$; $NR^{14}R^{15}$ bedeutet;
$R^5$ für H; $COOR^{13}$, $CONR^{13}$, $OR^{13}$; $C_{1-5}$-Alkyl, gesättigt oder ungesättigt, verzweigt oder unverzweigt, unsubstituiert oder einfach oder mehrfach mit OH, F, $CF_3$ oder CN substituiert, steht;
$R^6$ für H steht;
oder $R^5$ und $R^6$ gemeinsam $(CH_2)_n$ mit n = 2, 3, 4, 5 oder 6 bedeuten, wobei einzelne Wasserstoffatome auch durch F, Cl, $NO_2$, $CF_3$, $OR^{13}$, CN oder $C_{1-5}$-Alkyl ersetzt sein können;
$R^7$, $R^6$, $R^9$ und $R^{10}$ unabhängig voneinander für H, F, Cl, Br, $NO_2$, $CF_3$, OH, $OCH_3$, CN, $COOR^{13}$, $NR^{14}R^{15}$; $C_{1-5}$-Alkyl, Heteroaryl, unsubstituiert oder einfach oder mehrfach mit Benzyl, $CH_3$, Cl, F, $OCH_3$ oder OH substituiert, stehen;

wobei $R^{13}$ H oder $C_{1-5}$-Alkyl bedeutet;
$R^{14}$ und $R^{15}$ unabhängig voneinander H oder $C_{1-5}$-Alkyl bedeuten;
X für O, S, SO, $SO_2$ oder $NR^{17}$ steht;
$R^{17}$ für H; $C_{1-6}$-Alkyl, gesättigt oder ungesättigt, verzweigt oder unverzweigt; $COR^{12}$ oder $SO_2R^{12}$ steht;
in Form ihrer reinen Diastereomeren, ihrer Racemate, ihrer reinen Enantiomeren, oder in Form von Mischungen der Stereoisomeren in einem beliebigen Mischungsverhältnis; als Basen oder in Form ihrer Salze, insbesondere der physiologisch verträglichen Salze oder Salze physiologisch verträglicher Säuren oder Kationen;

- zur Anwendung bei einer Narkose;
- zur Anwendung bei der narkosebegleitenden Behandlung von Schmerz;
- zur Anwendung bei der Behandlung von neuropathischem Schmerz ausgewählt aus der Gruppe bestehend aus chronischem neuropathischem Schmerz, polyneuropathischem Schmerz und Cytostatika-induziertem Polyneuropathieschmerz.

**2.** Verbindungen zur Anwendung bei der Behandlung von chronischem neuropathischem Schmerz nach Anspruch 1, welche in einer Dosierung angewendet werden, die um mindestens einen Faktor 2 niedriger liegt als die im Akutschmerz angewandte Dosierung.

**3.** Verbindungen zur Anwendung nach Anspruch 2, welche in einer Dosierung angewendet werden, die um mindestens einen Faktor 5 niedriger liegt als die im Akutschmerz angewandte Dosierung.

**4.** Verbindungen zur Anwendung nach Anspruch 1, ausgewählt aus der Gruppe bestehend aus
1,1-(3-Methylamino-3-phenylpentamethylen)-6-fluor-1,3,4,9-tetrahydropyrano[3,4-b]indol Hemicitrat;
1,1-(3-Methylamino-3-phenylpentamethylen)-1,3,4,9-tetrahydropyrano[3,4-b]indol Hemicitrat;
1,1-[3-Dimethylamino-3-(3-thienyl)pentamethylen]-1,3,4,9-tetra-hydro-pyranot[3,4-b]lindol Hemicitrat;
1,1-(3-Dimethylamino-3-phenylpentamethylen)-6-fluor-1,3,4,9-tetrahydropyrano[3,4-b]indol Hemicitrat;

1,1-[3-Methylamino-3-(2-thienyl)pentamethylen]-1,3,4,9-tetrahydro-pyrano[3,4-b]-6-fluorindol Citrat;
1,1-[3-Dimethylamino-3-(2-thienyl)pentamethylen]-1,3,4,9-tetra-hydro-pyrano[3,4-b]-6-fluorindol Hemicitrat;
1,1-[3-Dimethylamino-3-(2-thienyl)pentamethylen]-1,3,4,9-tetra-hydro-pyrano[3,4-b]indol Citrat;
1,1-[3-Dimethylamino-3-(3-thienyl)pentamethylen]-1,3,4,9-tetra-hydro-pyrano[3,4-b]-6-fluorindol Hemicitrat;
1,1-(3-Dimethylamino-3-phenylpentamethylen)-1,3,4,9-tetrahydropyrano[3,4-b]indol Hemicitrat; und
1,1-[3-Methylamino-3-(2-thienyl)pentamethylen]-1,3,4,9-tetrahydro-pyrano[3,4-b]indol Citrat.

**5.** 1,1-(3-Methylamino-3-phenylpentamethylen)-6-fluor-1,3,4,9-tetrahydropyrano[3,4-b]indol in Form eines reinen Diastereomers oder in Form von Mischungen der Stereoisomere in einem beliebigen Mischungsverhältnis; als Base oder in Form eines physiologisch verträglichen Salzes; zur Anwendung bei der Behandlung von neuropathischem Schmerz.

**6.** 1,1-(3-Dimethylamino-3-phenylpentamethylen)-6-fluor-1,3,4,9-tetrahydropyrano[3,4-b]indol in Form eines reinen Diastereomers oder in Form von Mischungen der Stereoisomere in einem beliebigen Mischungsverhältnis; als Base oder in Form eines physiologisch verträglichen Salzes; zur Anwendung bei der Behandlung von neuropathischem Schmerz.

## Claims

**1.** Compounds which exhibit an affinity for the μ-opioid receptor of at least 100 nM ($K_i$ value, human) and an affinity for the ORL-1 receptor, wherein the ratio between the affinities ORL1/μ defined as $1/[K_i(ORL1)/K_i(\mu)]$ is from 0.1 to 30, and which are the spirocyclic cyclohexane derivates of the general formula (I)

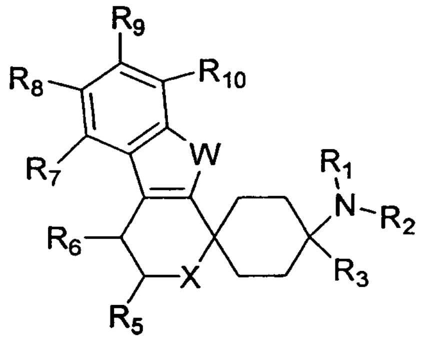

I

in which
$R^1$ and $R^2$ mutually independently denote H or $CH_3$, wherein $R^1$ and $R^2$ do not simultaneously mean H;
$R^3$ denotes phenyl, benzyl or heteroaryl, in each case unsubstituted or mono- or polysubstituted with F, Cl, OH, CN or $OCH_3$;
W denotes $NR^4$, O or S
and
$R^4$ denotes H; $C_{1-5}$ alkyl, phenyl; phenyl, $COR^{12}$ attached via a $C_{1-3}$ alkyl group; $SO_2R^{12}$,
wherein $R^{12}$ means H; $C_{1-7}$ alkyl, branched or unbranched, saturated or unsaturated, unsubstituted or mono- or polysubstituted with OH, F or $COOC_{1-4}$ alkyl; $C_{4-6}$ cycloalkyl; aryl, or heteroaryl, unsubstituted or mono- or polysubstituted with F, Cl, Br, $CF_3$, $OCH_3$, $C_{1-4}$ alkyl, branched or unbranched, unsubstituted or substituted with F, Cl, CN, $CF_3$ $OCH_3$ or OH; or phenyl or heteroaryl, unsubstituted or mono- or polysubstituted with F, Cl, Br, $CF_3$, $OCH_3$, $C_{1-4}$ alkyl, branched or unbranched, unsubstituted or substituted with F, Cl, CN, $CF_3$, $OCH_3$ or OH, attached via saturated or unsaturated $C_{1-3}$ alkyl; or $C_{5-6}$ cycloalkyl attached via saturated or unsaturated $C_{1-3}$ alkyl; $OR^{13}$; $NR^{14}R^{15}$;
$R^5$ denotes H; $COOR^{13}$, $CONR^{13}$, $OR^{13}$; $C_{1-5}$ alkyl, saturated or unsaturated, branched or unbranched, unsubstituted or mono- or polysubstituted with OH, F, $CF_3$ or CN;
$R^6$ denotes H;
or $R^5$ and $R^6$ together mean $(CH_2)_n$ with n = 2, 3, 4, 5 or 6, wherein individual hydrogen atoms may also be replaced by F, Cl, $NO_2$, $CF_3$, $OR^{13}$, CN or $C_{1-5}$ alkyl;
$R^7$, $R^8$, $R^9$ and $R^{10}$ mutually independently denote
H, F, Cl, Br, $NO_2$, $CF_3$, OH, $OCH_3$, CN, $COOR^{13}$, $NR^{14}R^{15}$; $C_{1-5}$ alkyl, heteroaryl, unsubstituted or mono- or polysubstituted with benzyl, $CH_3$, Cl, F, $OCH_3$ or OH;
wherein $R^{13}$ means H or $C_{1-5}$ alkyl;
$R^{14}$ and $R^{15}$ mutually independently mean H or $C_{1-5}$ alkyl;
X denotes O, S, SO, $SO_2$ or $NR^{17}$;
$R^{17}$ denotes H; $C_{1-5}$ alkyl, saturated or unsaturated, branched or unbranched; $COR^{12}$ or $SO_2R^{12}$,
in the form of the pure diastereomers thereof, the racemates thereof, the pure enantiomers thereof, or in the form of mixtures of the stereoisomers in any desired mixing ratio; as bases or in the form of the salts thereof, in particular the physiologically acceptable salts, or salts of physiologically acceptable acids or cations;

- for use in narcosis;
- for use in the treatment of pain accompanying narcosis;
- for use in the treatment of neuropathic pain selected from the group consisting of chronic neuropathic pain, polyneuropathic pain and polyneuropathic pain induced by cytostatics.

2. Compounds for use in the treatment of chronic neuropathic pain according to claim 1, which are used at a dosage which is lower by a factor of at least 2 than the dosage used against acute pain.

3. Compounds for use according to claim 2, which are used at a dosage which is lower by a factor of at least 5 than the dosage used against acute pain.

4. Compounds for use according to claim 1, selected from the group consisting of
1,1-(3-methylamino-3-phenylpentamethylene)-6-fluoro-1,3,4,9-tetrahydropyrano[3,4b]indole hemicitrate;
1,1-(3-methylamino-3-phenylpentamethylene)-1,3,4,9-tetrahydropyrano[3,4-b]indole hemicitrate;

1,1-[3-dimethylamino-3-(3-thienyl)pentamethylene]-1,3,4,9-tetrahydropyrano[3,4-b]indole hemicitrate;
1,1-(3-dimethylamino-3-phenylpentamethylene)-6-fluoro-1,3,4,9-tetrahydropyrano[3,4-b]indole hemicitrate;
1,1-[3-methylamino-3-(2-thienyl)pentamethylene]-1,3,4,9-tetrahydropyrano[3,4-b]-6-fluoroindole citrate;
1,1-[3-dimethylamino-3-(2-thienyl)pentamethylene]-1,3,4,9-tetrahydropyrano[3,4-b]-6-fluoroindole hemicitrate;
1,1-[3-dimethylamino-3-(2-thienyl)pentamethylene]-1,3,4,9-tetrahydropyrano[3,4-b]indole citrate;
1,1-[3-dimethylamino-3-(3-thienyl)pentamethylene]-1,3,4,9-tetrahydropyrano[3,4-b]-6-fluoroindole hemicitrate;
1,1-(3-dimethylamino-3-phenylpentamethylene)-1,3,4,9-tetrahydropyrano[3,4-b]indole hemicitrate; and
1,1-[3-methylamino-3-(2-thienyl)pentamethylene]-1,3,4,9-tetrahydropyrano[3,4-b]indole citrate.

**5.** 1,1 -(3-methylamino-3-phenylpentamethylene)-6-fluoro-1,3,4,9-tetrahydropyrano[3,4b]indole in the form of a pure diastereomer or in the form of mixtures of the stereoisomers in any desired mixing ratio; as a base or in the form of a physiologically acceptable salt; for use in the treatment of neuropathic pain.

**6.** 1,1-(3-dimethylamino-3-phenylpentamethylene)-6-fluoro-1,3,4,9-tetrahydropyrano[3,4-b]indole in the form of a pure diastereomer or in the form of mixtures of the stereomers in any desired mixing ratio; as a base or in the form of a physiologically acceptable salt; for use in the treatment of neuropathic pain.

**Revendications**

**1.** Composés qui présentent une affinité pour le récepteur des μ-opioïdes d'au moins 100 nM (valeur $K_i$ humaine) et une affinité pour le récepteur ORL-1, le rapport entre les affinités pour ORL1/μ défini comme $1/[K_i(ORL1)/Ki(\mu)]$ étant de 0,1 à 30, et qui sont des dérivés spirocycliques du cyclohexane de formule générale (I)

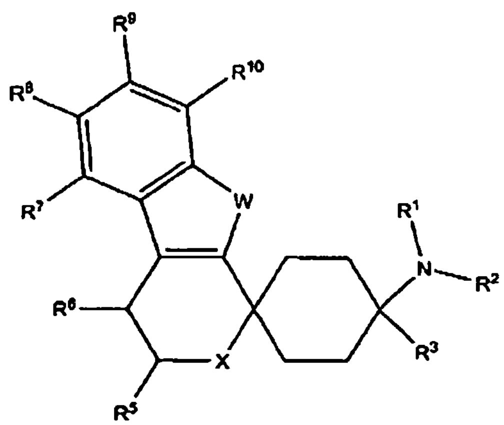

(I)

où
$R^1$ et $R^2$ représentent, indépendamment l'un de l'autre, H ou $CH_3$, où $R^1$ et $R^2$ ne signifient pas simultanément H ;
$R^3$ représente phényle, benzyle ou hétéroaryle, à chaque fois non substitué ou monosubstitué ou polysubstitué par F, Cl, OH, CN, ou $OCH_3$ ;
W représente $NR^9$, O ou S ; où
$R^4$ représente H ; $C_{1-5}$-alkylphényle ; phényle, $COR^{12}$ lié via un groupe $C_{1-3}$-alkyle ; $SO_2R^{12}$,
$R^{12}$ signifie H ; $C_{1-7}$-alkyle, ramifié ou non ramifié, saturé ou insaturé, non substitué ou monosubstitué ou polysubstitué par OH, F ou $COOC_{1-4}$-alkyle ; $C_{4-6}$-cycloalkyle ; aryle ou hétéroaryle, non substitué ou monosubstitué ou polysubstitué par F, Cl, Br, $CF_3$, $OCH_3$, $C_{1-4}$-alkyle, ramifié ou non ramifié, non substitué ou substitué par F, Cl, CN, $CF_3$, $OCH_3$ ou OH ; ou phényle ou hétéroaryle - lié via $C_{1-3}$-alkyle saturé ou insaturé - non substitué ou monosubstitué ou polysubstitué par F, Cl, Br, $CF_3$, $OCH_3$, $C_{1-9}$-alkyle, ramifié ou non ramifié, non substitué ou substitué par F, Cl, CN, $CF_3$, $OCH_3$ ou OH ; ou $C_{5-6}$-cycloalkyle lié via $C_{1-3}$-alkyle saturé ou insaturé ; $OR^{13}$ ; $NR^{14}R^{15}$ ;
$R^5$ représente H ; $COOR^{13}$, $CONR^{13}$, $OR^{13}$ ; $C_{1-5}$-alkyle, saturé ou insaturé, ramifié ou non ramifié, non substitué ou monosubstitué ou polysubstitué par OH, F, $CF_3$ ou CN ;
$R^6$ représente H ; ou
$R^5$ et $R^6$ signifient ensemble $(CH_2)_n$ avec n = 2, 3, 4, 5 ou 6, où différents atomes d'hydrogène peuvent également être remplacés par F, Cl, $NO_2$, $CF_3$, $OR^{13}$, CN ou $C_{1-5}$-alkyle ;

$R^7$, $R^8$, $R^9$ et $R^{10}$ représentent, indépendamment l'un de l'autre, H, F, Cl, Br, $NO_2$, $CF_3$, OH, $OCH_3$, CN, $COOR^{13}$, $NR^{14}R^{15}$ ; $C_{1-5}$-alkyle, hétéroaryle, non substitué, monosubstitué ou polysubstitué par benzyle, $CH_3$, Cl, F, $OCH_3$ ou OH ;

- où $R^{13}$ signifie H ou $C_{1-5}$-alkyle ;
- $R^{14}$ et $R^{15}$ signifient indépendamment l'un de l'autre, H ou $C_{1-5}$-alkyle ;

X représente O, S, SO, $SO_2$ ou $NR^{17}$ ;
$R^{17}$ représente H ; $C_{1-5}$-alkyle, saturé ou insaturé, ramifié ou non ramifié ; $COR^{12}$ ou $SO_2R^{12}$ ;
sous forme de leurs diastéréo-isomères purs, leurs racémates, leurs énantiomères purs ou sous forme de mélanges des stéréo-isomères dans un rapport de mélange quelconque ; comme bases ou sous forme de leurs sels, en particulier les sels physiologiquement acceptables ou les sels d'acides ou de cations physiologiquement acceptables ;

- destinés à une utilisation lors d'une narcose ;
- destinés à une utilisation lors du traitement de la douleur accompagnant une narcose ;
- destinés à une utilisation lors du traitement de la douleur neuropathique choisie dans le groupe constitué par la douleur neuropathique chronique, la douleur polyneuropathique et la douleur polyneuropathique induite par les cytostatiques.

**2.** Composés destinés à une utilisation lors du traitement de la douleur neuropathique chronique selon la revendication 1, qui sont utilisés en un dosage qui est inférieur d'au moins un facteur 2 au dosage utilisé en cas de douleur aiguë.

**3.** Composés destinés à une utilisation selon la revendication 2, qui sont utilisés en un dosage qui est inférieur d'au moins un facteur 5 au dosage utilisé en cas de douleur aiguë.

**4.** Composés destinés à une utilisation selon la revendication 1, choisis dans le groupe constitué par
l'hémi-citrate de 1,1-(3-méthylamino-3-phénylpentaméthylène)-6-fluoro-1,3,4,9-tétrahydropyrano[3,4-b]indole ;
l'hémi-citrate de 1,1-(3-méthylamino-3-phénylpentaméthylène)-1,3,4,9-tétrahydropyrano[3,4-b]indole ;
l'hémi-citrate de 1,1-[3-diméthylamino-3-(3-thiényl)pentaméthylène]-1,3,4,9-tétrahydropyrano[3,4-b]indole ;
l'hémi-citrate de 1,1-(3-diméthylamino-3-phénylpentaméthylène)-6-fluoro-1,3,4,9-tétrahydropyrano[3,4-b]indole ;
le citrate de 1,1-[3-méthylamino-3-(2-thiényl)pentaméthylène]-1,3,4,9-tétrahydropyrano[3,4-b]6-fluoro-indole ;
l'hémi-citrate de 1,1-[3-diméthylamino-3-(2-thiényl)pentaméthylène]-1,3,4,9-tétrahydropyrano[3,4-b]-6-fluoro-indole ;
le citrate de 1,1-[3-diméthylamino-3-(2-thiényl)pentaméthylène]-1,3,4,9-tétrahydropyrano[3,4-b]indole ;
l'hémi-citrate de 1,1-[3-diméthylamino-3-(3-thiényl)pentaméthylène]-1,3,4,9-tétrahydropyrano[3,4-b]-6-fluoro-indole ;
l'hémi-citrate de 1,1-(3-diméthylamino-3-phénylpentaméthylène)-1,3,4,9-tétrahydropyrano[3,4-b]indole ; et
le citrate de 1,1-[3-méthylamino-3-(2-thiényl)pentaméthylène]-1,3,4,9-tétrahydropyrano[3,4-b]indole.

**5.** 1,1-(3-méthylamino-3-phénylpentaméthylène)-6-fluoro-1,3,4,9-tétrahydropyrano[3,4-b]indole sous forme d'un diastéréo-isomère pur ou sous forme de mélanges des stéréo-isomères dans un rapport de mélange quelconque ; comme base ou sous forme d'un sel physiologiquement acceptable, destiné à une utilisation lors du traitement de la douleur neuropathique.

**6.** 1,1-(3-diméthylamino-3-phénylpentaméthylène)-6-fluoro-1,3,4,9-tétrahydropyrano[3,4-b]indole sous forme d'un diastéréo-isomère pur ou sous forme de mélanges des stéréo-isomères dans un rapport de mélange quelconque ; comme base ou sous forme d'un sel physiologiquement acceptable ; destiné à une utilisation lors du traitement de la douleur neuropathique.

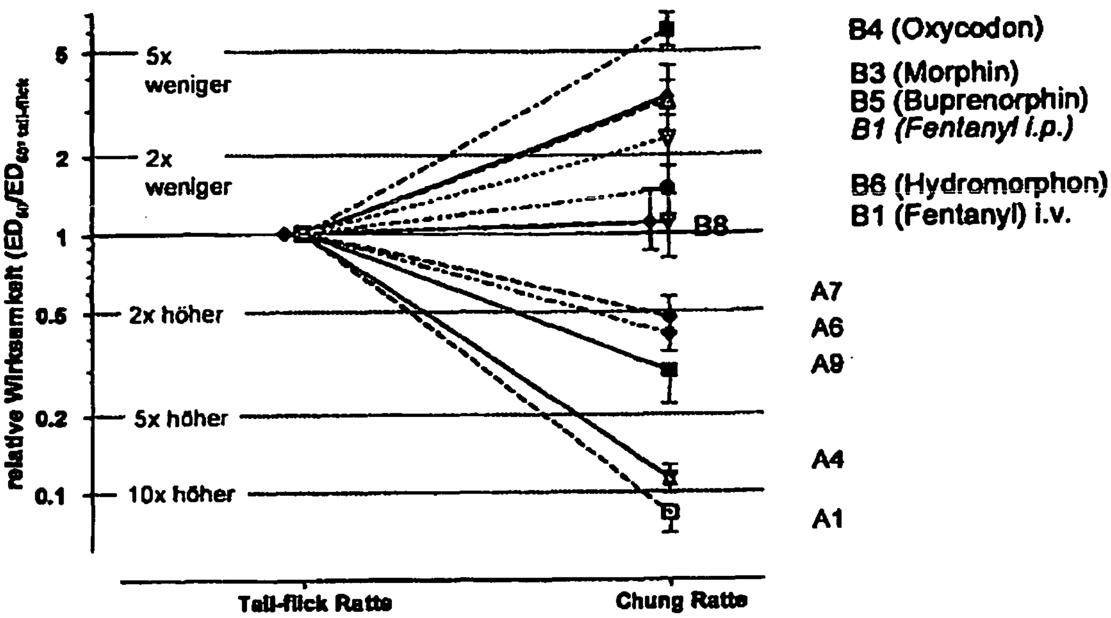


**Abbildung 1**: Analgetische Wirksamkeit im Akutschmerz und im neuropathischen Schmerz, Chung-Modell

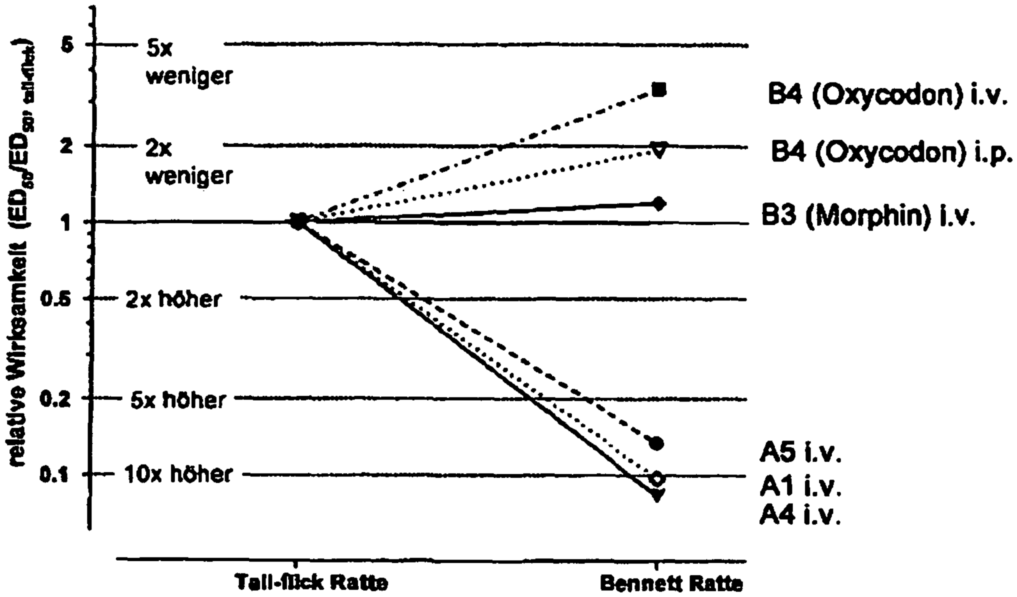


**Abbildung 2:** Vergleich der analgetischen Wirksamkeit im Akutschmerz und im neuropathischen Schmerz, Bennett-Modell

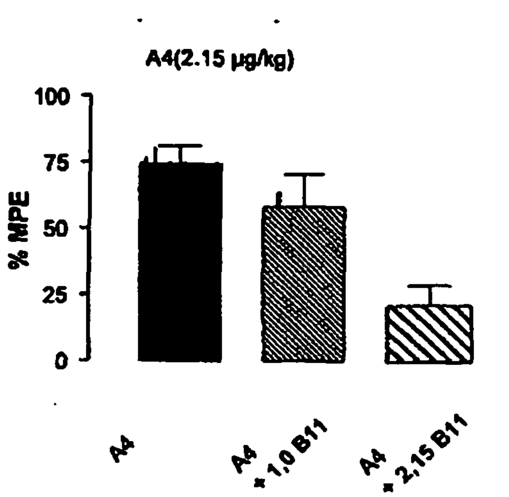


**Abbildung 3:** Antagonisierung des antinociceptiven Effekts durch B11, Chung-Modell 30 min (Dosierungsangaben B11 in mg/kg).

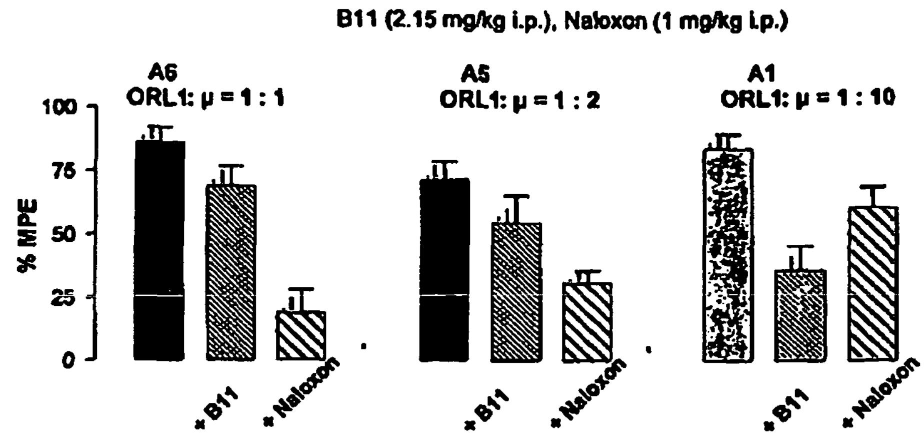


**Abbildung 4**: Antagonisierung des antinociceptiven Effekts durch B11 bzw. Naloxon, Chung-Modell 30 min

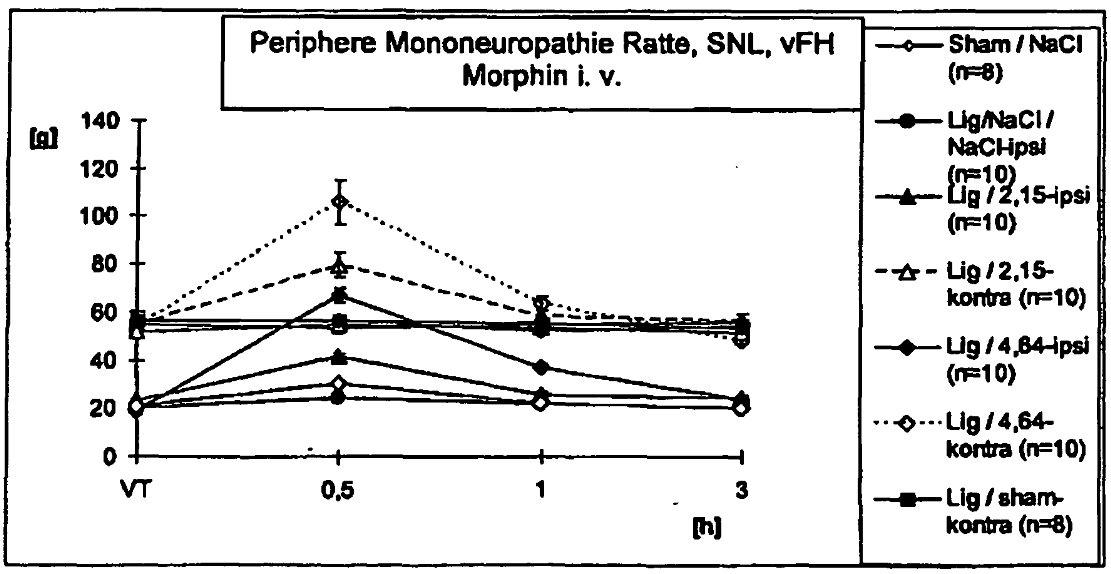


**Abbildung 5**: Trennung von antinociceptivem und antiallodynischem Effekt bei neuropathischen Tieren, Morphin

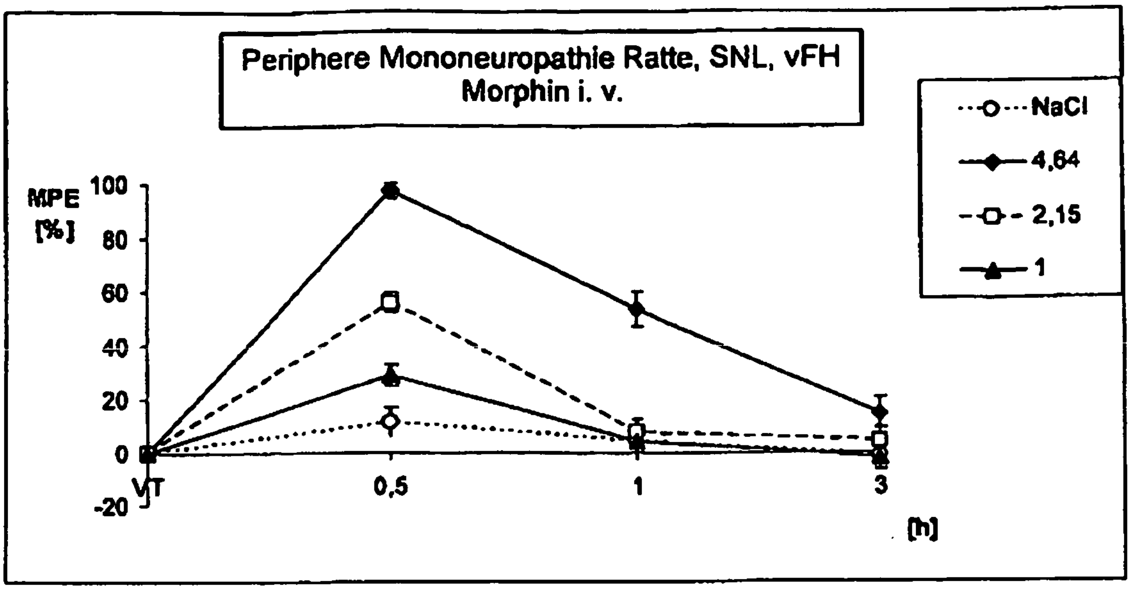


**Abbildung 5a**: Trennung von antinociceptivem und antiallodynischem Effekt bei neuropathischen Tieren, Morphin

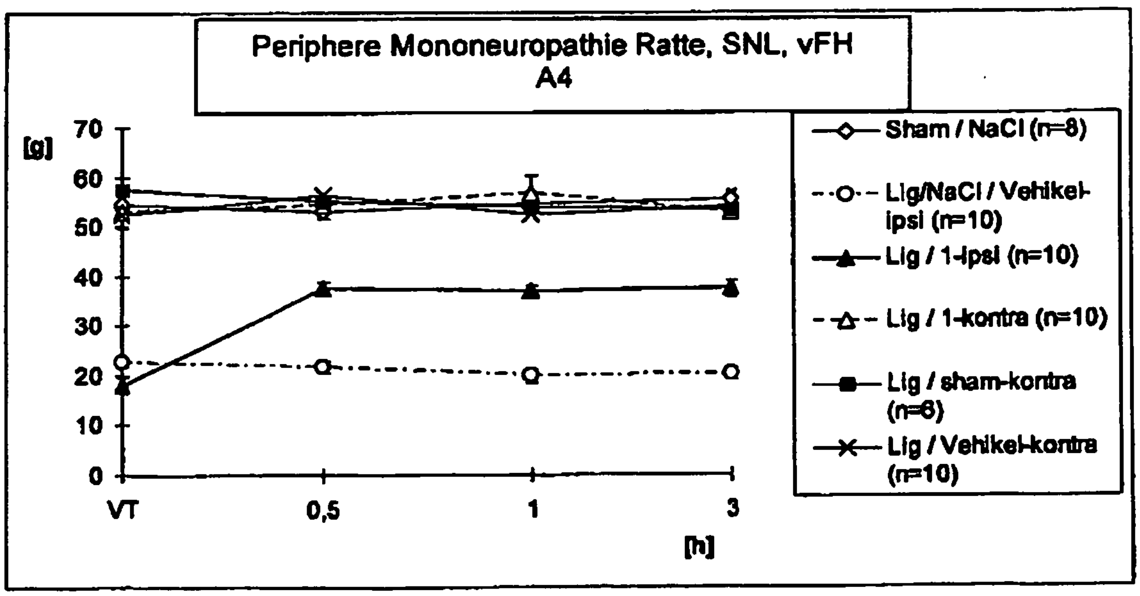


**Abbildung 6**: Trennung von antinociceptivem und antiallodynischem Effekt bei neuropathischen Tieren, A4

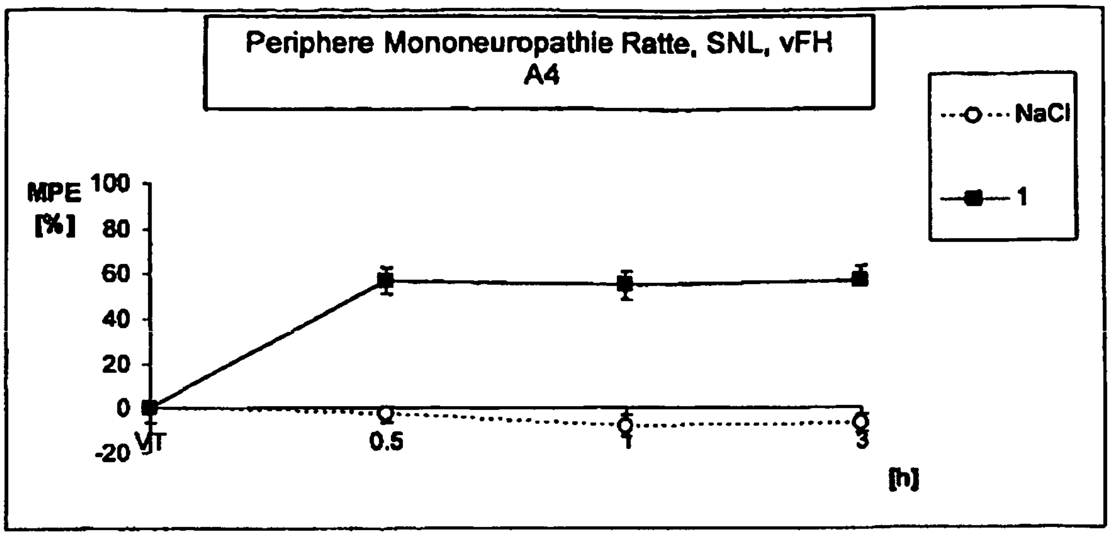


**Abbildung 6a**: Trennung von antinociceptivem und antiallodynischem Effekt bei neuropathischen Tieren, A4

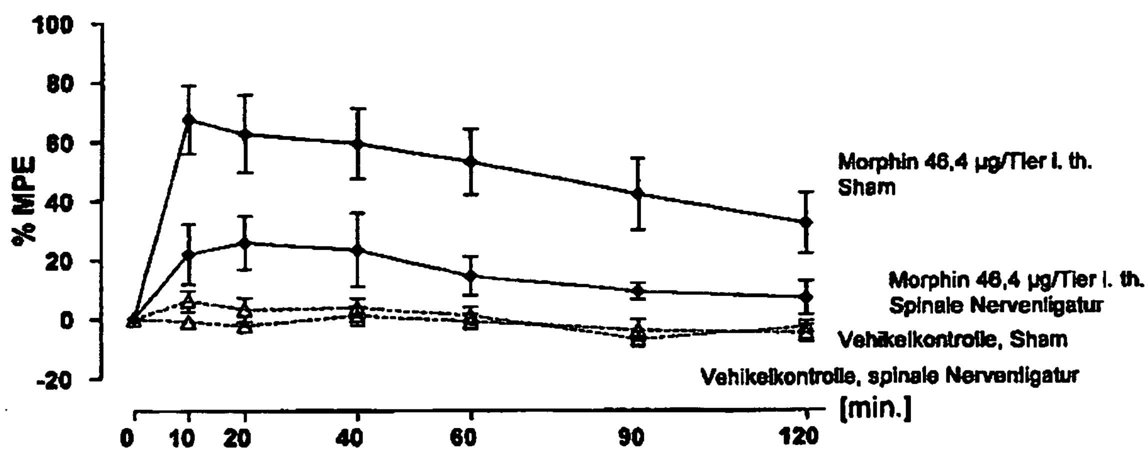


**Abbildung 7**: Morphin in naiven und neuropathischen Tieren, Vergleich

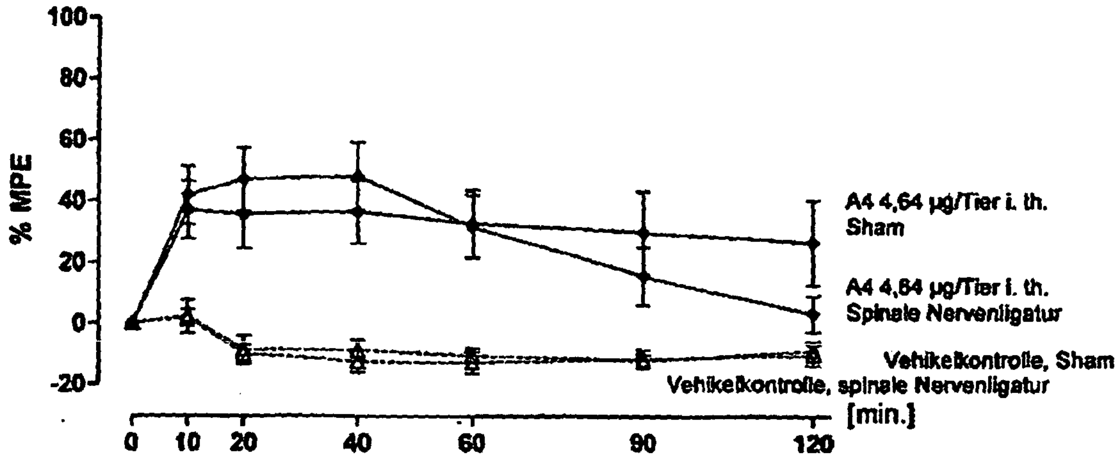


Abbildung 8: A4 in naiven und neuropathischen Tieren, Vergleich

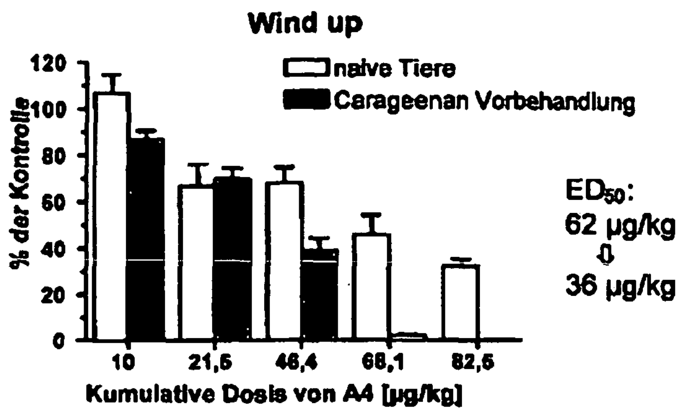


**Abbildung 9**: Entzündungsschmerz: Single-Motor-Unit Ableitungen in spinalisierten Ratten, Vergleich naive Tiere und Carrageenan-vorbehandelte Tiere, A4

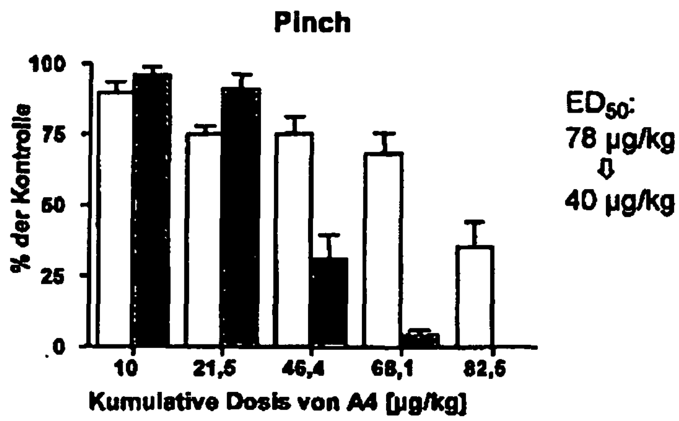


**Abbildung 10**: Entzündungsschmerz: Single-Motor-Unit Ableitungen in spinalisierten Ratten, Vergleich naive Tiere und Carrageenan-vorbehandelte Tiere, A4

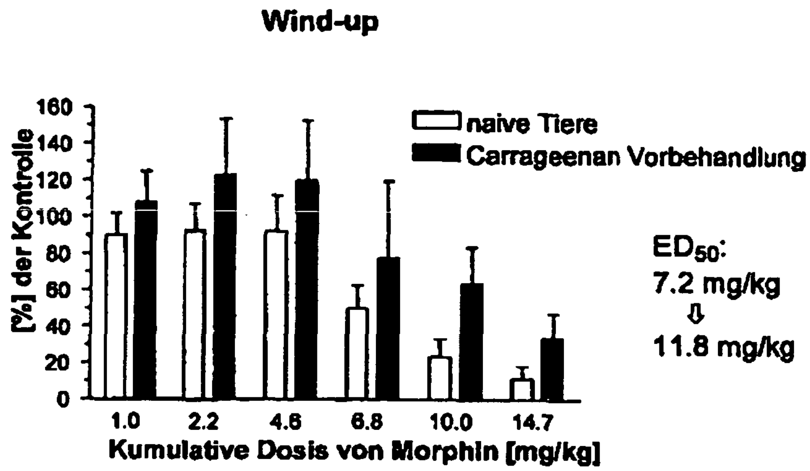


**Abbildung 11**: Entzündungsschmerz: Single-Motor-Unit Ableitungen in spinalisierten Ratten, Vergleich naive Tiere und Carrageenan-vorbehandelte Tiere, Morphin

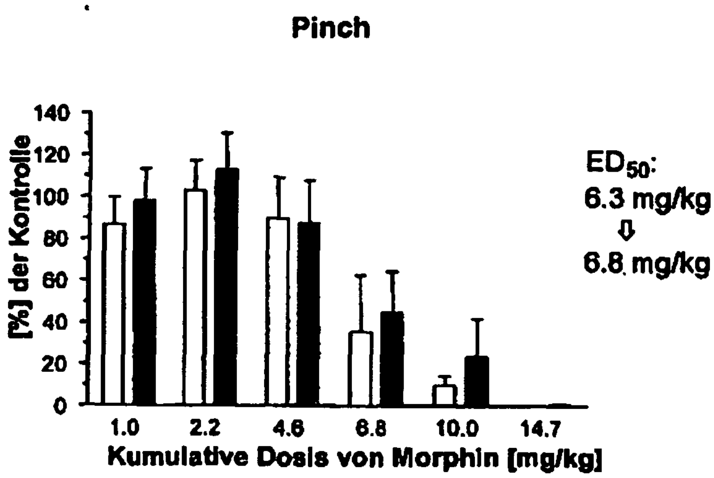


**Abbildung 11a**: Entzündungsschmerz: Single-Motor-Unit Ableitungen in spinalisierten Ratten, Vergleich naive Tiere und Carrageenan-vorbehandelte Tiere, Morphin

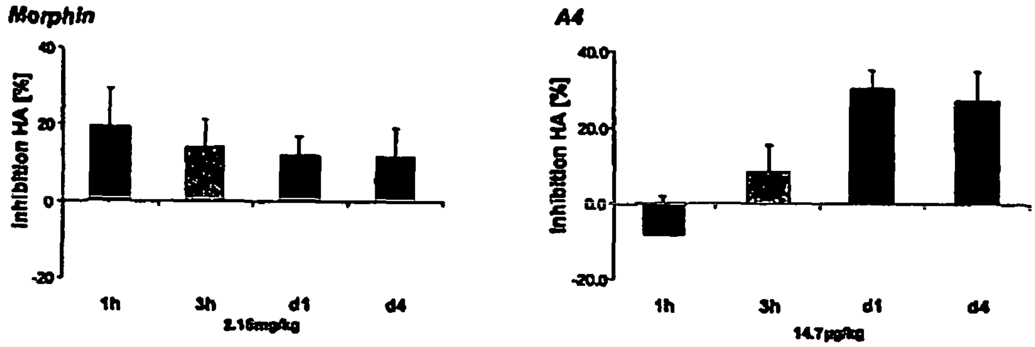


**Abbildung 12:** Ratte CFA-induzierte Hyperalgesie: Bestimmung des antinociceptiven (inkl. anti-hyperalgetischen) Effektes (Zeitabhängigkeit – 1 h bis 4 Tage nach CFA-Gabe)

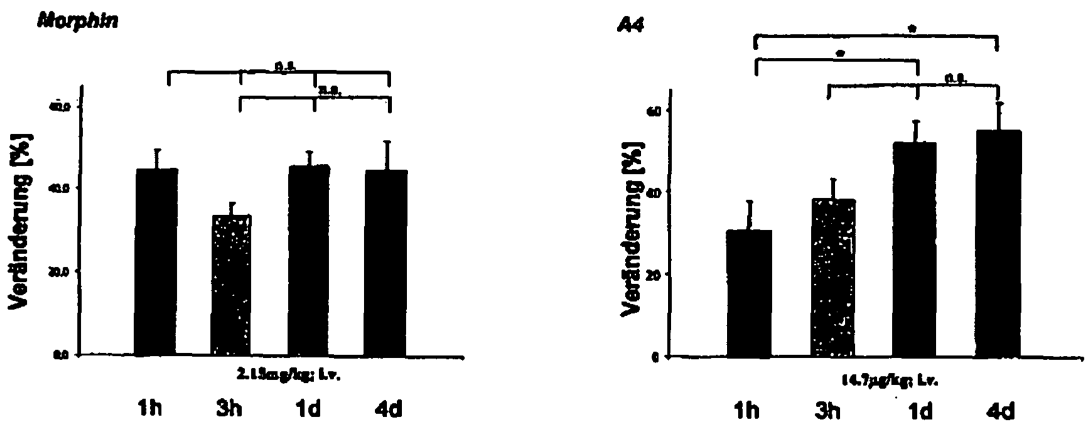


**Abbildung 12a:** Veränderung des antinociceptiven (inkl. anti-hyperalgetischen) Effektes

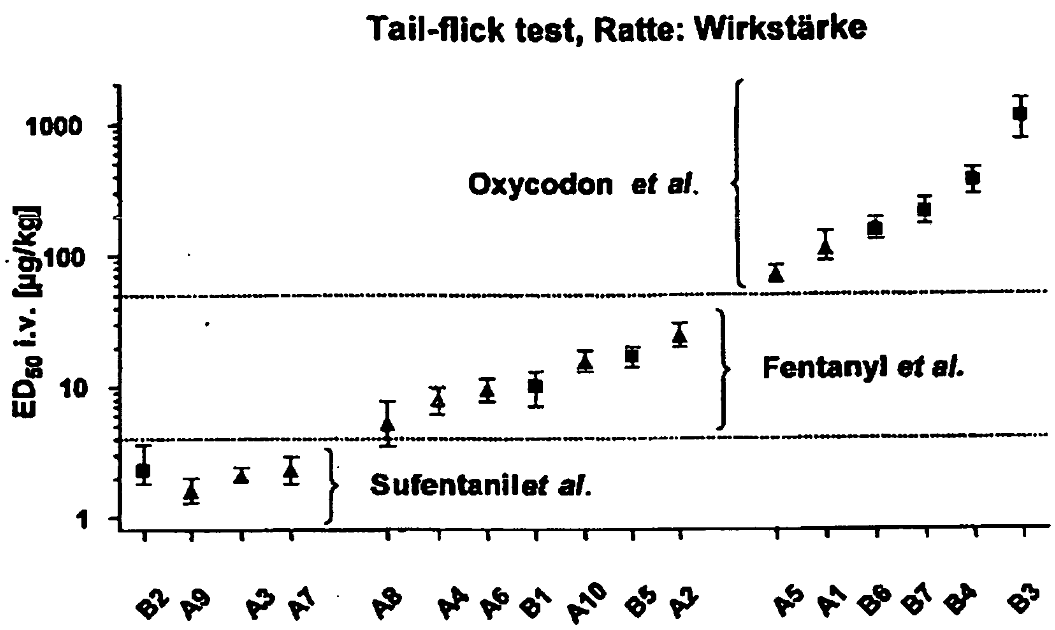


**Abbildung 13:** Vergleich der halbmaximal wirksamen Dosierungen von gemischten ORL1/µ-Agonisten und Standardopioiden nach i.v. Bolusgabe in einem Nager-Akutschmerzmodell (Tail-flick, Ratte)

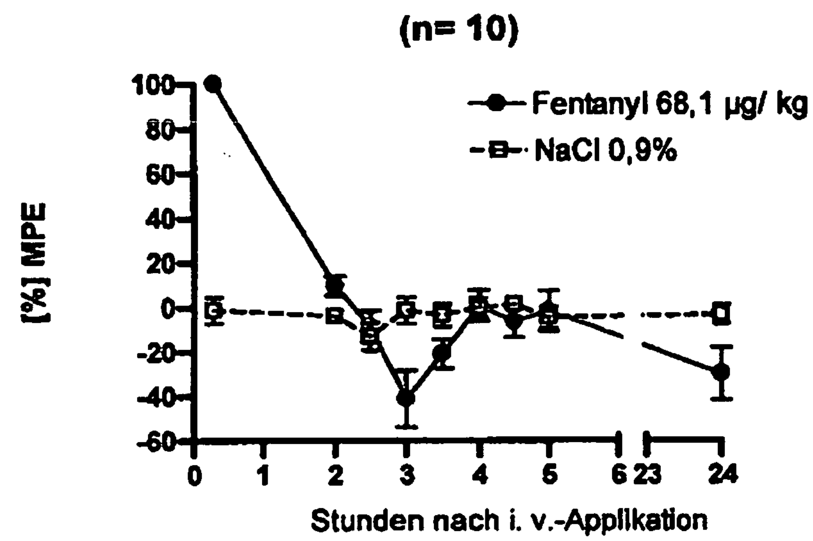


**Abbildung 14**: Auftreten von transienter Hyperalgesie nach Gabe von Fentanyl

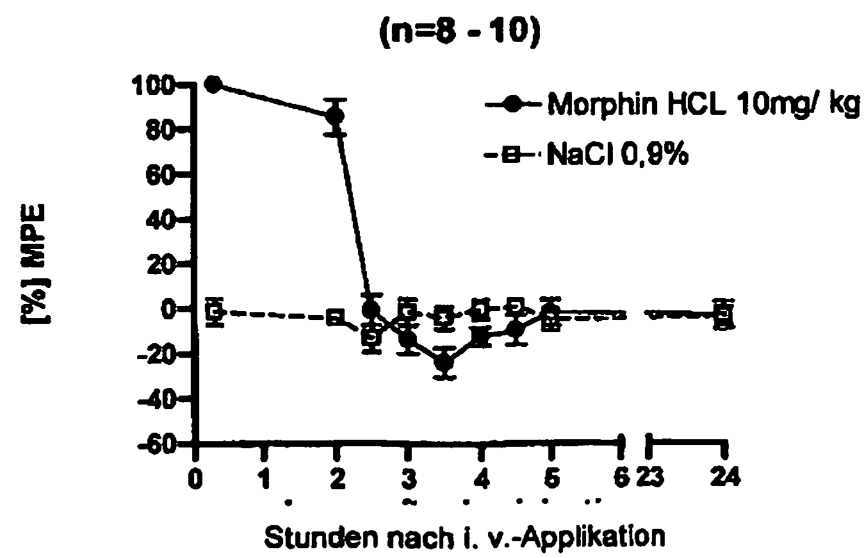


**Abbildung 14a**: Auftreten von transienter Hyperalgesie nach Gabe von Morphin

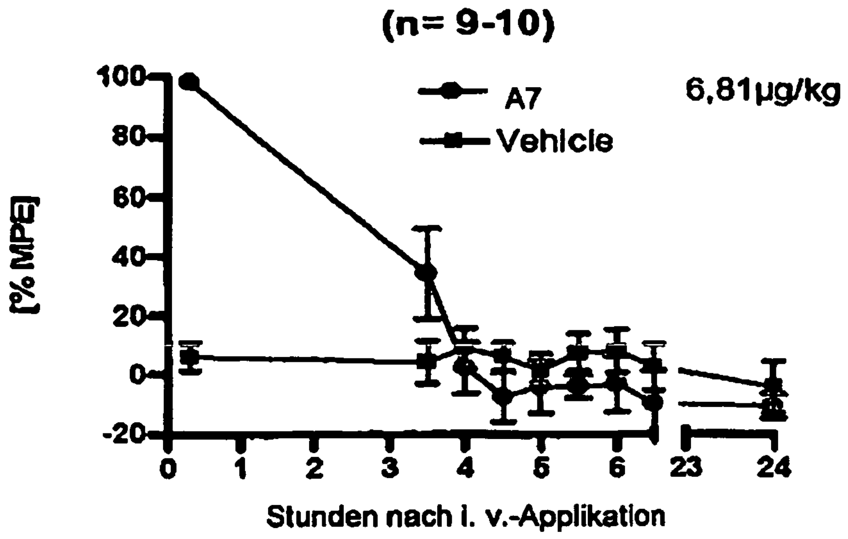


**Abbildung 14b:** Auftreten von transienter Hyperalgesie nach Gabe von A7

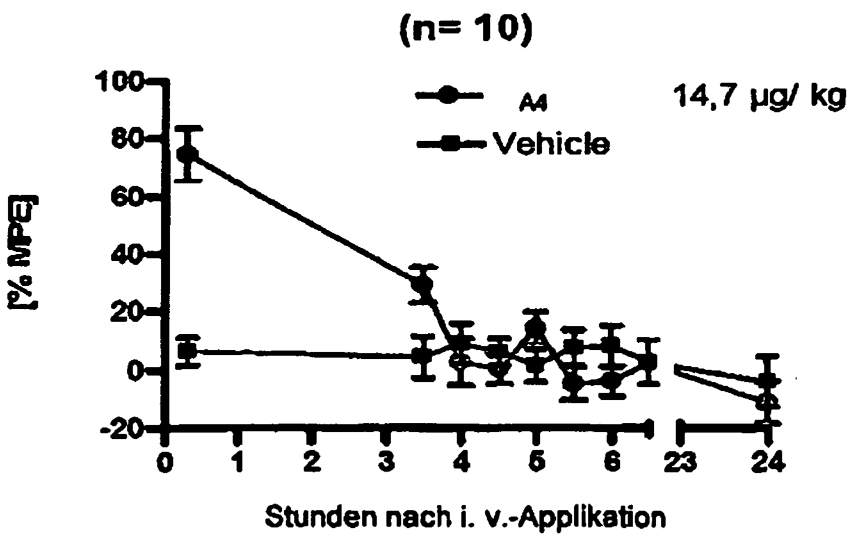


**Abbildung 14c:** Auftreten von transienter Hyperalgesie nach Gabe von A4

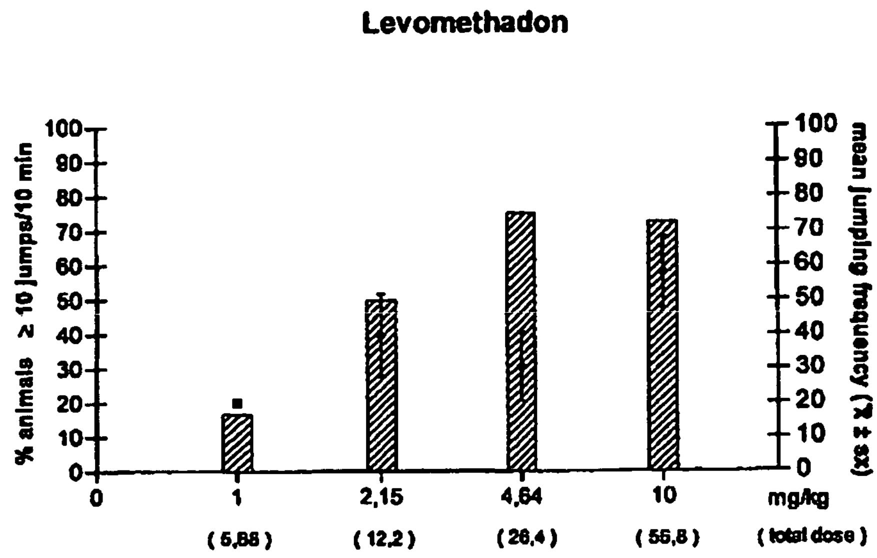


**Abbildung 15**: Entzugsspringen nach Applikation von Levomethadon

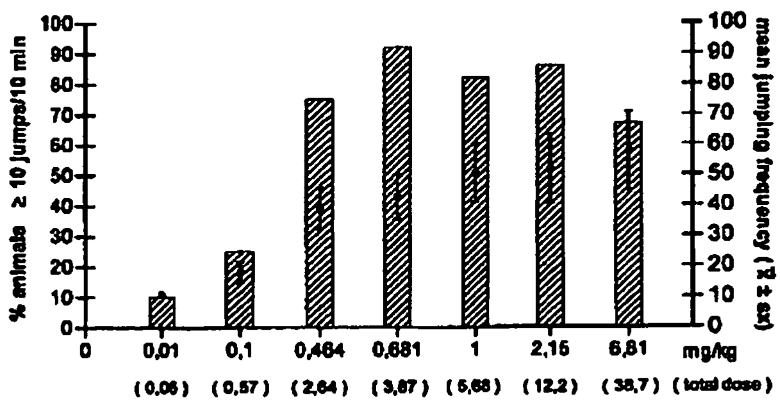


**Abbildung 15a**: Entzugsspringen nach Applikation von B8

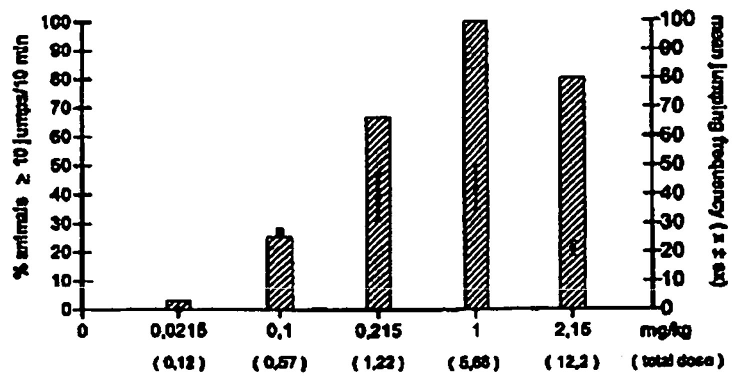


**Abbildung 15b**: Entzugsspringen nach Applikation von A1

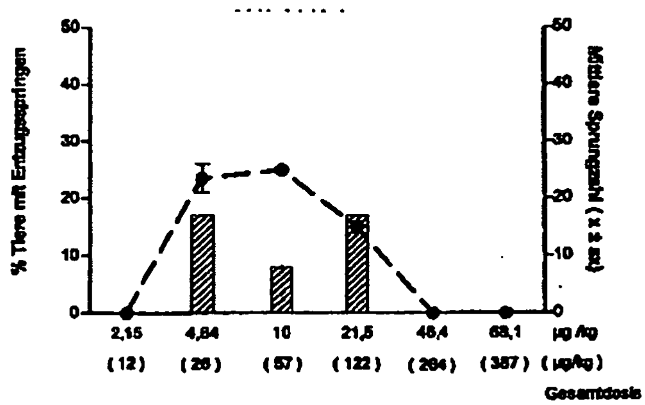


**Abbildung 15c**: Entzugsspringen nach Applikation von A9

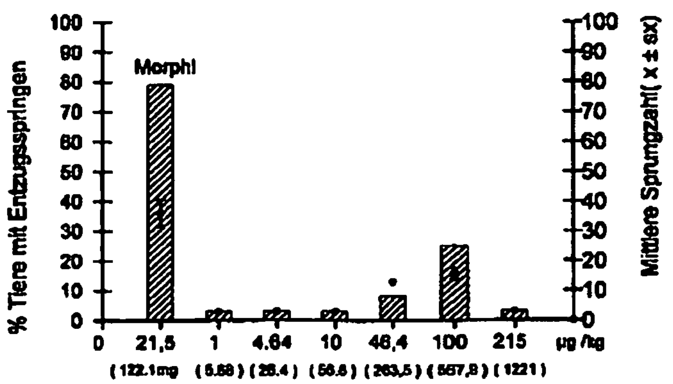


**Abbildung 15d**: Entzugsspringen nach Applikation von A4 mit Morphin als Vergleichssubstanz

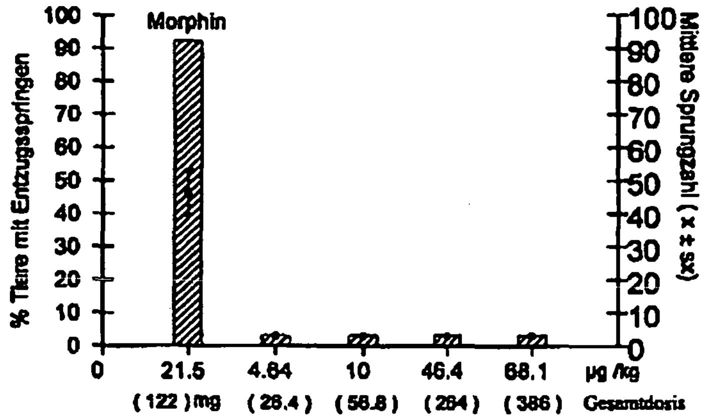


**Abbildung 15e**: Entzugsspringen nach Applikation von A7 mit Morphin als Vergleichssubstanz

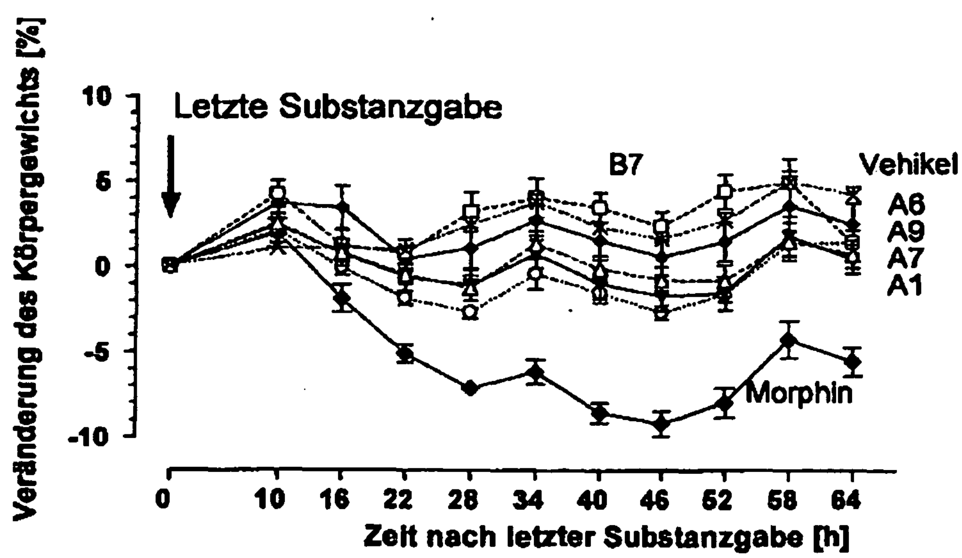


**Abbildung 16**: Spontanentzug

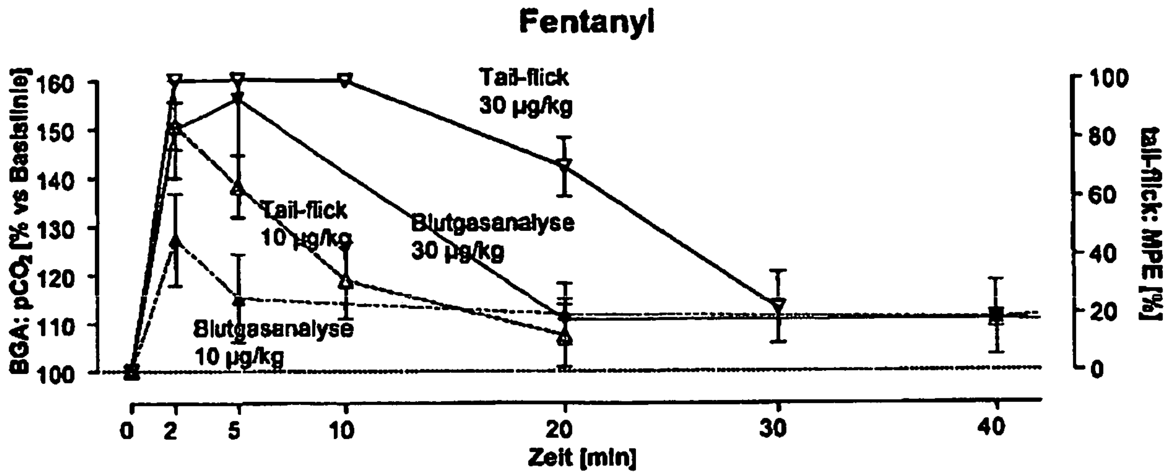


**Abbildung 17**: Zeitverlauf für die analgetische Wirkung im Tail-flick Test und vergleichend dazu der Zeitverlauf des arteriellen $pCO_2$ für jeweils eine analgetisch voll wirksame Dosierung und die analgetische Schwellendosierung (Applikation jeweils Bolus i. v.).

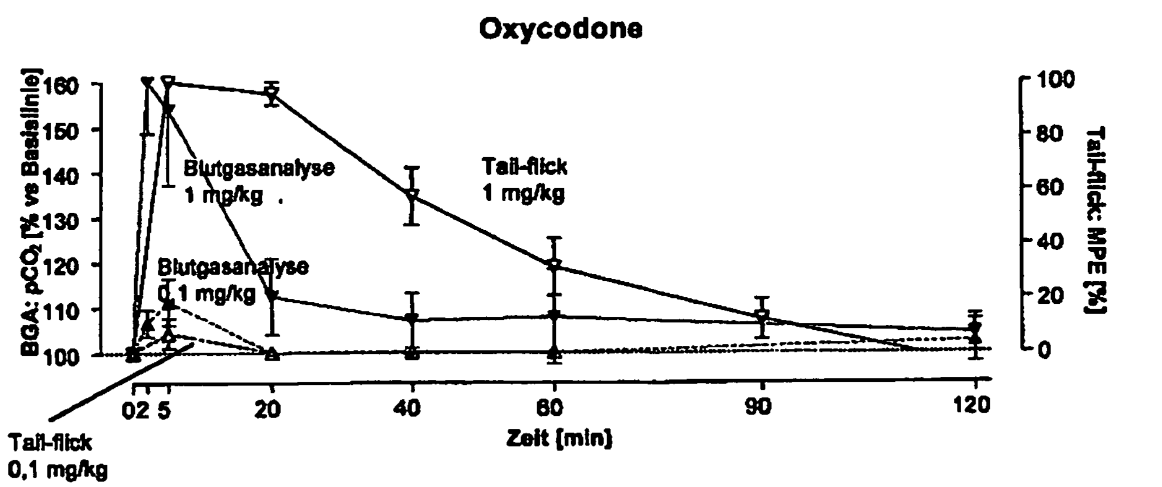


**Abbildung 17a**: Zeitverlauf für die analgetische Wirkung im Tail-flick Test und vergleichend dazu der Zeitverlauf des arteriellen $pCO_2$ für jeweils eine analgetisch voll wirksame Dosierung und die analgetische Schwellendosierung (Applikation jeweils Bolus i. v.).

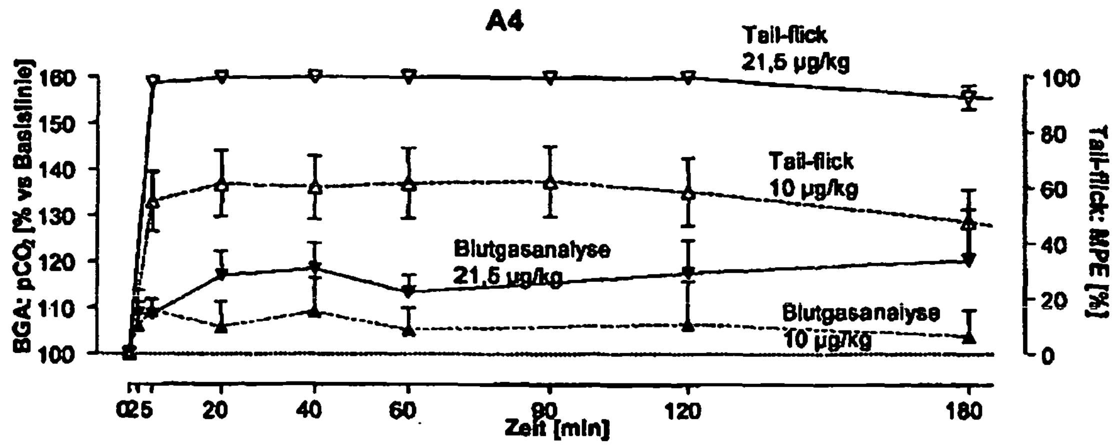


**Abbildung 17b**: Zeitverlauf für die analgetische Wirkung im Tail-flick Test und vergleichend dazu der Zeitverlauf des arteriellen $pCO_2$ für jeweils eine analgetisch voll wirksame Dosierung und die analgetische Schwellendosierung (Applikation jeweils Bolus i. v.).

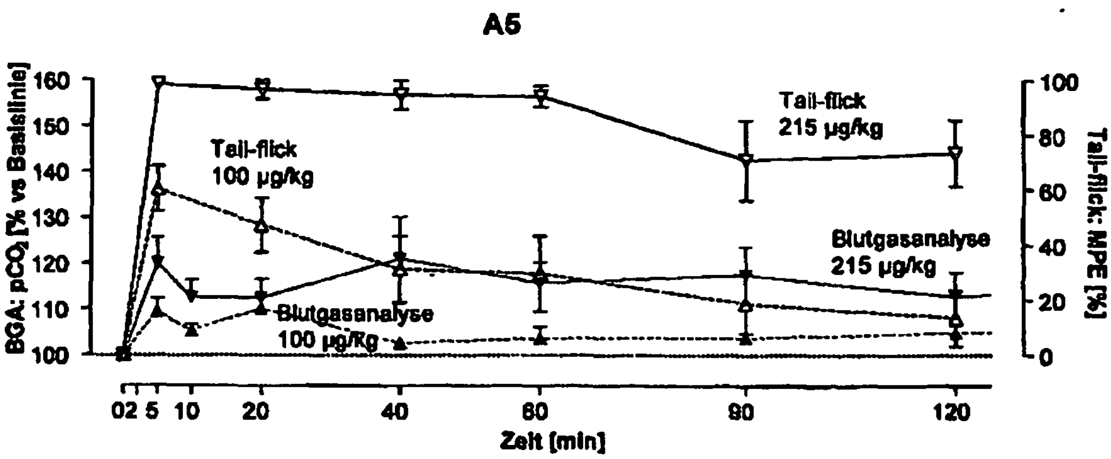


**Abbildung 17c**: Zeitverlauf für die analgetische Wirkung im Tail-flick Test und vergleichend dazu der Zeitverlauf des arteriellen $pCO_2$ für jeweils eine analgetisch voll wirksame Dosierung und die analgetische Schwellendosierung (Applikation jeweils Bolus i. v.).

**A6**

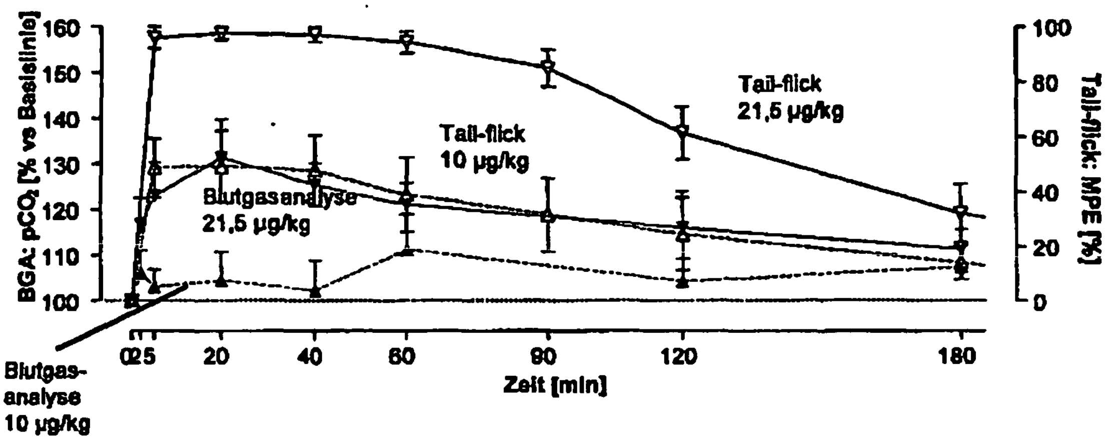


**Abbildung 17d**: Zeitverlauf für die analgetische Wirkung im Tail-flick Test und vergleichend dazu der Zeitverlauf des arteriellen $pCO_2$ für jeweils eine analgetisch voll wirksame Dosierung und die analgetische Schwellendosierung (Applikation jeweils Bolus i. v.).

**A9**

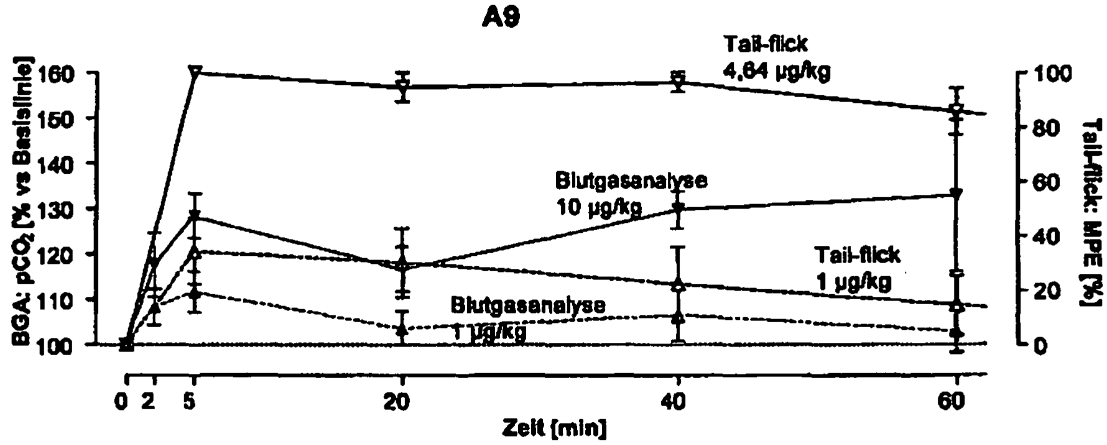


**Abbildung 17e**: Zeitverlauf für die analgetische Wirkung im Tail-flick Test und vergleichend dazu der Zeitverlauf des arteriellen $pCO_2$ für jeweils eine analgetisch voll wirksame Dosierung und die analgetische Schwellendosierung (Applikation jeweils Bolus i. v.).

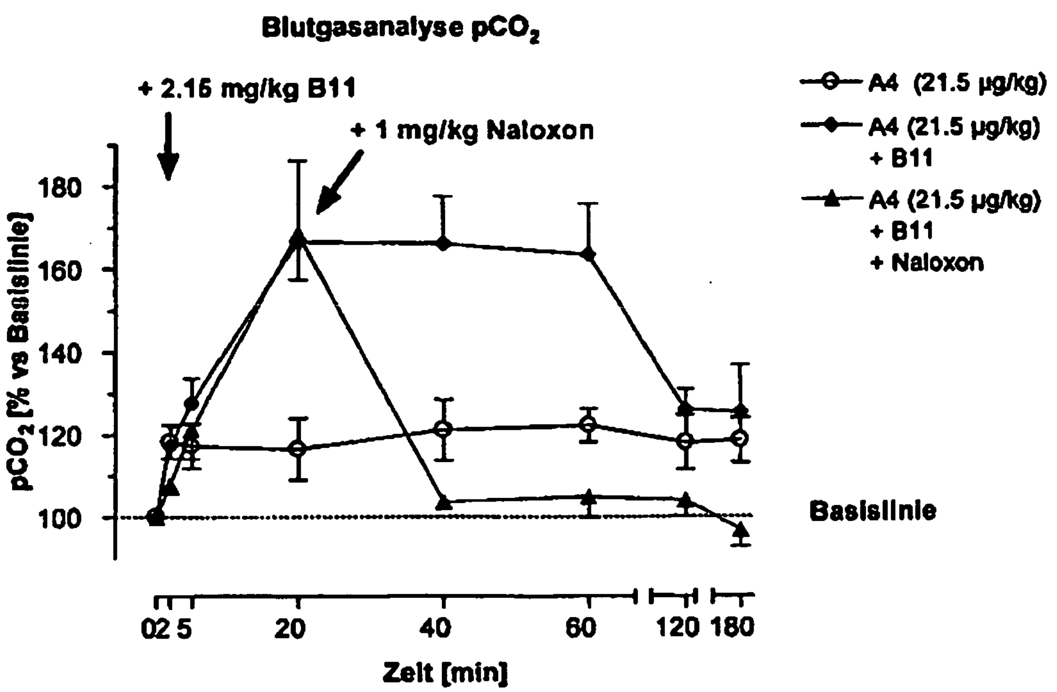


**Abbildung 18**: Nachweis des positiven Effekts der gemischten ORL1/µ-Agonisten auf die Atemdepression anhand von Antagonisierungsexperimenten

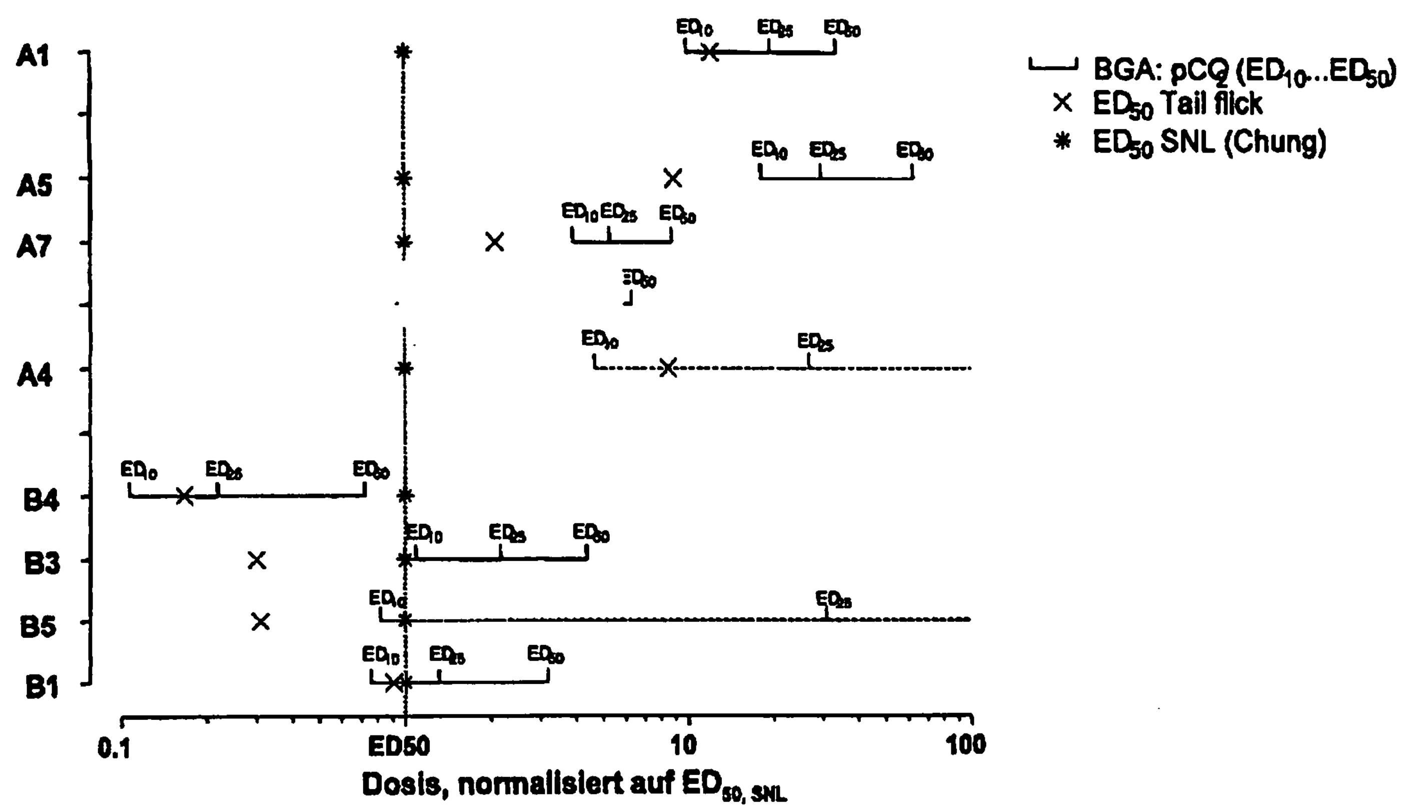


**Abbildung 20**: Abstände zwischen Analgesie und Nebenwirkung am Beispiel der Atemdepression für reine µ-Opioide und gemischte ORL1/µ-Agonisten im Vergleich

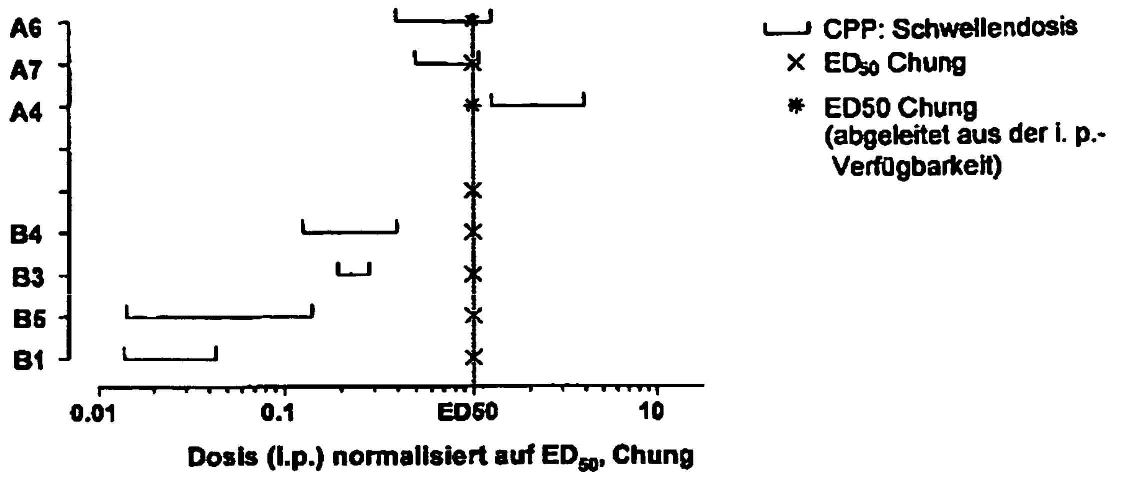


**Abbildung 21**: Abstände zwischen Analgesie und Nebenwirkung am Beispiel der psychischen Abhängigkeit für reine µ-Opioide und gemischte ORL1/µ-Agonisten im Vergleich

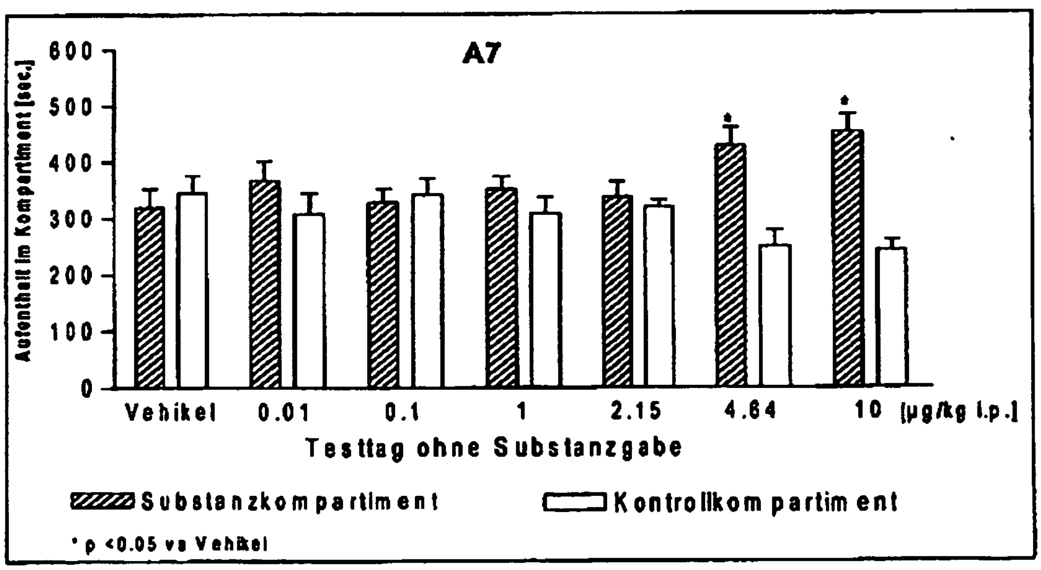


**Abbildung 22**: Beobachtete Platzpräferenz nach Gabe von A7

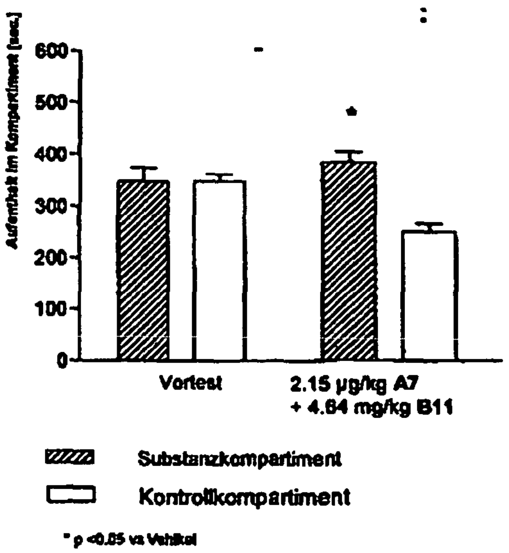


**Abbildung 23**: Verstärkung der Platzpräferenz nach Antagonisierung der ORL1-Komponente

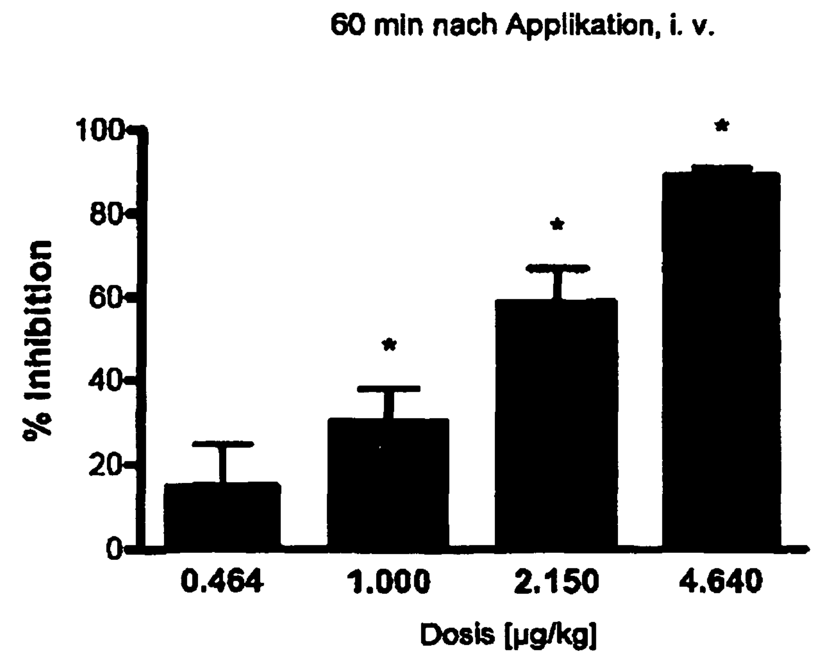


Abbildung 24: Cytostatika-induzierter Polyneuropathieschmerz, A4

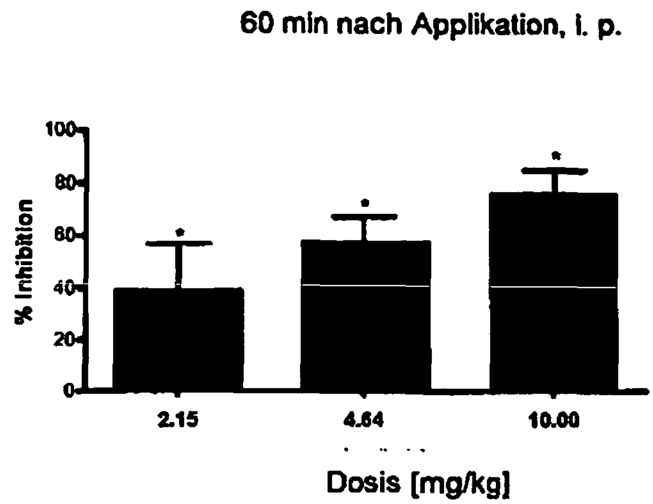


Abbildung 25: Cytostatika-induzierter Polyneuropathieschmerz, Morphin

a)

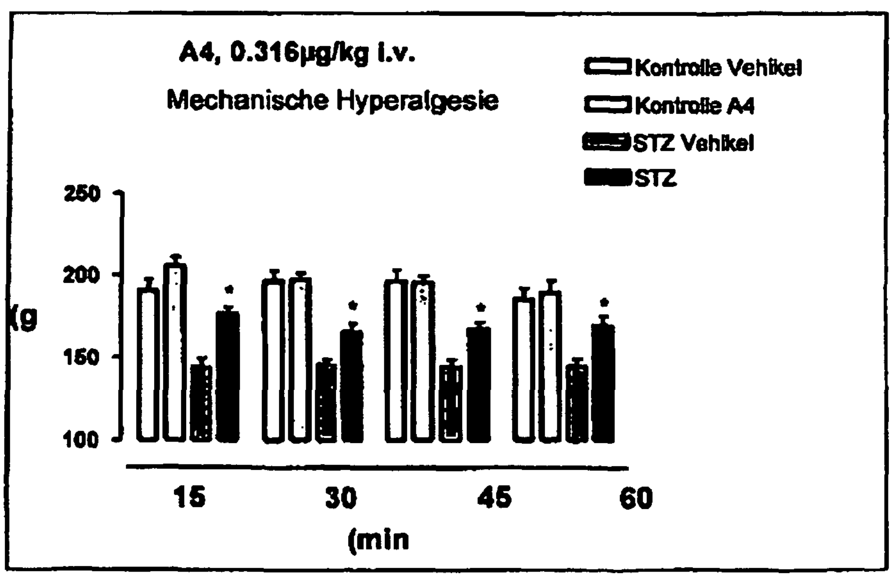

b)

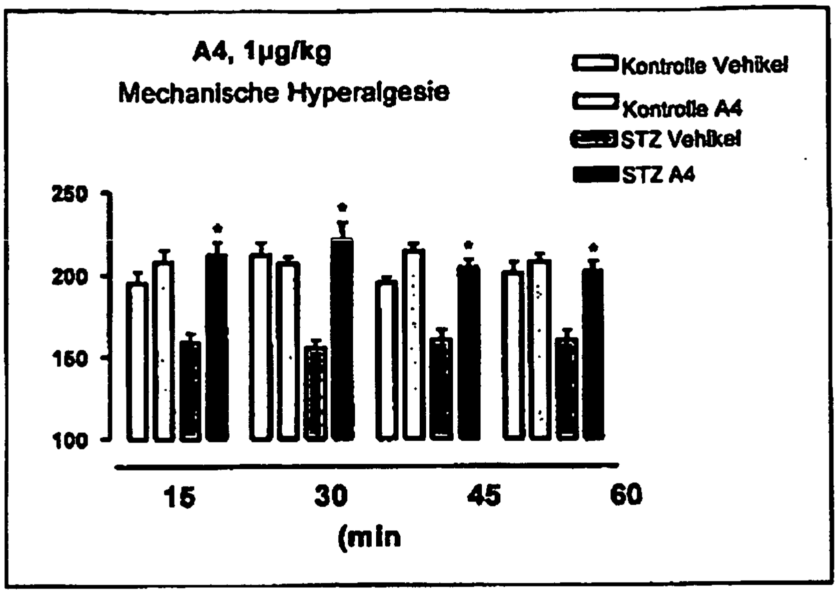


c)

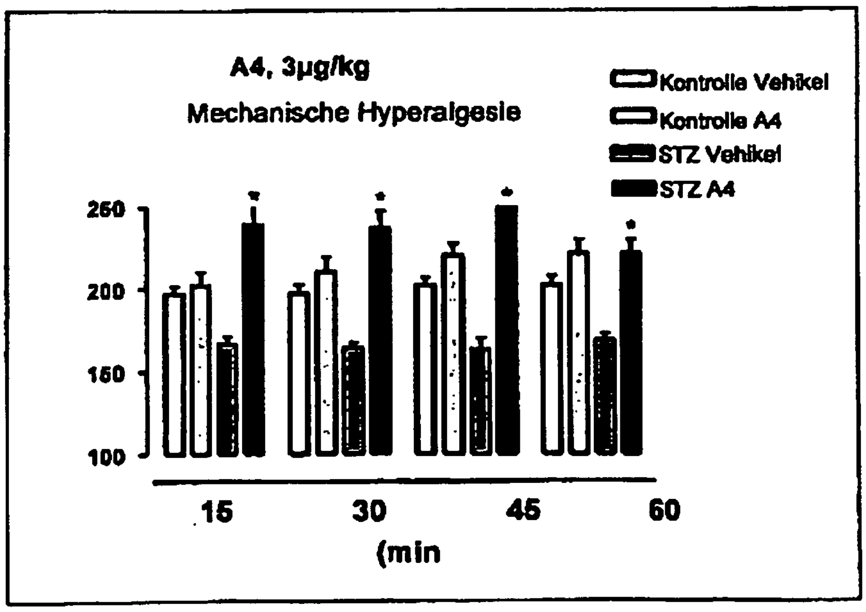


Abbildung 26: STZ-induzierte Polyneuropathieschmerz, A4, drei Dosierungen

a)

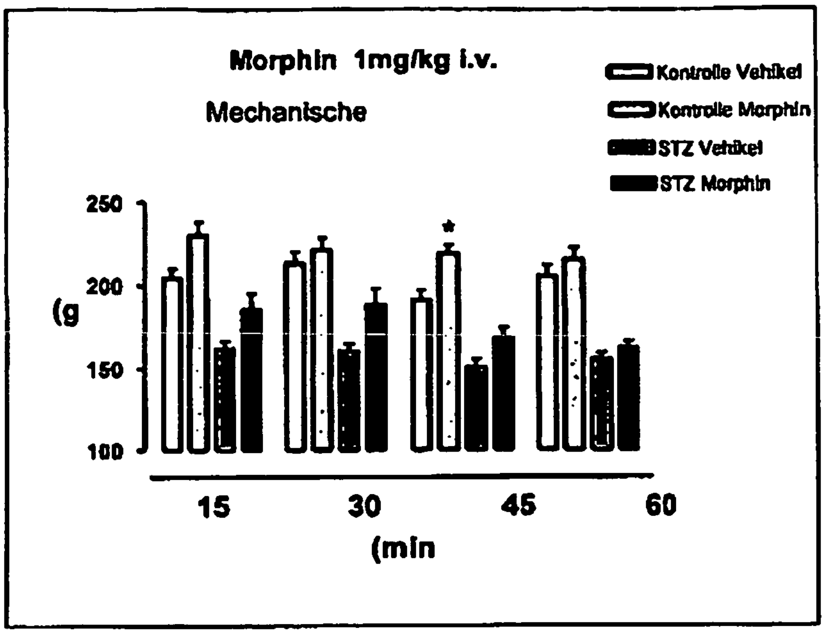


b)

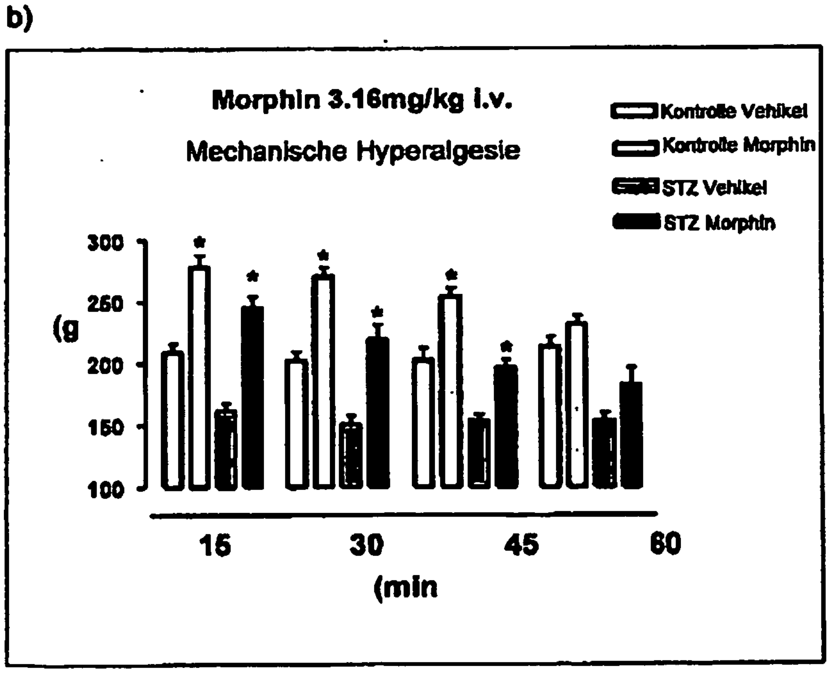


Abbildung 27: STZ-induzierte Polyneuropathieschmerz, Morphin, zwei Dosierungen

a)

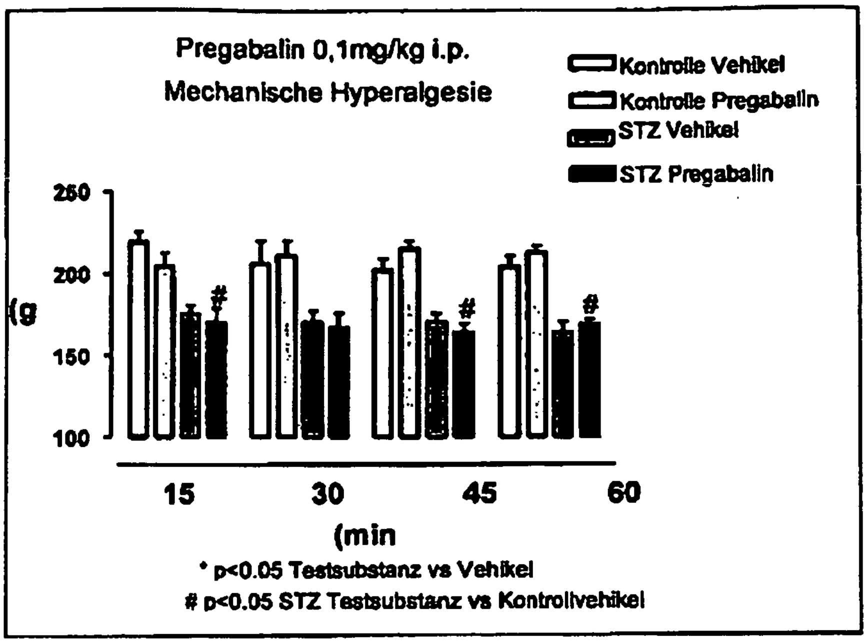
Pregabalin 0,1mg/kg i.p.
Mechanische Hyperalgesie
Kontrolle Vehikel
Kontrolle Pregabalin
STZ Vehikel
STZ Pregabalin
250
200
150
100
(g
#
#
#
15
30
45
60
(min
* p<0.05 Testsubstanz vs Vehikel
# p<0.05 STZ Testsubstanz vs Kontrollvehikel

b)

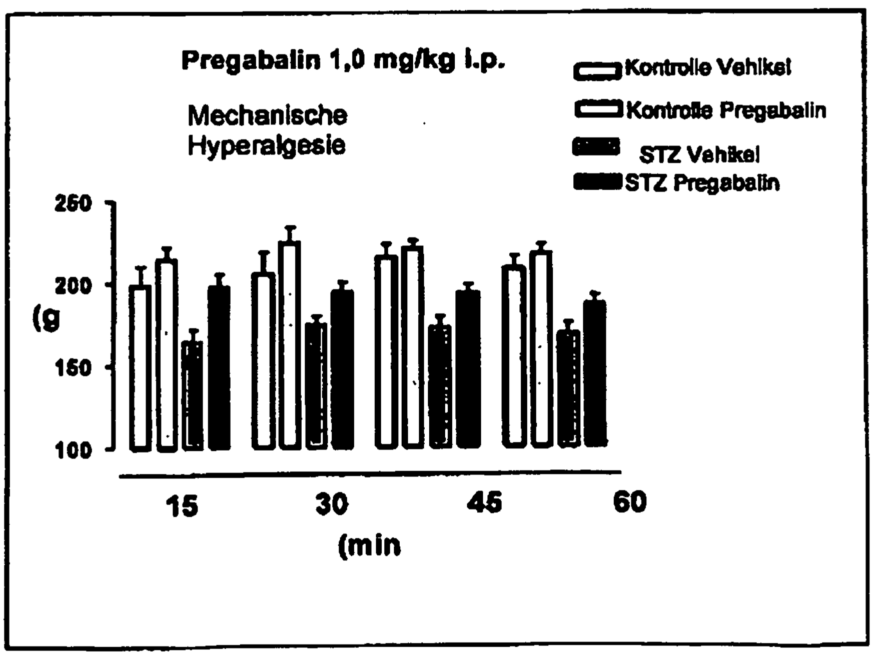
Pregabalin 1,0 mg/kg i.p.
Mechanische Hyperalgesie
Kontrolle Vehikel
Kontrolle Pregabalin
STZ Vehikel
STZ Pregabalin
250
200
150
100
(g
15
30
45
60
(min

c)

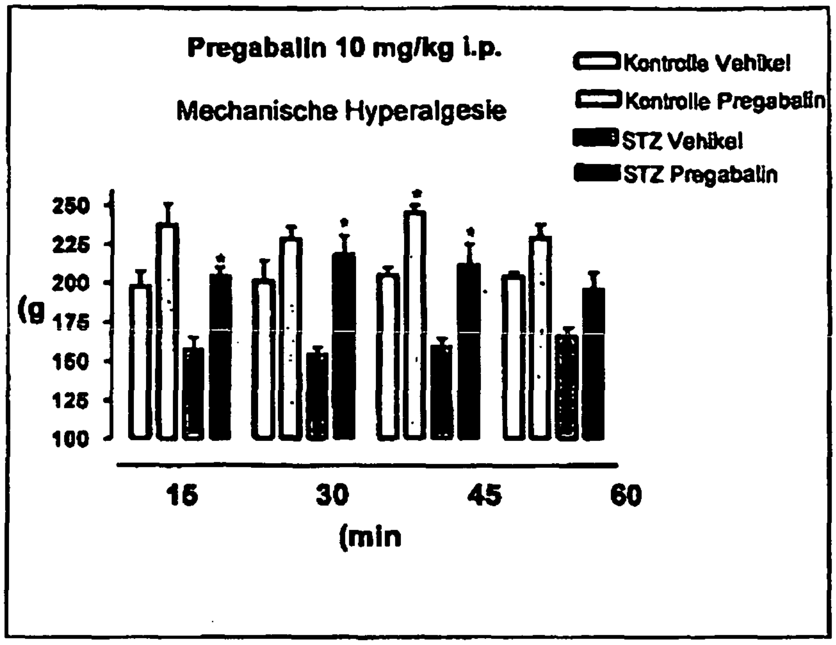


Abbildung 28: STZ-induzierte Polyneuropathieschmerz, Pregabalin, drei Dosierungen

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0997464 A
- WO 1999059997 A
- WO 2001039775 A
- EP 08856514 A
- EP 1392641 A
- WO 9854168 A


### In der Beschreibung aufgeführte Nicht-Patentliteratur


- **Power.** *Brit. J. Anaesth.,* 2005, vol. 95, 43-51
- **Davis et al.** *Respiratory Care Journal,* 1999, vol. 44 (1
- **Cepeda et al.** *Clinical Pharmacology and Therapeutics,* 2003, vol. 74, 102-112
- **Coderre et al.** *Pain,* 1993, vol. 52, 259-285
- **Yaksh et al.** *PNAS,* 1999, vol. 96, 7680-7686
- **Dickensen, A.** *International Congress and Symposium Series - Royal Society of Medicine,* 2000, vol. 246, 47-54
- **Vercauteren et al.** *Acta Anaesthesiologica Belglca,* 1994, vol. 45, 99-105
- **England et al.** *Neurology,* 1996, vol. 47, 272-276
- **Baron.** *Clin. J. Pain,* 2000, vol. 16 (2), 12-20
- *Saudi Pharm. J.,* 2002, vol. 10 (3), 73-85
- **Angst et al.** *Anesthesiology,* 2006, vol. 104, 570-587
- **Pud et al.** *Drug and Alcohol Dependence,* 2006, 218-223
- **Meunier et al.** *Nature,* 1995, vol. 377, 532-535
- **Ronzoni et al.** *Exp Opin Ther Patents,* 2001, (11), 525-546
- **Reinscheid et al.** *Science,* 1995, vol. 270, 792-794
- **Mogil et al.** *Neuroscience,* 1996, vol. 75, 333-337
- **Abdulla ; Smith.** *J. Neurosci.,* 1998, vol. 18, 9685-9694
- **Courteix et al.** *Pain,* 2004, vol. 110, 236-245
- **Calcutt ; Chaplan.** *Br. J. Pharmacol.,* 1997, vol. 122, 1478-1482
- **Enggaard et al.** *Pain,* 2001, vol. 92, 277-282
- **Chu et al.** *J. Pain,* 2006, vol. 7, 43-48
- **Angst et al.** *Pain,* 2003, vol. 106, 49-57
- **Saelens JK.** *Arch Int Pharmacodyn,* 1971, vol. 190, 213-218
- **Paton et al.** *Journal of Genetic Psychology,* 1977, vol. 131, 267-289
- **Cepeda et al.** *Clinical Pharmacology & Therapeutics,* 2003, vol. 74, 102-112
- **Ardati et al.** *Mol. Pharmacol.,* 1997, vol. 51, 816-824
- **D'Amour ; Smith.** *J. Pharm. Exp. Ther.,* 1941, 72 74 79
- **D'Amour ; Smith.** *J. Pharm. Exp. Ther.,* 1941, 72, 74 79
- **Kim,S.H. ; Chung,J.M.** An experimental model for peripheral neuropathy produced by segmental spinal nerve ligation in the rat. *Pain,* 1992, vol. 50, 355-363
- **Bennett ; Xie.** *Pain,* 1988, vol. 33, 87-107
- **KO Aley ; DB Reichling ; JD Levine.** *Neuroscience,* 1996, vol. 73, 259-265
- **SC Ahlgren ; JD Levine.** *Neuroscience,* 1993, vol. 52, 1049-1055
- **Herrero ; Headley.** *Br J Pharmacol,* 1996, vol. 118, 968-972
- **Christoph et al.** *Eur. J. Pharmacol.,* 2005, vol. 507, 87-98
- **Angst ; Clark.** *Anesthesiology,* 2006, vol. 104, 570-87
- Drug addiction and drug abuse. **Jaffe JH.** The pharmacological basis of therapeutics. Pergamon Press, 1990, 522-573
- **Bläsig J ; Herz A ; Reinhold K ; Zieglgänsberger S.** Development of physical dependence on morphine in respect to time and dosage and quantification of the precipitated withdrawal syndrome. *Psychopharmacology,* 1973, vol. 33, 19-38
- **Tzschentke, T.M. ; Bruckmann, W. ; Friderichs, F.** Lack of sensitization during place conditioning in rats is consistent with the low abuse potential of tramadol. *Neuroscience Letters,* 2002, vol. 329, 25-28